# EUROPEAN PATENT APPLICATION

(11) **EP 3 933 051 A1**
(43) Date of publication of application: **05.01.2022**
(21) Application number: 20718359.1
(22) Date of filing: 21.02.2020
(51) Int. Cl.: C12Q 1/6883

(54) **METHOD FOR DETECTING DUPLICATIONS AND/OR DELETIONS IN CHROMOSOMAL REGION 22Q11.2**

(30) Priority: 25.02.2019 ES 201930159
(71) Applicant: Servei de Salut de Les Illes Balears - Ibsalut, 07120 Palma de Mallorca (Islas Baleares) (ES)
(72) Inventor: HEINE SUÑER, Alexander Damian, 07120 Palma de Mallorca (Islas Baleares) (ES); TORRES JUAN, Laura, 07120 Palma de Mallorca (Islas Baleares) (ES)
(74) Representative: Bergenstråhle & Partners AB
(86) International application number: PCT/ES2020/070129
(87) International publication number: WO 2020/174109

(57) **Abstract**

The present invention relates to a method for detecting duplications and/or deletions in chromosome 22, particularly in chromosomal region 22q11.2, which comprises determining the number of alleles which have a set of genetic markers present between the Low Copy Repeats regions (LCR22). Said method is applicable in the diagnosis of diseases associated with said genetic anomalies, such as congenital cardiopathies and DiGeorge syndrome, inter alia. Furthermore, the present invention also relates to capable primers useful in the detection of the number of alleles, the kit comprising said primers and use thereof to diagnose the diseases associated with said genetic anomalies.

SEQUENCE LIST

<110>  FUNDACIÓ INSTITUT D'INVESTIGACIÓ SANITÀRIA ILLES BALEARS
       (IDISBA)

<120>  A METHOD FOR DETECTING DUPLICATIONS AND/OR DELETIONS IN THE
       CHROMOSOME REGION 22q11.2

<130>  ES3478.3

<150>  ES P201930159
<151>  2019-02-25

<160>  167

<170>  PatentIn version 3.5

<210>  1
<211>  25
<212>  DNA
<213>  Artificial Sequence

<220>
<223>  D22S420 forward primer

<400>  1
tgttctacac tgaaaattct gacgg                                           25


<210>  2
<211>  19
<212>  DNA
<213>  Artificial Sequence

<220>
<223>  D22S420 reverse primer

<400>  2
gagggcgtta tccatgacc                                                  19


<210>  3
<211>  24
<212>  DNA
<213>  Artificial Sequence

<220>
<223>  CATCH5 forward primer

<400>  3
tatggattca cagtcttggc caac                                            24


<210>  4

<211>  20
<212>  DNA
<213>  Artificial Sequence

<220>
<223>  CATCH5 reverse primer

<400>  4
tgtcagtggg atccccatgc                                                    20


<210>  5
<211>  22
<212>  DNA
<213>  Artificial Sequence

<220>
<223>  CATCH53 forward primer

<400>  5
acacccgtgg catggggcat tc                                                 22


<210>  6
<211>  23
<212>  DNA
<213>  Artificial Sequence

<220>
<223>  CATCH53 reverse primer

<400>  6
cgtactttct atcaaacccg tgg                                                23


<210>  7
<211>  20
<212>  DNA
<213>  Artificial Sequence

<220>
<223>  CATCH52 forward primer

<400>  7
   tggtacattt tcgggggttg                                                   20


<210>  8
<211>  23
<212>  DNA
<213>  Artificial Sequence

<220>
<223>  CATCH52 reverse primer

<400>  8
tgagctgaga tcacgccacc ctg                                              23


<210>  9
<211>  24
<212>  DNA
<213>  Artificial Sequence

<220>
<223>  D22S427 forward primer

<400>  9
tgctgttttg tagagtgttt agac                                             24


<210>  10
<211>  16
<212>  DNA
<213>  Artificial Sequence

<220>
<223>  D22S427 reverse primer

<400>  10
aaatacggct gggcac                                                      16


<210>  11
<211>  21
<212>  DNA
<213>  Artificial Sequence

<220>
<223>  D22S1638 forward primer

<400>  11
gacaacagca aattgcacat t                                                21


<210>  12
<211>  19
<212>  DNA
<213>  Artificial Sequence

<220>
<223>  D22S1638 reverse primer

<400>  12
tcacgccact accctccag                                                   19


<210>  13

<211>  20
<212>  DNA
<213>  Artificial Sequence

<220>
<223>  CATCH4 forward primer

<400>  13
aaaaaattca gcaaggggtg                                                    20


<210>  14
<211>  21
<212>  DNA
<213>  Artificial Sequence

<220>
<223>  CATCH4 reverse primer

<400>  14
agtattctgt ctatggcttg c                                                  21


<210>  15
<211>  22
<212>  DNA
<213>  Artificial Sequence

<220>
<223>  CATCH6 forward primer

<400>  15
cgtcttggta tttccaagca gc                                                 22


<210>  16
<211>  21
<212>  DNA
<213>  Artificial Sequence

<220>
<223>  CATCH6 reverse primer

<400>  16
ggaggacaag cttgccagga c                                                  21


<210>  17
<211>  24
<212>  DNA
<213>  Artificial Sequence

<220>
<223>  22K48-2 forward primer

<400>  17
cggtgtgaat ctataatcat ctcg                                              24

<210>  18
<211>  24
<212>  DNA
<213>  Artificial Sequence

<220>
<223>  22K48-2 reverse primer

<400>  18
acctctggtg tttggatatt tacc                                              24

<210>  19
<211>  21
<212>  DNA
<213>  Artificial Sequence

<220>
<223>  D22S1648 forward primer

<400>  19
agttgtcaga tgcctaagag a                                                 21

<210>  20
<211>  20
<212>  DNA
<213>  Artificial Sequence

<220>
<223>  D22S1648 reverse primer

<400>  20
cagatgcttc aggagaagtg                                                   20

<210>  21
<211>  23
<212>  DNA
<213>  Artificial Sequence

<220>
<223>  D22S941 forward primer

<400>  21
caggttacaa agtacattaa ctt                                               23

<210>  22

<211>  21
<212>  DNA
<213>  Artificial Sequence

<220>
<223>  D22S941 reverse primer

<400>  22
accagctcca accatttctt g                                                21


<210>  23
<211>  25
<212>  DNA
<213>  Artificial Sequence

<220>
<223>  CATCH48 forward primer

<400>  23
gatccttact gagaggaatg ttgtg                                            25


<210>  24
<211>  21
<212>  DNA
<213>  Artificial Sequence

<220>
<223>  CATCH48 reverse primer

<400>  24
agggtgatga aaaggttctg g                                                21


<210>  25
<211>  21
<212>  DNA
<213>  Artificial Sequence

<220>
<223>  D22S944 forward primer

<400>  25
catgtgaaag atgctacttc c                                                21


<210>  26
<211>  19
<212>  DNA
<213>  Artificial Sequence

<220>
<223>  D22S944 reverse primer

<400>  26
atggggagga gcatgggat                                                    19


<210>  27
<211>  21
<212>  DNA
<213>  Artificial Sequence

<220>
<223>  D22S1623 forward primer

<400>  27
cacaactcct gggctcaagc t                                                 21


<210>  28
<211>  23
<212>  DNA
<213>  Artificial Sequence

<220>
<223>  D22S1623 reverse primer

<400>  28
acgtaaatct cataccatgt aaa                                               23


<210>  29
<211>  20
<212>  DNA
<213>  Artificial Sequence

<220>
<223>  CATCH 22 forward primer

<400>  29
agtaggcagg ggccataagg                                                   20


<210>  30
<211>  21
<212>  DNA
<213>  Artificial Sequence

<220>
<223>  CATCH 22 reverse primer

<400>  30
cactgcactc cagcttgggt g                                                 21


<210>  31

<211>  20
<212>  DNA
<213>  Artificial Sequence

<220>
<223>  CATCH 7 forward primer

<400>  31
cagcagcctg agcagcctgg                                                    20


<210>  32
<211>  20
<212>  DNA
<213>  Artificial Sequence

<220>
<223>  CATCH 7 reverse primer

<400>  32
ccccggggcc ctcgggctcg                                                    20


<210>  33
<211>  24
<212>  DNA
<213>  Artificial Sequence

<220>
<223>  CATCH 23 forward primer

<400>  33
tgtgacagag tgacagcccg tctc                                               24


<210>  34
<211>  20
<212>  DNA
<213>  Artificial Sequence

<220>
<223>  CATCH 23 reverse primer

<400>  34
aggcccaccg tggcagcagc                                                    20


<210>  35
<211>  22
<212>  DNA
<213>  Artificial Sequence

<220>
<223>  CATCH 19 forward primer

<400>  35
gcacgttctg cacatgtacc cc                                                22

<210>  36
<211>  20
<212>  DNA
<213>  Artificial Sequence

<220>
<223>  CATCH 19 reverse primer

<400>  36
tggcccagga gtccccatgc                                                   20

<210>  37
<211>  20
<212>  DNA
<213>  Artificial Sequence

<220>
<223>  D22S264 forward primer

<400>  37
attaactcat aaaggagccc                                                   20

<210>  38
<211>  20
<212>  DNA
<213>  Artificial Sequence

<220>
<223>  D22S264 reverse primer

<400>  38
ggaatacctc tggtggggtg                                                   20

<210>  39
<211>  23
<212>  DNA
<213>  Artificial Sequence

<220>
<223>  CATCH 45 forward primer

<400>  39
tgtttctctg tgtctggtgg aag                                               23

<210>  40

<211>  25
<212>  DNA
<213>  Artificial Sequence

<220>
<223>  CATCH 45 reverse primer

<400>  40
atgtagacac agatatgcac cctgc                                          25


<210>  41
<211>  20
<212>  DNA
<213>  Artificial Sequence

<220>
<223>  CATCH 10 forward primer

<400>  41
cacggtgtcc ggcagcatcc                                                20


<210>  42
<211>  20
<212>  DNA
<213>  Artificial Sequence

<220>
<223>  CATCH 10 reverse primer

<400>  42
cgcctcatgg agggcgcagg                                                20


<210>  43
<211>  21
<212>  DNA
<213>  Artificial Sequence

<220>
<223>  CATCH 20 forward primer

<400>  43
agatgagtga taaccgagtg c                                              21


<210>  44
<211>  20
<212>  DNA
<213>  Artificial Sequence

<220>
<223>  CATCH 20 reverse primer

<400>  44
tgaattggtg gctgggcctg                                                    20


<210>  45
<211>  20
<212>  DNA
<213>  Artificial Sequence

<220>
<223>  CATCH42 forward primer

<400>  45
tgctatgttg cccaagctgg                                                    20


<210>  46
<211>  24
<212>  DNA
<213>  Artificial Sequence

<220>
<223>  CATCH42 reverse primer

<400>  46
actgtgtgtg tctctgtgtc cagc                                               24


<210>  47
<211>  24
<212>  DNA
<213>  Artificial Sequence

<220>
<223>  CATCH41 forward primer

<400>  47
ctgttaccac taaatgggaa aatg                                               24


<210>  48
<211>  22
<212>  DNA
<213>  Artificial Sequence

<220>
<223>  CATCH41 reverse primer

<400>  48
tgggtgacag agtgagactc tg                                                 22


<210>  49

<211>  20
<212>  DNA
<213>  Artificial Sequence

<220>
<223>  CATCH11 forward primer

<400>  49
agccaaaatc acgccactgc                                                    20


<210>  50
<211>  22
<212>  DNA
<213>  Artificial Sequence

<220>
<223>  CATCH11 reverse primer

<400>  50
   ctttatggtt accccttgga cc                                                22


<210>  51
<211>  24
<212>  DNA
<213>  Artificial Sequence

<220>
<223>  D22S311 forward primer

<400>  51
tttttgtatt tttagtagag acgg                                               24


<210>  52
<211>  22
<212>  DNA
<213>  Artificial Sequence

<220>
<223>  D22S311 reverse primer

<400>  52
gctagtgtga gataacgaag cc                                                 22


<210>  53
<211>  20
<212>  DNA
<213>  Artificial Sequence

<220>
<223>  CATCH12 forward primer

<400>  53
aaaggcagaa agcagccctg                                                    20


<210>  54
<211>  23
<212>  DNA
<213>  Artificial Sequence

<220>
<223>  CATCH12 reverse primer

<400>  54
gggtgaagag taaagccttt tcc                                                23


<210>  55
<211>  20
<212>  DNA
<213>  Artificial Sequence

<220>
<223>  CATCH13 forward primer

<400>  55
cgtttggtgg aggagggtcc                                                    20


<210>  56
<211>  23
<212>  DNA
<213>  Artificial Sequence

<220>
<223>  CATCH13 reverse primer

<400>  56
taacactgat cctttctggg agg                                                23


<210>  57
<211>  21
<212>  DNA
<213>  Artificial Sequence

<220>
<223>  CATCH14 forward primer

<400>  57
gaaggaaagg aaagggaaag g                                                  21


<210>  58

<211>  20
<212>  DNA
<213>  Artificial Sequence

<220>
<223>  CATCH14 reverse primer

<400>  58
ggccccgagg cttcccctgc                                                    20


<210>  59
<211>  21
<212>  DNA
<213>  Artificial Sequence

<220>
<223>  CATCH39 forward primer

<400>  59
cactgcactc cagcctggga g                                                  21


<210>  60
<211>  21
<212>  DNA
<213>  Artificial Sequence

<220>
<223>  CATCH39 reverse pimer

<400>  60
caggctctgg gatagccagt c                                                  21


<210>  61
<211>  21
<212>  DNA
<213>  Artificial Sequence

<220>
<223>  D22S1709 forward primer

<400>  61
cacttcagca agaacagcag a                                                  21


<210>  62
<211>  21
<212>  DNA
<213>  Artificial Sequence

<220>
<223>  D22S1709 reverse primer

<400>  62
ctcttccaag ttcagtgctc t                                                21

<210>  63
<211>  25
<212>  DNA
<213>  Artificial Sequence

<220>
<223>  CATCH38 forward primer

<400>  63
gtacctgtag aattaccagt gctgg                                            25

<210>  64
<211>  21
<212>  DNA
<213>  Artificial Sequence

<220>
<223>  CATCH38 reverse primer

<400>  64
ctccagcctg ggcaacagag c                                                21

<210>  65
<211>  20
<212>  DNA
<213>  Artificial Sequence

<220>
<223>  CATCH37 forward primer

<400>  65
tggccgctgt gggctgcatg                                                  20

<210>  66
<211>  20
<212>  DNA
<213>  Artificial Sequence

<220>
<223>  CATCH37 reverse primer

<400>  66
gtgcatgcca tcatgcccag                                                  20

<210>  67

<211>  20
<212>  DNA
<213>  Artificial Sequence

<220>
<223>  CATCH36 forward primer

<400>  67
aatgccgggg ccgtcggtac                                                    20


<210>  68
<211>  21
<212>  DNA
<213>  Artificial Sequence

<220>
<223>  CATCH36 reverse primer

<400>  68
aacatctgcc catgcatacg g                                                  21


<210>  69
<211>  21
<212>  DNA
<213>  Artificial Sequence

<220>
<223>  CATCH35 forward primer

<400>  69
ctgtactcca gcctgggcaa c                                                  21


<210>  70
<211>  23
<212>  DNA
<213>  Artificial Sequence

<220>
<223>  CATCH35 reverse primer

<400>  70
gtaaatgatg ttccacccgt ttg                                                23


<210>  71
<211>  19
<212>  DNA
<213>  Artificial Sequence

<220>
<223>  D22S446bis forward primer

<400>  71
ccaaggcagg cgaatcagg                                                    19


<210>  72
<211>  20
<212>  DNA
<213>  Artificial Sequence

<220>
<223>  D22S446bis reverse primer

<400>  72
tgtctggatg ggcgtggtgg                                                   20


<210>  73
<211>  24
<212>  DNA
<213>  Artificial Sequence

<220>
<223>  CATCH51 forward primer

<400>  73
actttcggag gctgaggcag gtgg                                              24


<210>  74
<211>  21
<212>  DNA
<213>  Artificial Sequence

<220>
<223>  CATCH51 reverse primer

<400>  74
cctgggacta caggcgtgtg g                                                 21


<210>  75
<211>  20
<212>  DNA
<213>  Artificial Sequence

<220>
<223>  CATCH33bis forward primer

<400>  75
cctggtccca gatacagtgc                                                   20


<210>  76

<211>  22
<212>  DNA
<213>  Artificial Sequence

<220>
<223>  CATCH33bis reverse primer

<400>  76
gggtaatggg tggagattga tg                                                22


<210>  77
<211>  20
<212>  DNA
<213>  Artificial Sequence

<220>
<223>  D22S539 forward primer

<400>  77
cattatggct gtaggctgta                                                   20


<210>  78
<211>  19
<212>  DNA
<213>  Artificial Sequence

<220>
<223>  D22S539 reverse primer

<400>  78
cataccaatg caatatgaa                                                    19


<210>  79
<211>  22
<212>  DNA
<213>  Artificial Sequence

<220>
<223>  CATCH31 forward primer

<400>  79
gaacacacag gcccgctgtt cc                                                22


<210>  80
<211>  26
<212>  DNA
<213>  Artificial Sequence

<220>
<223>  CATCH31 reverse primer

<400>  80
caacaaaaat acccatgata cagctc                                          26


<210>  81
<211>  20
<212>  DNA
<213>  Artificial Sequence

<220>
<223>  CATCH25 forward primer

<400>  81
ttgtcaaggt cacaggcatc                                                 20


<210>  82
<211>  22
<212>  DNA
<213>  Artificial Sequence

<220>
<223>  CATCH25 reverse primer

<400>  82
gtggcattgt atgctgagat ag                                              22


<210>  83
<211>  24
<212>  DNA
<213>  Artificial Sequence

<220>
<223>  CATCH24 forward primer

<400>  83
tttacccagg gttgggatag gtgg                                            24


<210>  84
<211>  23
<212>  DNA
<213>  Artificial Sequence

<220>
<223>  CATCH24 reverse primer

<400>  84
ttctccatgg taacgctaaa acc                                             23


<210>  85

<211>  24
<212>  DNA
<213>  Artificial Sequence

<220>
<223>  CATCH26 forward primer

<400>  85
gcatatcaaa agctaagtta tagc                                              24


<210>  86
<211>  20
<212>  DNA
<213>  Artificial Sequence

<220>
<223>  CATCH26 reverse primer

<400>  86
gttccttctt ttggttgaag                                                   20


<210>  87
<211>  20
<212>  DNA
<213>  Artificial Sequence

<220>
<223>  CATCH30 forward primer

<400>  87
gtctgctgac ctgcatactc                                                   20


<210>  88
<211>  20
<212>  DNA
<213>  Artificial Sequence

<220>
<223>  CATCH30 reverse primer

<400>  88
ccaagggaac tggaagcagg                                                   20


<210>  89
<211>  22
<212>  DNA
<213>  Artificial Sequence

<220>
<223>  CATCH29 forward primer

<400>  89
ttagatactc tgatgagcca gc                                                22

<210>  90
<211>  22
<212>  DNA
<213>  Artificial Sequence

<220>
<223>  CATCH29 reverse primer

<400>  90
ccctcaaatc tttcagaatc ag                                                22

<210>  91
<211>  20
<212>  DNA
<213>  Artificial Sequence

<220>
<223>  D22S308 forward primer

<400>  91
tcctgcaaca gcactagacc                                                   20

<210>  92
<211>  21
<212>  DNA
<213>  Artificial Sequence

<220>
<223>  D22S308 reverse primer

<400>  92
gctaaaggaa aaggaggcat c                                                 21

<210>  93
<211>  19
<212>  DNA
<213>  Artificial Sequence

<220>
<223>  CATCH15 forward primer

<400>  93
taggcaggaa tgggtcctg                                                    19

<210>  94

<211>  20
<212>  DNA
<213>  Artificial Sequence

<220>
<223>  CATCH15 reverse primer

<400>  94
ttcaacctga gggtggctgg                                                    20


<210>  95
<211>  21
<212>  DNA
<213>  Artificial Sequence

<220>
<223>  D22S306 forward primer

<400>  95
ctctttcgct ggaacatcaa a                                                  21


<210>  96
<211>  21
<212>  DNA
<213>  Artificial Sequence

<220>
<223>  D22S306 reverse primer

<400>  96
ggtccagact gtataaatgg c                                                  21


<210>  97
<211>  24
<212>  DNA
<213>  Artificial Sequence

<220>
<223>  CATCH16 forward primer

<400>  97
gcagtaagat gatgactccg gacc                                               24


<210>  98
<211>  24
<212>  DNA
<213>  Artificial Sequence

<220>
<223>  CATCH16 reverse primer

<400>  98
atatggtaac acacatgcgt gtgc                                              24


<210>  99
<211>  20
<212>  DNA
<213>  Artificial Sequence

<220>
<223>  CATCH28 forward primer

<400>  99
acctgcacca tgcttcctgc                                                   20


<210>  100
<211>  25
<212>  DNA
<213>  Artificial Sequence

<220>
<223>  CATCH28 reverse primer

<400>  100
gtaatatagc ctaaacataa agtac                                             25


<210>  101
<211>  23
<212>  DNA
<213>  Artificial Sequence

<220>
<223>  CATCH17 forward primer

<400>  101
ccttacttgt atcctaggcc agc                                               23


<210>  102
<211>  20
<212>  DNA
<213>  Artificial Sequence

<220>
<223>  CATCH17 reverse primer

<400>  102
tgatgctgca gggcttggac                                                   20


<210>  103

<211>  20
<212>  DNA
<213>  Artificial Sequence

<220>
<223>  CATCH27 forward primer

<400>  103
tgttccagcc tgggcgacag                                                    20


<210>  104
<211>  19
<212>  DNA
<213>  Artificial Sequence

<220>
<223>  CATCH27 reverse primer

<400>  104
cctggtattg cctttccag                                                     19


<210>  105
<211>  21
<212>  DNA
<213>  Artificial Sequence

<220>
<223>  CATCH18 forward primer

<400>  105
gaagatagcc caccttgctg g                                                  21


<210>  106
<211>  23
<212>  DNA
<213>  Artificial Sequence

<220>
<223>  CATCH18 reverse primer

<400>  106
cctgagatat tagggcaaac agg                                                23


<210>  107
<211>  21
<212>  DNA
<213>  Artificial Sequence

<220>
<223>  D22S686 forward primer

<400>  107
ttgattacag agtggctctg g                                                21

<210>  108
<211>  20
<212>  DNA
<213>  Artificial Sequence

<220>
<223>  D22S686 reverse primer

<400>  108
taagccctgt tagcaccact                                                  20

<210>  109
<211>  19
<212>  DNA
<213>  Artificial Sequence

<220>
<223>  D22S1174 forward primer

<400>  109
gaatcactag gggccttca                                                   19

<210>  110
<211>  18
<212>  DNA
<213>  Artificial Sequence

<220>
<223>  D22S1174 reverse primer

<400>  110
tgaggctatg tggcccag                                                    18

<210>  111
<211>  744
<212>  DNA
<213>  Artificial Sequence

<220>
<223>  Marker CATCH5

<400>  111
gtgctgaaag aacagaacac ctgtggcaca gatgcaaatg aacaggatgg ccagcatccc      60

tggtctatcc tggcataact actcgttttc accatcagtg attttgttat ctttatgtga     120

tagaggctat tccatacaac ctattggctg aaaataatgc ctaacaataa aaaagctact 180

aagaattcaa cattttccca tgcatttgta ttctgttcat cacaacggca tctacatacc 240

ctgcaaaaaa aagtagaaca catatattta aggcatttac tcaagcgaca atatttgatg 300

aatctgccct atggattcac agtcttggcc actgggcagt gtacatgcaa acacacacac 360

acacacacac acacacacac ccacacacac ccactcatgc ctacagcatg gggatcccac 420

tgacaacagc tcagaagaga ggtaaaggtt aaggacaggc ttctaacaaa ttcagttcaa 480

tacatttaag tacatattat aataaaaatg aggaccaggt gtggtggctc acgcctgtaa 540

tcccagcact ttgggaggcc aagatgggaa gatcacttga gaccaggaat ttgagaccag 600

cctgggcaac acagcgaaac cccttctcta ttgtaaaaat gactaaaaat tggctgggcg 660

tggtggctca cgcctgtaat cccagcactt tgggaggccg aggtgggcag atcactaggt 720

caggagttcg agaccagcct gacc 744

<210> 112
<211> 741
<212> DNA
<213> Artificial Sequence

<220>
<223> Marker CATCH53

<400> 112
catgaggcag accatggtcc cgacctgcat tggctggtgc tggctgcagc tgctgatgga 60

ggctacaaac agtgatagaa gcatgcagaa gctacacagc acaggccaag gtgctgtggg 120

agaggcggag gaggaacact gaccactggc ggcctgggga cacgggaggg ctggcttctg 180

aaactccatg gtgtgagaat tctgatcgca gtaggtaact tttctgatgg gaaaggtgca 240

gcattggtgg aaaaacagga gatggcactc aagaatttat actggagaga gaccaaggac 300

agagattcta aaagcttaaa gaaggataaa cacccgtggc atgggcattc gtgtgtgtgt 360

gtgtgtgtgt gtgtgtgtgt gtgtgtgtgt gaattgccag agcaaactct tatccaccat 420

gttgtgcaaa agttattagg ttgattaagt tgttggcttt tctttccacg ggtttgatag 480

aaagtacgca cacacatctt ttgtgtggta tgacagtgtg tgtgtactca ggggagggtg 540

cacgggaggt cagtggaagc ttgtgtgtgg agggcagggg aggatttggg tgtgaaaagc 600

ccagaaatag ggtgagaggc aatgtgttcc atagccaagg gtgagagtca ggcagagatt 660

tgctttcttg ggctttgagt catgccgata ctcatcgctt agcctcatat atggtaagaa 720

tggagcgggt gctctctgtc t 741

<210> 113
<211> 732
<212> DNA
<213> Artificial Sequence

<220>
<223> Marker CATCH52

<400> 113
tggaagggat gtggggggcg gggcagacgg gagacatggg ttttgaaggc agtgaacaat 60

aaaaccttag ggaggtggca ccgaggcctt agcatttgag gagctgaaat gtttcagtgt 120

tgttttctca ccagccacaa gcatcttatg agtttctgtg caaggaagcg tagagcactg 180

ctgtgctgtc aggatgaaga agggctttct gcaggggctg gcctttctct acccagaggc 240

caagcaggct gccctgcact gtgtccttgg tgttgatgcc caaaatagag aaggtgcttg 300

ctagcttttc cttggtacat tttcgggggt tgcaaggcaa caagttttct tttgttttgt 360

ttgtttgttt gtttgtttgt ttttgagatg gagtctctgt cacccaggct ggagtgcagt 420

ggcgtgatct cagctcactg caagctccgc ctcccgggtt catgccattc tcctgcctca 480

gcctcccgag tagctgggac tacaggtgcc caccacctcg cctggctaat atttttgtat 540

ttttagtaga gtctgggttt cactgtgtta gccgggatgg tctcgatctc ctgaacttgt 600

gatccaccca cctaggcctc ccaaagtgct gggattacag gtgtgagcca tcgcgcccgg 660

ccaacttttc tttagaaaaa tgagtaaagt aggtaaactt cacaggcccc ttagaattaa 720

atccttaggg ca 732

<210> 114
<211> 740
<212> DNA
<213> Artificial Sequence

<220>
<223> Marker CATCH4

<400> 114
gatttaatgc aaatccagta aaaaattcaa caagaggctg ggcgcggtgg ctcaagcctg 60

taatcccagc actttgggag gctgaggcag gcagatcaca aggtcaggag atcgagacca 120

tcctggctaa catggtgaaa tcccgtctct actaaaaaaa aaaaaaaaaa aaaaaatgag 180

ccaggcgtgg tggcgggtgc ctgtagtccc agctactcag gaggctgagg cagggaatgg 240

tgtcaaccca ggaggcagag cttgcagtga gccgagatcg agacactgca ctccagcctg 300

ggcgacagag cgagactctg tctcaaaaaa aaaaaaaaat tcagcaaggg gtgtgtgtgt 360

gtgcctgtgt gtgtgtgtgt gtgtgtgtgt atgcaagcca tagacagaat actttttttt 420

tctttttgag acagagtctc actctgtcgc ccaggctgga gtgcagtggc tcgatcttgg 480

ctcactgcaa cctccgcctc ccaggttcaa gtgattctcc tgtccctgcc tcctgtgtag 540

ctgggattac aggtgtgcac caccacatct ggctaatttt tgtattttta gtagacacag 600

ggtctcacca tgttggccag gctagtctcc aactcctggc cgcaagtaat ctgcccactt 660

cagcctccca aagtgttggg attataggcg tgagccaccg agcccagcta gtacacagaa 720

taccattttt tacttaagta 740

<210> 115
<211> 728
<212> DNA
<213> Artificial Sequence

<220>
<223> Marker CATCH6

<400> 115
tcactcctca tttatccttc ttagagatac ttaagacatc aacagcagga ctgggatgtg 60

tttacgaaaa gaacccaaat cctagctcaa gccacaggca caaagtacag cccaacagac 120

agggaagggg cagggcaagg gagtgtgacc ctggcggcag ctggagaagg cactgctgga 180

gtatgtccag gaggagctcg ccagtgccag ggcaatggga caggaagggc aagccaagtc 240

cgagcccagg gaatgtaaag gaaacagcag ccatgtttcc tttacgtcgt cttggtattt 300

ccaagcagct ccgcataaaa tctttgcgca cacacacaca tgcatgctca ctctctctct 360

ctctctctct ctctctctat ctgcctgata gtttccttca ggtctggatt ctacgtctga 420

cctttgtcct ggcaagcttg tcctcccctc cagcctgcca ggggcacact ctgcatgctc 480

atttttatac tccaaggtct ttaacttcaa aaggaggcgg gggacaatct agaaagctgg 540

ccaaagtgag atgcaacaga gggttccaga gacagccacc gcccaatgca gggcctgatc 600

tgcaggctgc actggcccca tgaccgcagc cctccctgca actggggctc acccactccc 660

tgcacagcac ccctacgggc caggcagcac tgggtaagag ggaggagggg aggccccgaa 720

tcagggtg 728

<210> 116
<211> 794
<212> DNA
<213> Artificial Sequence

<220>
<223> Marker CATCH48

<400> 116
gaattatgga atggtccttc taagctcctg aataattaaa ccagtgagca gagccactgt 60

cccatcccgt tggccacacc agacacttgg gaattatttt tggttccttc ttctacctct 120

tttcaattcc aataattacc aagttctatc aattttaatc ccctaatatc tctctaatcg 180

atctcttcag ttgctattta agtaactctt catttcttct cacctgtgtt actacagaaa 240

ttcatttttc tatagttttg ccttccatca atcccttctc catcttaaac caaactgatc 300

ttactgagag gaatgttgtg atttcctctc tctctctctc tctggaatga atatatacat 360

acatacatat atatatatat atatatgtgt gtgtatatat atatatatat atgtgtgtgt 420

gtgtatatat atatatatat atatgtgtgt gtgtgtatat atatgtatat atattccaga 480

accttttcat caccctaaag agaaactctg tccctaaatc ccattccccc tcccctagcc 540

cctggtaacc accaaccacc atctcacttt cgctctacaa atttgattat tctaggtacc 600

ccatataacg actcgtacgg tatttgtcct tttgtgtctg gcttatgtca cttagcccaa 660

tatttttcag gtttatctat gttgtgtcat gtgtcagtat caggatttca tctctttctg 720

aggctgaatg atgccccact gtgtttatcc attcatctgt tggacacctg ggctgcttct 780

atctcttgct attg 794

<210> 117
<211> 768
<212> DNA
<213> Artificial Sequence

<220>
<223> Marker CATCH22

<400> 117
cactccagct taggtgacag aggaagactc catctcttaa aaaacaaaaa aacaaaaaaa 60

aaaaactaat aaaataaaaa catgaaatcc aaaccaaaaa gagatgagag gggaggtagg 120

cagagagaga cagagggaga gagagaccag gagagagcca gggagagagg aagggagaga 180

ggcttggact gagtgtctga gtctccatac agacagtcag atcactggta tgttgtgggg 240

gtgcagttgg ggaaatgagg aaactccagg catcaggggg ccccatttga gatgtaccat 300

ccagagtgtg agaattctaa tgagtaggca ggggccataa ggaccatttt cttcctccct 360

cccttccttc cttccttcct tccttccttc cttccttcct tccttccttc cttcttccct 420

ccctcccgcc ctcccttctt tccttccttt ttcgtttttg aaatagggtc tcactctgtc 480

acccaagctg gagtgcagtg acacaatcat agctcactgc tgcctcgaac tccttggcta 540

aagcaatcct cccaccttag cctcctgagg agctaggact acaggcatgc accaccacac 600

ccaattaatt taaaaaaatt taatagagat ggggtcttgc cgtgttccag gctggtcttg 660

agctcctgga ctcaagtgat cctcccacct cagcctccca aagagccagg attgtaggca 720

tgagccaccg tgtccagcct ctttataaat aaaagatcag tgacacag 768

<210> 118
<211> 730
<212> DNA
<213> Artificial Sequence

<220>
<223> Marker CATCH19

<400> 118
gctttttatg cctacttggt gtcagtctgg aacccacaga gcaattggct tcaagaggtc 60

gttagctcaa gtaggagtgg gatgtgactg ctgtttcatt ccagtgcctc tctgggccag 120

ataattaaag ggagctcaca ttcctcagat aaaaaagttc ctttcgcatt aggagaaata 180

cgtaatgtag atgataggtt catgggcaca gcaaaccacc atggcacgtg tatacctatg 240

taacaaacct gcacgttctg cacatgtacc ccagaactta aaagaatata tatatgaata 300

tatatatata ctttatacat atatatataa acttccattg tgcgcgcgcg cacacacaca 360

cacacacaca cacacacaca aagttccttt tctttttcat ggacatgagc agtatgtcac 420

tcaggcatgg ggactcctgg gccacctctg ggtcttcagg gagggcctgg tctggaagga 480

ggggtctcat cctccagagc cccagggatg ggtgatatat gtgtaggaat gggggagctg 540

gttcgcagac gtgcgtttcc cctgtgtgtg tggacacgat gggccccatg gctcttcccg 600

tcgggtggta tccaccgaag gcctttgctc tgcggccact gtccacctcc acttggcctc 660

ccgagtccac caggctggct ggtgacacct tgggttagtg tggggtgact ggtgtgtgtt 720

ggggagctgc 730

<210> 119
<211> 828
<212> DNA
<213> Artificial Sequence

<220>
<223> Marker CATCH7

<400> 119
gggagggatc tagtccattg tctccgcgcg ggggtggggg ggccccgggc ggggcggggc 60

ggggcgcctc ctcggggccg gcctgcggtg tggggctgca cggcccgggg cgcacgcagc 120

gcggcgcccg ccactcggcc cgcggcgcgg ggcagcgctc agcttggtgg cgggggcggc 180

ggcggcggcc cgcgggtcat gatctccgcc gtgtccagcc cgtggctcac gcagctctcg 240

catttctgcg acgttgcagc cttcacggcc agcagcctga gcagcctggg ggccgcgggg 300

ggcttcccgg gcgccgcgtc gcccggcgcc gacccgtacg gcccgcgcga gcccccgccg 360

ccgccgccgc gctacgaccc gtgcgccgcc gccgcccccg gcgccccggg cccgccgccg 420

ccgccgcacg cctacccgtt tgcgccggcc gccggggccg ccaccagcgc cgccgccgag 480

cccgagggcc ccggggccag ctgcgcggcc gcagccaagg cgccggtgaa gaagaacgcg 540

aaggtggccg gtgtgagcgt gcagctagag atgaaggcgc tgtgggacga gttcaaccag 600

ctgggcaccg agatgatcgt caccaaggcc ggcaggtcag ggcgcccctc cccacgccgc 660

gaccctcccc acgtgctgcc gccagggctg cgggcctccg cctgatccgc gcgagcgggg 720

ccgaaagccg ggtcgggggc gcggcctggc cacctgcggg cccctctggg ccccgctgcc 780

tccttcgctg ttcgccgtcc cggctccggc gacagccgcc cggcgctc 828

<210> 120
<211> 755
<212> DNA
<213> Artificial Sequence

<220>
<223> Marker CATCH23

<400> 120

actctagcct gggagacaga gcaagactcc gtctcaaaac aaaacaaaac aacaacaaaa 60

aaaaaacagc cgagcgcagt ggctcacgcc tgtaatccca gcactttggg aggccgaggt 120

gggcagatca caaggtcagg agttcaagac cagcctgacc aacatggtga aaccttgtct 180

ctactaaaaa tacaaaaatt agctaggcgt ggtggtgtga gcctataatc ccagctactc 240

aggaggctga ggcaggagaa tcgcttgaac ccaggaggcg gaggttgcag tgagccgaga 300

tcgcgccact gcactccagt ctgtgtgaca gagtgacagc ccgtctcaaa aaataaataa 360

ataaataaat aaataaataa ataaataaat aaataaataa ataaaatgaa gcagcagcag 420

ctttgttagt gtttgttcaa ggcctgatag gccaaggtga ggctcagccg agaaacgtgc 480

tcagaggagc ctggccggag tgtgatgggg gagtctttgt ccaggctgct gccacggtgg 540

gcctcaggac tcatggcctg ggcgctgggg gtggggctgc cactcagcct tgaggagagc 600

aatgaggagg tcagattcca ctcacatcca gtgcggtctg gatgcaggcc tggcccagag 660

cctggtccag atgctggggc tttgtttttt tgttgttgtt tttttgagac agagtctcgc 720

tctgttgccc aggctggagt gcagtggcac tatct 755

<210> 121
<211> 726
<212> DNA
<213> Artificial Sequence

<220>
<223> Marker CATCH45

<400> 121
tatccgaatt cccgtgctca agtgcgcctc ctgccttggc tttcctgagt gtggggatga 60

caggcgtgag ccaccacgct gggcctcatg atctgtttct agtgcagtga gtgcatctta 120

gggtgggtgc ccgcctccat gcttagtagg gcatggatgc tgtgtatgcc atctgtcggt 180

aggatggtta cacttctgtg tgttggctga tgtgtgtgtc tcagtattta gtcggctctg 240

gtggcacatg tgtgtgttgc aggttgtata agttgctgtt tctctgtgtc tggtggaagg 300

tgtctatggt tgcagagtga gggtctcttg gtgtttctgt gtgtcagagg gtgtgtgtgt 360

gtgtgtgtgt gtgtgttttg caggatgagg tgtgttctgt gagtgtgatg tggggggtgc 420

agggtgcata tctgtgtcta catttctttt ttttcttctt cttctttttt ttgagacaga 480

gtctcgctct gtcgcccagg ctggagcgca gtgatgcgat ctccactcac tgcaaacact 540

gcctcccagg tttaagcaat tctcccagct cagcctccca agtagctgag attacaggcg 600

catgccacca cgcccggcta attttttgta tttttagtag agatgcggtc tcaccatgtt 660

ggccaggctg gtcttaaact cctgatctca agtgatctgc ctgcctcggc ctcccaaagt 720

gctggg 726

<210> 122
<211> 751
<212> DNA
<213> Artificial Sequence

<220>
<223> Marker CATCH10

<400> 122
tgggtgccca cggcactcca tcctcccctc aggtgacttc tcgtccaggg ttccctggga 60

tgcactgtcg tggcccatgg ccgaggctgg gcctggaacg gagccgcgct ggctagggag 120

ccgaaggggc ccacacttgg cgtaggaagg ttccggtagc cccctcccaa tgcccggcgc 180

cgccacacca ctgcccagag cgtccctctc gccccctccc ccagccaggc cggctcctac 240

ccgggagcac ggcacacgtt gcggccgcga gggttcagtt cctgaggcgc cacggtgtcc 300

ggcagcatcc agagcagcag cagcagcagc agcgacggca gcagcggtga cggcggcccc 360

ccggctcccc ggcgccgcgc cggcccggcc ccccggggcc ctgcgccctc catgaggcgc 420

ggggcaggcg cggggcgggc acgggcgcgg gtgcggccgc agcgagagcg gccggaagcg 480

gagtgcgagg ccgggcgggg ggcggcagga ggggcgccga gccgggcagg aaattccaca 540

tcggctccct gatcccgggc cagccgcggc cggccaggcg gcagcgcccg ccccgcgctc 600

cagaccccac cctccggcct agccaccgcc cctccgcgca gcccccgccc ccatttggcc 660

gctcgcaaac ttgggggcga acttggagga ggcccgccga gcgctggtgg agggagggtc 720

cgcccgagga atccccggtt cccgcgcctt t 751

<210> 123
<211> 793
<212> DNA
<213> Artificial Sequence

<220>
<223> Marker CATCH20

<400> 123

acctctgccc caccagttta agtgattctc ctgcctcagc ctcccaagta gctgggatta 60

caggtgtgag ccaccatgcc tggctaattt ttgtattatt agtagagacg gggtttcgcc 120

aggttgtcca ggctggtctc gaactcctga cctcaggtga tctgcccgcc tcggcctccc 180

aaagtgctgg gattacaggc gtgagccact gcacccagcc aggaaggctt tcttgatggc 240

tctgcagatg gccacctggg cttcctgagg cccagactgt cacagggagg agctggtgcc 300

acagaggcag atgagtgata accgagtgct gcttgttctg tctctagata cagcagcagc 360

aacagcagct gcagcgaata gcacagctgc agctccaaca acagcaacag cagcagcagc 420

agcagcagca gcagcagcag caggctttgc aggcccagcc accaattcag cagccaccga 480

tgcagcagcc acagcctccg ccctcccagg ctctgcccca gcagctgcag cagatgcatc 540

acacacagca ccaccagccg ccaccacagc cccagcagcc tccagttgct cagaaccaac 600

catcacaact cccgccacag tcgcagaccc agcctttggt gtcacaggcg caagctctcc 660

ctggacaaat gttgtatacc caaccaccac tgaaatttgt gagtacctgt ggcccacagt 720

ggagcacatg cagcccgtgg ctctgtcagc agtagtttct aggctctttg gctagagata 780

gcatatccta ttc 793

<210> 124
<211> 753
<212> DNA
<213> Artificial Sequence

<220>
<223> Marker CATCH42

<400> 124
tagccgggag cagtggctca tgcctgtaat cctagcactt tgggaggctg aggcgggcag 60

atcatctgag gtcaggagtt caagaccagc ctgggcaaca tggtaaaacc ctgtctctac 120

taaaaacaca aaaattagcc aggcatgatg gcaggcgcct gtaatcccag ctacttggga 180

ggctgaggca ggagaatcgc ttgaacccag gaggcagagg ttgcagcaag ctaagattga 240

gccactgcac tccagcctgg gcaacagagc aaaaactccg tctcaaaaaa aaaaaaaaaa 300

aaagagagag agagagatcg gttttgctat gttgcccaag ctggacacgg acacacacac 360

acacacacac acacacacac acacacacac acacacacac acaagctgga cacagagaca 420

cacacagtga cagggcaaag gttccaaaat tttaaacctg gtaaatctgg gtacgggtat 480

acaggagttg ttctactaca ctattctttc aacttttttg aaagtttgaa gttatttcaa 540

aagaaaaagt tttccaaact ttagtgatcc tcctgcctca gcctcccaaa gtgctgggat 600

gataggcatg agccaccgtg cctgacccct ctgtatattt ttagaatttc atgttaaaag 660

atggaaaagt ctggatgagg tagttcacgc ctgtcttccc agctctttgg gaggccaagg 720

tgggaagact gcttgaagcc agacgttcaa gac 753

<210> 125
<211> 736
<212> DNA
<213> Artificial Sequence

<220>
<223> Marker CATCH41

<400> 125
agttcaagac cagcctggcc agcatggtga aacccgtctc tactaaaaat acaaaacatt 60

agccagacgt ggtggtgggc acctgtagtc ccagctacta gggaggctga ggcaggagaa 120

tggcgtgaac ccaggaggcg gagcttgcag tgagctgaga ttgcgccact gcactccagc 180

ctgggcgaca gagcgagact ctgtctcaaa aaaaaaaaag ccatacacaa gctgttacca 240

ctaaatggga aaatgactga aaaatgtcaa tgtcaagagg gactgaaatc aaatttttcc 300

aatagtgggt tacatgatca gaaatccaaa tagacaggaa atatgttggc tttatttatt 360

tatttattta tttatttatt tatttagaca gagtctcact ctgtcaccca ggctggagta 420

cagtggcatg aactcggctc actgcaacct tcacctccca ggttcaagcg attgtcctgc 480

ctcagcctcc cgagtagctg ggactacaga tgtgtgccac cacacccagc taatttttgt 540

atttttagta gggacggggt tttaccatgt tggtcaggct ggtcttgaac tcctgacctc 600

aagtgatcca cccgccttgg cctctcaaag tgctgggatt acaggtgtga gccaccacac 660

ctggccggta ctggctttaa aaataacaaa agtaatacat acacatagaa aaaggtcaaa 720

caaagaagta cataga 736

<210> 126
<211> 747
<212> DNA
<213> Artificial Sequence

<220>
<223> Marker CATCH11

<400>  126
agtatatctt tcttttaaaa aataaaataa gtaggccagg agtggtggct cacacctgta      60

atcccagcac tttgggaggc tgaggcaggt ggatcacttg aaaccaggag tttgagacca     120

gcctggtgaa catggcaaaa actgatttct actaaaaata caaaaattag ccgtgcatgg     180

tggtgcacac ctgtaattcc agctgcttgg aaggctgagg caggagaata gcttgaaccc     240

aggagatgga agttgcagtg agccaaaatc acgccactgc actccagtct gggtgacaga     300

gtgagactct gtctcaaaaa taaaaataaa aataaaaaaa tttaaaaaaa acaaacacac     360

acacacacac acacacacac acacacacac acacacaaca aaaaaaacca cctctggaaa     420

ttagtttctt ccagaaggtc caaggggtaa ccataaagta aatgcaagga cccatgatct     480

ctgtgctctt tgttgagcaa taaaatcaaa tatttgtttc ctcctccata atataaccat     540

taactttcac taaaaataca cttattcttc tcatgtaaga ggtcacatca ggggagtatt     600

taaatgagca tgtgctaagc tatcacagat aacagttctg cctgtaccac ccaccccagc     660

agcccggaga ggcgcaggct cacacacgat cccacatgta ggaccaggag tccaacctgg     720

aaccctgtga ccctcaaatt tgtgtaa                                         747


<210>  127
<211>  736
<212>  DNA
<213>  Artificial Sequence

<220>
<223>  Marker CATCH12

<400>  127
ctgtagtccc agctactcag gaggctgagg cagaagaatt gcttgaaccc aggagacgga      60

ggttgcagtg agccgagatc atggcactgc actccagcct ggacaaaaga gcaaaatgct     120

gtctcaaaaa aaaaaaaaac aaaaaactga ttttgcttag atttgtggta ccagtgctaa     180

gcctttgtgt atcctgatcc tttttctctt agtgttttat atttaatctt gcaagtaaga     240

aggcttacaa agcaccacct cttctctctc ctctctggct ctttcctggc tgctgttcct     300

agtaaaaagg cagaaagcag ccctgggcat atctccctac ccccagcgtg cacaaacaca     360

cacacacata cacacacaca cacacagtca caaacctggg aaaaggcttt actcttcacc     420

ctgacagaat caattaagtc aagactcatc tctgcagtgg gtgcagtccc cccaggcaac     480

acagatccct gtctctccca cttacccaca ccatcaagaa gcatttgcag gtgccgtgac 540

tgtttgctca tgcctgcgtg tcatttcctc ctcctgcaga tgagctgtcc atgggactgg 600

gtcgtctgaa ggacatagcc ctggggatgc agacagaaat tgaggagcaa gatgacattc 660

ttgaccggct gacaaccaaa gtggacaagt tagatgtcaa cataaaaagc acagaaagaa 720

aagttcgaca actctg 736

<210> 128
<211> 745
<212> DNA
<213> Artificial Sequence

<220>
<223> Marker CATCH13

<400> 128
taggtcttaa gacatttttg ctgttataag gaagtgtttg tcccacattt tcctagggtt 60

aacacctcac caagttcttc cagcaaaatg cttattagag tttttgtctg agcacagtag 120

cctggccctg catatcttgg gcgtttgatt accatgctgg aataagaaga gttgcatttc 180

tcattttaat gagcatatgg aaagatggga acagaatttc agtcttgcag ccatgagcct 240

ctatttctca ttcctaagcc ttaccatgag tcagagtgtt aagggggcct gtgaaccagt 300

cgtttggtgg aggagggtcc ttcccacacc tttgtttagg agtcatcatt cacacacaca 360

cacacacaca cacacacaca cacacacaca cacactctct gagcacatta tctgtgattc 420

tttcatcctc ccagaaagga tcagtgttag aatgggaaat agagtgtgcc atcttgctct 480

tttgttggca gatgtccccg cttccctcca gccccccatc cttggcatgt atattagtag 540

gagggcttca caccagcact gctcactgcg actcctggaa atgtgtgggg aattgggtga 600

gcccctcttc cccacgtgct cccatctgta actctccatc tgtgatttgc aacaggtctt 660

ccactggctc gaggctgcga ggctgaccga caggggcgcc caatagagtt tgtgggaatg 720

gagtattgca cagatgcagc tgcac 745

<210> 129
<211> 775
<212> DNA
<213> Artificial Sequence

<220>
<223> Marker CATCH14

<400>  129
acaggctggg cacggtggct cacgcctgta atcctagcac tttgggaagc tgaggcaggc      60

agatcacctg aggtcaggag tttgagacca gcctggccaa catggtgaaa ccccgtttct     120

actaaaaata caaaaattag cagggcatgg cggcggacag ctgtaatccc agctactcgg     180

gaggctgagg caggagaatc gcttgaaccc gggaggcgga ggttgcagtg agccgagatc     240

gcgcccctgc actccaacct ggtgacagag cgagactccg tcgaaagaag gaaaggaaag     300

ggaaagggaa aggaaaggga aagggaaagg aaaggaagag agagagagag agaaagaaaa     360

gaaaagaaaa aagaaaagaa agaaagaaag aaagaaagaa agaaagaaag aaagaaagaa     420

agaaaacaaa caaaaacaaa tagggattaa taaataggca gaagcaggca taatccgggg     480

caggggaagc ctcggggcca cgcccgagag gcatttgtat tttactttca gcagttcaga     540

gcccatgata gccaggttac ccacttgtgg acttccttga ccacttaatt gagtcaatct     600

aaccctcacc ttctctctcc tctcactcca tttagcgggt cacgtagcca ctagggcttc     660

cttagcctat caggccacct ggggactgag tcattcagtc agtcacctat ctggtggtca     720

catctgcccc tgggctcatc cttttactgg gccacctcgg cccatcggga cctgt          775

<210>  130
<211>  746
<212>  DNA
<213>  Artificial Sequence

<220>
<223>  Marker CATCH39

<400>  130
cattgcactc cagcctgggc aaaaagagtg aaactccatc tcaaaaaaaa aaaaaaaaaa      60

aaaaagtccg ggcgcggtgg ctcacacctg taatcccagc actttgggag gccaaggtgg     120

gcggatcaca aagtcaggag atcgagacca tcctgactca cacggggaaa ccctgtcttt     180

atgaaaaata ccaaaaaatc ttagcccggc atggtggcag gcgcctgtag tcccagctac     240

tggggaggct gaggcaggag aatggcgtga acccaggagg cggagcttgc agtgagccaa     300

gatcgcgcca ctgcactcca gcctgggaga cagagcaaga gtccgtctca aaataaataa     360

ataaataaat aaataaataa ataaataaat aaataagact ggctatccca gagcctgggg     420

acaggagagt gtgcgctgca aagccagcca tttgaggagg gcctagtctg aactgaatag     480

gtcaatgggc tggagctgac tttggacaga gttgaacaac cccaaagaat ccagtgggtg 540

ctgggcatac agtcgtcagg cctgtgtaga cagacctggg ccgagaagaa gctgggctgc 600

agtagccagg gcaaggcaga ggggcaggaa caggcatggg cgatggggtt gggccccagt 660

tctttgacct ggggaatccc ccaacctatg cttccatatg aatgaatcac tgtgcatctg 720

tgggcaccac tgcatcagct tgcccc 746

<210> 131
<211> 1038
<212> DNA
<213> Artificial Sequence

<220>
<223> Marker CATCH38

<400> 131
caaggtctcc ttgccgatgg tataatggcc tctggagtaa ttattggctg catcttcctt 60

cccactgatc agctgctctg ggtggaagag ctgcctgtag gtccctgtgt gcacttcacc 120

tacaaagaga ccacatgtgc tgtgagtctt taaggcctac tgtacaccaa ggtgaacacg 180

ttccaggact gttcacttct acaaagtaca tgctgttcaa agtacatgag ctacatgttg 240

taggtcaaca gtggcggtgt ctggtagaaa atgtctctgt gataaccaat cacaaatgca 300

atttaaggcc ctaggaagtt aaagtcagtt cttgtataaa tgtcagcaca ggccgggcgc 360

ggtggctcac gcctgtaatc ccagcacttt gggaggccaa ggcgggcgga tcatgacgtc 420

aggagatcga gaccatcctg gctaacacgg tgaaaaccca tctctactaa aaatacaaaa 480

acaaaattag ctggacgtgg tggcgggcgc ctgtagtccc agctacttgg gaggctgagg 540

caggaggacg gcgtgaaccc aggaggcgga ggttgcagtg agccgagatc gtgccactgc 600

actccagcct gggcaacaga gcaagaatct ttctctaaat aaataaataa ataaataaat 660

aaataaataa ataaatacat aaataaatgt cagcataaac agttcccagt gaagggaaat 720

aagctctcgc atcgtccccc agcactggta attctacagg tacatacatc tttcctaccc 780

ttagctacta catcaaaggc tgtaagttaa aaatctctta caaactggac agtcaccagg 840

tcagtgtgac ttctgaaggg cagtcccatc aacctgtgtg gcctgggtcc cacgtgcttt 900

caggtgacat tgtcacctcc tgggggacac tgaaaggtac tatctctttg cagctctctt 960

tggaaactat ctgggacttc taatctggat gaaggaaatg gatgttctgt acttaccttc 1020

tacgcctaag atgcacat                                                    1038

<210>  132
<211>  737
<212>  DNA
<213>  Artificial Sequence

<220>
<223>  Marker CATCH37

<400>  132
ctcccgacca ctggggcagg gacaatgggc ccggctctga cttcgggcac gctcgtcgtg      60

tgcagagaca cggccgcaga gtcctggggc tcgctcgcca tcctctgccc ggcacacatc     120

gacttgagca tctggaagac ggccacgggg gccacgttca acttcacctg gctctgaagc     180

tggccgctgt gggctgcatg agaatggtgt ttctataaaa catgggaatt gctggtcaga     240

cacagtgact catgcccgca atcctaccat tttgggaggc tgaggcggga ggatcgcttg     300

agcctaggag tttgagacca gcctagggaa cacagcaaca cctcatctct acacacacac     360

acacacacac acacacacac acacacaatt agctgggcat gatggcatgc acttgtagtc     420

tcagctacta aggaggctga ggtgggagga tcacttcagc ccagattgag gctgcagtga     480

gccatgatca tgccattgca ttgcagctac ttgggaggct gaggcaggaa gatcatgtga     540

gcccaggagt ttgaggttgc agtgagctgt gcctctaatc ccagcacttt gggaggctga     600

ggtgggcgga tcacaaggtc aggagttcga gaccagcctg accaacatgg tgaaacccca     660

tctctactaa aagtatttaa aaaattaacc tggtgtggtg gcgggcgcct gtagtcccag     720

ttactcaaga ggctgag                                                    737

<210>  133
<211>  761
<212>  DNA
<213>  Artificial Sequence

<220>
<223>  Marker CATCH36

<400>  133
ccggggctga gcctgggtct gaagactgaa tgtaacaggg gccgctggca ctcctgcgcg      60

tcccccggct ctggcgctgc aggggtgtcg gcgcgccacc tctccaggcc ccagaagccc     120

ttcttgccca aaggcttccc tggtccctga gccctgcctc ggctgcctca ggggagagtg     180

cttttcctgt agtttccaag gaatattctt tgtttagagg tacttgtttt ttattaagag 240

aaaaaccagt gtaacgttta tgtgactgtt tgaactggaa ggtctggggt tccggggggt 300

ggggggaggc tagactcaat gccggggccg tcggtactct tgttttcatt tgtgtgtgcg 360

tgcgtgtgtg tgtgtgtatg tgcgtatgtg tgtgtctatg tgtggtgtgt gccgtatgca 420

tgggcagatg tttcgctttc cctccttcct cgctgaacaa ggtggagact tctgaccttt 480

tgctacctct tgaattcatg acaacgatat gttggtgact ggcagtggaa gcccttgaca 540

ctcttgtttt ccttcgctta gcacctagga agtcgtgtgc aggagccagc agggcgccaa 600

gtggcccagt gcctgcagct cacctgtggg ttttcccttt gagcctttgg ccacatccac 660

ctcgagggga ccctggcacc ccgggcacac aaacctctgt ggtataggtg aggggtgtga 720

ggccccagcc tggagccagg cactgggtgc tgacagggga a 761

<210> 134
<211> 733
<212> DNA
<213> Artificial Sequence

<220>
<223> Marker CATCH35

<400> 134
ctcacactgt tttataaaaa gacaattcag aattctggcc gagcgtggtg gtgcactact 60

gtaatcccag caatttggga ggagtgaggt gggggatcac ttgagctcag gtattcaaat 120

tagccaggca tgttggtgcg cacctgtagt cccagctact tgggaggcgt gagtgggaga 180

atctcttgag cccagagggt ggaggcaggc acctgtagtc ccagctactc gggaggctga 240

ggcaggagaa ttgcttgaac ccgggaggtg gagcttgcag tgagccgaga tcacgccact 300

gtactccagc ctgggcaaca gtacgagact ccatctcaaa aaataaaaag taataataat 360

aataataata ataataataa taaatcttta agttcaccaa taacaataga tacaaaatat 420

tccatcaaac gggtggaaca tcatttactc aactgttctt gctttttgaa atatttctcc 480

aatggggtca atttttaggt tattggtgca ttttgctttc tgactgagtc atcatggtgc 540

atcaccacaa atgggttatt tttaattggt ttgtatttat ttgtttgctt gcttatttag 600

agacagagtt gtgctctgtt gcccaggctg tagtgcagtg gcgcgatctc agcttactgc 660

aacctctgcc tcccaggttc aagcgatcac catgcctcag gcctcctgag gagctgggat 720

cacaggcaca tgc 733

<210> 135
<211> 770
<212> DNA
<213> Artificial Sequence

<220>
<223> Marker CATCH31

<400> 135
cttcccaaaa tacagcaact tggcatactg aatattttaa gctgaaggaa tttgagaaat 60

ggcatgggca ggcaggattt tctgaccttc ccctgtagta ggtataagac cctcctgtga 120

gaggtactcc ccctatacag gaaggaaagg catatcctta tttccaaaga tgaacgcttg 180

ccaagaggga tctgaacaca caggcccgct gttccgccat tttattacac ttggctcata 240

ctctcatact tcttttgtct tatcattatt tctccatgac taatcaaaat ttttttctct 300

ctctatatat aaacatatat atataatata tgtgtgtgtg tgtgtgtgtg agagagagag 360

agagagagaa agacagagag acagagagag acagagaaag aaagagagag ggaaagagag 420

gagctgtatc atgggtattt ttgttgatgt ctgaattaga aacaagcatg cacactatca 480

acattgtttg ggaaagttgt agccggtgca gtaagacaca taacaatagg gttgtctagg 540

gcaataaaaa gaggcaaacg attatgtaca tagtgtagga aaaaatggtg ttgcggctca 600

gaatgtcacc ccgaaatacg gaactttgaa ggtgtctctc tgacttttcc ctgcctttct 660

gtgtgagagc tagtcataaa ggaattcttt gaccttcctt tcctgaaagt aggcagtgag 720

acctccaccc catgtgacag gtgtcctgtc ctattaccag gaggaaagaa 770

<210> 136
<211> 733
<212> DNA
<213> Artificial Sequence

<220>
<223> Marker CATCH25

<400> 136
gaactcacat tggcatttga tcagtgactc tccagctgta atgagaatag aatggagagg 60

cctgggacag gaagacagga tgtggttgct catgccatcc tggtgggaag atctaggtga 120

gttactgtgg ggtaaagagg agagagcaga ttccttgtca ctttatgctg tagaatacat 180

tggacatgga gagggcttgg agcttgtgtg tgagaaaagc agatctatct cggctgactc 240

ccaacaaact ggctgagaat ttagatggag gattattatt aaataaaaga ggaagactga 300

aatgaatatt gtcatcgtat ttttttaccc agggttggga taggtggaaa tgtgtgtgtg 360

tgtgtgtgtg tgtgtgtgtg tgtttaaata ttcactaatt tttcaattca aaaatattta 420

ctgcgtgact actaagtaat aatattatac aggttttagc gttaccatgg agaatcaaat 480

tgtcaaggtc acaggcatct ttttgttttg tgtggatcac tgtgaaagat actatgctaa 540

attttttctg aatcatgcag atgaaagcat taaacacata cacaaacatt tacgtttgtg 600

tgtgtgttgt gtgtgtgtgt gtgtgtatgt gtaatctatc tcagcataca atgccactga 660

aactacactt gcggattagg tcctcggaaa aataatcgtg ttaacattca cgatgcaaat 720

agaatggtac ctg 733

<210> 137
<211> 733
<212> DNA
<213> Artificial Sequence

<220>
<223> Marker CATCH24

<400> 137
gaactcacat tggcatttga tcagtgactc tccagctgta atgagaatag aatggagagg 60

cctgggacag gaagacagga tgtggttgct catgccatcc tggtgggaag atctaggtga 120

gttactgtgg ggtaaagagg agagagcaga ttccttgtca ctttatgctg tagaatacat 180

tggacatgga gagggcttgg agcttgtgtg tgagaaaagc agatctatct cggctgactc 240

ccaacaaact ggctgagaat ttagatggag gattattatt aaataaaaga ggaagactga 300

aatgaatatt gtcatcgtat ttttttaccc agggttggga taggtggaaa tgtgtgtgtg 360

tgtgtgtgtg tgtgtgtgtg tgtttaaata ttcactaatt tttcaattca aaaatattta 420

ctgcgtgact actaagtaat aatattatac aggttttagc gttaccatgg agaatcaaat 480

tgtcaaggtc acaggcatct ttttgttttg tgtggatcac tgtgaaagat actatgctaa 540

attttttctg aatcatgcag atgaaagcat taaacacata cacaaacatt tacgtttgtg 600

tgtgtgttgt gtgtgtgtgt gtgtgtatgt gtaatctatc tcagcataca atgccactga 660

aactacactt gcggattagg tcctcggaaa aataatcgtg ttaacattca cgatgcaaat 720

agaatggtac ctg 733

<210> 138
<211> 738
<212> DNA
<213> Artificial Sequence

<220>
<223> Marker CATCH26

<400> 138
gtgttatgtt cttacaaagt caaaagaaag aaagaaagaa ctgttgagtc ataatgctat 60

atccaggtaa agattttttt tttttaaaaa aggtaaaaaa taagactttg tcagatgaag 120

aaaacctgaa agaattcatc taaaaaaaat caaaacaaat caactctaag agaaaatttt 180

caggtggaag ataaatgata atggaaacat tgaacccaaa gaatgcagga agaataacca 240

ataaatatag tagtaaaaga ccaaattttt agctctacag ttctggaaaa tatgcatatc 300

aaaagctaag ttatagcatt tttgatataa tttctactta tgaaaaatat tatatataca 360

tatatacaca tatatacata tatatacaca cacacacaca cacacacaca cacacacaca 420

cacacacata tatatatata catgaaactt caaccaaaag aaggaactgt aaggggtcta 480

catgactggg aaaatattag gcctattatt aaatctaaat agatcatgaa tagttacatg 540

tacatgttgt aatctttaca gcaaccaatg taaaactgtg cagagatacc aatataaaat 600

caatataaaa atcaaaagag cccactaaat aatactcaaa taatccagaa gtagcagaaa 660

cagggtggag aggaacaaaa gttataaaga gaaaaaggaa acttacacta aaattgtaag 720

cttaaatcca accatgtg 738

<210> 139
<211> 735
<212> DNA
<213> Artificial Sequence

<220>
<223> Marker CATCH30

<400> 139
gtcagcatag cagctatgcc attgaatgct ttcggcagca gccagggaag gcccatcagt 60

acctgatgga gcttaagagt gatggaagcc acaccaaggg ggctgggatt cccagtcact 120

agttctcagt ctccaggact ggagctgacc actatagtgt catttctaca atcccgtctg 180

aggatggagc tgactatatc tgtggtacag attgtagcat tggtgtgccg tttgggtaaa 240

gcacagataa atggggaaat gaggcaaaaa ctgtttttct actctgctac caaggttgaa 300

aaatggctct cagaaccagt gtctgctgac ctgcatactc aaatatgtta tgtgtgtgtg 360

tgtgtgtgaa tgcgtgtgtg tgtgtacttt aattttacga gtagagagcc agatattttc 420

tgattcctgc ttccagttcc cttggaaaca gctttttagg ccaggccttc tagggagtgc 480

actctgatct gtaactattg tgcaaatatt acaacagagc ctgtctcctc ctgctggtct 540

ggtgtattgt ggctatatag gggggtgcaa agaaattctc cttttcatat tcagacactg 600

ggactcagct gcatacctca gtcctgtctt attgagatca tttaaatgat caatcttaga 660

agaaaacttc tgacttaaat aaattttaga agtctatcaa catgctagaa agcatattcc 720

agataaataa aaata 735

<210> 140
<211> 728
<212> DNA
<213> Artificial Sequence

<220>
<223> Marker CATCH29

<400> 140
actcctaaaa accagaaaaa agctatctgg taaaatgctt tgtgttgtgc tgtttcatgt 60

gacagaatgg tgcctgtgtt ttgattaaac aagtttgaaa cacttttttt ttgtagaata 120

taagtagtga tgttttagaa tgaattgagc ccttataaga aaatgcaaat atttaaccat 180

aaaaataaga aacaaacaac ctgtgaaatc actttgtgat atcctttttt tatgtcacag 240

aatggtacct gtgttttgat ttaacaaatt tgaaacacac tttttgtaga atctgagatg 300

tgacatttac aaaccaatct taagccagtt agatactctg atgagccagc tgtgtgtgta 360

tgtgtgtatg tgtgtgtgat ctttgattat taaaactgaa aaaataatcg tatattcatg 420

actagtagac acagtgtttt aaaactgatt ctgaaagatt tgagggttag gcttgtttta 480

aaaacaattg cataggtcct ggaaacacca agaattagtg gcttctcatt cctagtcaca 540

tgaaaaatat tttactactc ttgtgaatgg ccataaataa tatgcagaat tttttctgtg 600

tcctaacctt ttatataatt ggttctacag tgtacatcac tgtgtgcaac tttgttttgt 660

gtgctcagac ttacattttc cagttttctc cactttgata caattggtct gtgtttcata 720

cattttgt                                                          728

<210>  141
<211>  744
<212>  DNA
<213>  Artificial Sequence

<220>
<223>  Marker CATCH15

<400>  141
taatattaaa tctactggaa agaatattct cctttgaaaa gattctcagc tcagtgatca      60

tagaacctgt acttgcgttt aattcaatga ggaaagacat agaaatgcca cgttaatatc     120

ctcatttagg tggtttctcc cttcactgat cagcctatga atgtgtcaat gcagtactcc     180

ccagtttagg cttaggtact tattgaaagg atgctgcctg agcattttta ataattgcta     240

tttcaagatc acattttggc catgaggcag gctgccactc actttaggct ctgcctaggc     300

aggaatgggt cctggaagac acatgttctc tctctctctc tctctctttc acacacacac     360

acacacacac acacacacac acacacacac acacccctct cttctctctg ggtacacatt     420

attctggaca tcttcagttt ccaggattct ctatgtctcc agctagaagt cagggcttag     480

cctgtggtga tccagccacc ctcaggttga atctcgatga agtcacagga accaccagtg     540

gcatggaggg taagggatcc tgctatcagg ttcagccagg aaatgctctg gacacctgct     600

ggtggaagag tttaatgctg ggcaatcaca agtcaggtcc cttcttccag tcggcagttc     660

agcctccctg actttctctg ggttccagtc agtgcatgag gatgatggtg acatttattc     720

aggggcagca cagcagcctc agtc                                            744

<210>  142
<211>  725
<212>  DNA
<213>  Artificial Sequence

<220>
<223>  Marker CATCH16

<400>  142
ctcctggccc tgagaaaagt gggctaaagt tctcccttct gcgtgtgtta cccagtggtt      60

tgcccccctt tcatcacctg tgaccaacat gggctcattg gggatatttg tgctgtcatt     120

tctggcacaa gaacccaaag atctaagacc ataaaatgca tttggtcttt catactgtct     180

attcccatca gggcagacat ctatccatta atacatttta tttaattttc ttgtcttata 240

ttttcagagc aatgatgagt aatgagtgag gtaaggggta cctttgcatt tttttgatct 300

cagggaaaat gtcctccatg cagtaagatg atgactccgg accaagacag gtgtgtgtgt 360

gtgtgtgtgt gtgtgcgcgc gcgcgcgcgt gcgcgtgcac acgtgcacac gcatgtgtgt 420

taccatatca taaacaaatt tatctgtttc tgtattctta cgtgattttt attagggata 480

gatattaaaa tttgccaaac acattaaaaa tctattgata caatatgatc tttggttgtg 540

atgtaataat atggtattat accaaaactt ttattaatgt tgaacccaac cattatttta 600

agtataaatc ccacttgaca atggtgtata aatgcgtaaa tctggtgttg aaatcagttc 660

gttcctcaaa tttcctggtt caagtgatcc tcccacctca gctccttgag tagccgggat 720

tccaa 725

<210> 143
<211> 721
<212> DNA
<213> Artificial Sequence

<220>
<223> Marker CATCH28

<400> 143
   ccaggagcat ggggagggtc cagggggttc ctgctgcatg gagggaagac cctctttcca 60
   caggggcccc ggagagcgag aggaaggagg agggcaggtc agtgagtgtg acggggtcac 120
   agtggagagg gaagcagaaa gaagtgtccc cacaacaaga cacacacagt gtctacgctg 180
   aagctacaga gagggcctct ccacctgtgt ctgccgcaaa gcagtggggc gtcttctggc 240
   agcccagagt cacctccaga tcccacctgc accatgcttc ctgcagggac tgcctgtctt 300
   cctaatacac tgtcttctga ccagagtctt ccagacaaat cactggttgc tatatatata 360
   tatatatata tttttaatag ctaatatctt acactgatat atttatatta tatataaata 420
   tttttgtact ttatgtttag gctatattac agatgtagtt agttagctat ttatgttata 480
   tataatataa acatgatgta gattcttata tttctctggg gactcactgt cttcttaata 540
   cactgtcttc tgaccaaatt cttccagaca aatcagctgt tgctatatat atatatatat 600
   gtgctatata tatatgtgct atatatatat gtgctatata ttttattgca tagaatatat 660
   tatatattaa ttatataata tacattatac attacatatt atatataata tattatatta 720
   t                                                                    721

<210>  144
<211>  728
<212>  DNA
<213>  Artificial Sequence

<220>
<223>  Marker CATCH17

<400>  144
agattttata aaaatggtgt tagtcaaggc atggcttaca aagatatttt caaggggctt      60

tactttccag caatcttacc agaaaacaac tgcataattt ccatgaacat tggactgtgc     120

ttcaagtatc ctccaaagga tctcacttac tgccctatga gtgacatggg ctgggatgct     180

ctggtagagc acactctggc tggtgtcttg gtgctagaaa tggattcaat cactggacaa     240

gaaagtcctt caggggcaaa cataaggaca ctgagggtca tcttattcta cctgccttct     300

ctccttactt gtatcctagg ccagcctatg ctagcgcaac tgtccacaga tctctctctc     360

tctttctgtc tctctctcgt gtgtgtgtat gtgtgtgtgt gtgtgcatgt gtgtatcaaa     420

tgatcaaaca tcttcattca aaagtccaag ccctgcagca tcatgagaca gtgctaccac     480

acatgaagaa aatgttcaaa gggcaccaaa tagagatcgc atgatccaaa gttatttctg     540

atcccagaaa agaatggcac acaatcaata tgaatagtat tagatatcaa aattatagta     600

caatctcatt tgtttttacc atagattcaa actctcttta gcatattgga aagcagaatc     660

catgttctac tgatgcaatt ccccttgacc atcatgaaag aattggactg aaagcaggac     720

atttctga                                                              728

<210>  145
<211>  744
<212>  DNA
<213>  Artificial Sequence

<220>
<223>  Marker CATCH18

<400>  145
ctttaacagg aagtacatac cggcagggaa atgcctgcct agtgcagtcc ccatacttct      60

ccggatgatc ctcactttat tatttatttt tatttttgta tgcactaaag tcgtcaatgt     120

aaatccttac gtcgaatgcc tccacttccc acattgtgta gcacagcctg gattcagttc     180

ttcagaaatg acgtcctctt caatgataaa ggaccactgc tctgtgaaca ccccactcta 240

gtgaagatag cccaccttgc tgggttattc atgtacatct tttgtggaag cagtaaccac 300

tgtgtaaagc tccaaagatg ttttaaagca atgaaaacaa agaggaagaa gtgtgtgtgt 360

gtgtgtgtgt gtgtgtgtgt gtgtgtgtgt gtgtagggtc catcctgttt gccctaatat 420

ctcaggttgc cgagctaggg agtatttgag tgacagactt ggtgccctct caggatcctc 480

ctcctctcac atcactgagt ccctgtcctg gaaacaccct acaggtggac aatctcccca 540

tagtatggaa gtttccaaaa tggctccaca gggaagagtt gagctgaaca cacccactct 600

ttctcatggg catcaaatat atctaatttt tctggggaaa tcatcaattt ctagctggaa 660

tcctgattaa tcgacagact gtgagaatta aaaattttaa aaatattcag acatatccag 720

agtccagtat ttaaattctc tgga 744

<210> 146
<211> 734
<212> DNA
<213> Artificial Sequence

<220>
<223> Marker CATCH33

<400> 146
ctttgtcctg ggcccatatc tcatggtttt gggtctactg tggaagtacc ttctctacag 60

aacaaagaca aagcaataga acccaaattc ttccaattcc ctttgggatc tattcccttc 120

ttctcctgcc taccagtatg aacttctatg ggattatgct gatgagaaaa aataaattac 180

atttaagtga ctgataccaa aaactggaat tacttcacag atgagaaacc tggtcccaga 240

tacagtgcac cttctggacc tcagtatctt caattttaaa atcattgttt tgaatctgat 300

ttctaattgt taagctgaag tgtttcttta aatttttaag ttggttaaaa tgtgtgtgtg 360

tgtgtgtgtg tgtgtgtgtg tgtgaaagaa cctgctccct catcctcaac agcctcatca 420

atctccaccc attacccatt tttattagtt gcttatcttt ccattatttc ctcatgcaaa 480

cacaaataca tattaattta tttacacgta ttagagtaaa gtgatttcta aatctgaaga 540

ggcatcagac tcatccaggg agctttttaa acaagtggac acttagtttc tattttatac 600

ctattgactg aacaaaaccc ctggagagtg gagcccagaa ctttgacaaa gttctctcag 660

gcagttttct gtgacagaca cctctcaggg tacttacccc agcccacatc acctttaccc 720

aatagggatc tgtc 734

<210> 147
<211> 997
<212> DNA
<213> Artificial Sequence

<220>
<223> Marker CATCH27

<400> 147
gcccgccgca aggattgccc ttaggaggac agagaaagca atggtccgta ccaagcaagg 60

tcataatgcc agatgtgctg tggcagatgg tgggagagga atcaaaaggt cctgggaggc 120

caggtgtggt ggcttatgcc tgtaatcccg gcaccttggg aggccactgt gggagaatgg 180

cttgaggcta ggagatcaag accagcctgg gcaatatagt gagacccttt ctgtacaaaa 240

gattaaaaaa aattagtcag gcatggtggc acactcctgt agtcccagct actagggagt 300

ctgaggtagg aggattgctt cagcctgggg agtttaagac tgtagccagc tgttccagcc 360

tgggcgacag agtgagaccc tgaaaaaaag aaagaaagag agagaaagag agagagagag 420

aaagaaagag agagatggag agaaagaaag gaaggaagga aggggaaggg aagggaggaa 480

gggaaggaag gagagaaaga aaaaagaaaa gaaagaaaga gagagggaga aagagagaaa 540

ggaaggaagg gagagagaga gggaaagaag gaaagaaaag aaaagaaaga aagagagaga 600

gagagagaag ggttctgaga gtgcttagct ggaaaggcaa taccaggtac tcatggacat 660

ctccccaagt gagagaattc tcaggagggc ctggggactg cagtctgtaa ttgattgata 720

agcggtgcac tggtgagaga aatccagcat ccttgagaaa ctcagtgggt agttctctag 780

accaggccag caggggaagc tggtaaggaa gttgatagca atacggatgg aaggaccatg 840

cagtgatagt gtcacattgt cagggcttaa gcataggagc caggtgtgtg gcaaaagatg 900

aagtggcagc aaggcacctg acattgaatg atggagtcag tcagtatagc atggcacctc 960

tcggggcaag atgcataagc caccaagaaa gggacag 997

<210> 148
<211> 733
<212> DNA
<213> Artificial Sequence

<220>
<223> Marker CATCH51

<400>  148
agtggggcgc tttttaaaaa cactttcttc acttagattc tggggctcca ctctcttcct      60

caccagcctc catttcccca cctgccctca ccctcctgat gttggaggcc ccaaaagttc     120

agcccttagg tctcctccct tccctccact ggcctgacag gtgatctcca ccaagctcat     180

tggcagtgga agaccacatg gagcctgcta ctgatcaagt gtgttcctca ccaaccacag     240

tggctcatgc ctataatggc agtactttcg gaggctgagg caggtggatc ccttgagccc     300

aggagcccaa gaccagactg ggcaacatgg taaaacctcc tctctctctc tgtgtgtgtg     360

tgtgtgtgtg tgtgtgtgtg tgtttagcca agtgtggtgg ccacacgcct gtagtcccag     420

gtacttgggg ggctcaggag ggaggatcgc ttaaacctgg gaagttgagg ctgcagtgag     480

cactccagcc tgggcaacag aacaaggctc tgtctcaaaa aaaaaaaagt gtgttcctca     540

gcacaaaccc tcctctgacc cccgagctct cacctacagc tgcccactcg cagtctcact     600

tggaggtcta cactttaaac tcaacacata ccaaaccaag cctctgtctc caccagctca     660

attaagggca ttgccatttt tcaggcacag gccaaatcct cagagtcctc cttgacccct     720

ctctttccct cac                                                        733


<210>  149
<211>  744
<212>  DNA
<213>  Artificial Sequence

<220>
<223>  Marker D22S420

<400>  149
caggcctgag ccccccgcgc ctggcattgt taattttttt ctctttccta gaacgtcatt      60

taaggaaaga agagagtttg gggctagtag ggagggaact gtgaagctaa ctgaggctga     120

taaagaaagg ccttccccta caaaaggcct ccctgaagca gggcctggct gcctagtgcc     180

gcagggagga ggggtgaggg gtgtttgtgc tgcagcctgg ggctggagcg gctgccattg     240

cctgtcaggg cacaaggctc ttgacagatc tcttgagcat tgagcagtgg tgtactgggg     300

agggcgttat ccatgaccag gtcaaaagtt cagtgggacc tacgttgaag gtgtgtgtgt     360

gtgtgtgtgt gtgtgtgtgt gtgtgtgtgt gtgtttgcga tatatgcata tgtaaagttc     420

attattccgt cagaattttc agtgtagaac acagcgacca catgagcttg aagctgctct     480

aaatgttttt taatgatttt tcatgtttaa ttttgctctc agcaagcatg gtgaaatatt 540

ttatggattc aaatagattt ttattttggt ttggggagca aacggtattg tgagtgtgac 600

tcaagcagtc tagtgcactg tgaagggctt tgagcttctt ggaagaaaga taccatagaa 660

cttcaaggaa atgagatcac attatctgtg gcttttgaag atgtttttca ttgaatgtcc 720

atctccaagt ccttcttttc catc 744

<210> 150
<211> 738
<212> DNA
<213> Artificial Sequence

<220>
<223> Marker D22S427

<400> 150
atgctatagt tagcatttat ggtgtagtca tagctccctc cagcccaaat tttcataaag 60

gggaaagatg aggtggttca agagccttgt cataatgaaa ggcaattcag ggccaccgtc 120

attactagta tcatcatcat caccatcatc atcatcttta tcccaacacc ttaagaaccc 180

gttcatgatt tatgtggtgg ttcacaaaga gaactcgaaa agtgaattta ttcagctatg 240

tttactgagc tcacctgatg cactcaacta gctatttttg gtagggaggg cactaagatg 300

aataacacaa ccccttcctt taaggtgttg ctgttttgta gagtgtttag acacacacac 360

acacacacac acacacacac acacacaccc ctatactcca aactagaaag tgataaatac 420

ggctgggcac ggtgactcac gcctgtaatt ccagcacttt gggaggccga ggcaggtgga 480

tcacgaggtc aggagttcga gaccagcctg aacaacatgg tgaaaccctg tctctactaa 540

agatacatat tagccgggca tggtggcgcc tgcctgtaat cccagctact caggaggctg 600

aggcaggaga atcgcttgaa cccaggaggc gaggttgcag tgagctcaga tcgcaccact 660

gcactccagc ctgggcaaca gagcgagact ttgtctcaaa aaaaaaaaaa aaagaaaaag 720

aaagtgataa atactatg 738

<210> 151
<211> 746
<212> DNA
<213> Artificial Sequence

<220>
<223> Marker D22S1638

<400>  151
gattgtccgt aatgataaag gattaaaaca attacaatag ccaaggcacc cttagaaaac      60

aatgtgggaa gacttcacgc tgcccagtaa gactttagag gcacgtagag cacatcataa     120

attcatatgt atctagtaac gcagccccag agaatataaa gggaaactga tagaactaaa     180

aggcgaaata ggcatatttg gaaattttaa cacattcctc tcattaacga atagaaaaag     240

caaatgaaat ttagtagagc tacagaacat gtaagtaatg ctattaacta ctttgtcctg     300

actgatccat aaaacactgc accagacaac agcaaattgc acattccctt tgtgtgtgtg     360

tgtgtgtgtg tgtgtgtgtg tgtgtgtgtg tgtgtgatgg agtccctctc tgtcacccag     420

actggagggt agtggcgtga tctcgactca ccacaacctc tgcctcccgg attcaagcga     480

ttctcctgcc tcagcctccc gagtagctgg gattacaggc atgtgccacc atgcttggct     540

aattttgtat tttcagtaga gacggggttt ctccatgctg gtcgggctgg tctcaaactc     600

ccgacctcag gtgatccacc cgcctcggcc tcccaaagtg ctgggattac aggtgtgggc     660

caccgcgcct ggcctgcaca ttccttttaa ttggccacag tatattcatc agaattggcc     720

acatgcactc tcaaaaaatt tcaatg                                          746

<210>  152
<211>  727
<212>  DNA
<213>  Artificial Sequence

<220>
<223>  Marker 22K48-2

<400>  152
gagccgagat cgcgcctgtt ccagcctggg caacagagcg agactctgtc tcaaaaaaat      60

aaaaataaaa aatatataaa aattgttgga actgttcagc atctcgactg cagtggtaga     120

tacatgaacc tgcacaggtg acaaaattgt atagaactaa acatacacac gcacaaataa     180

gtacaaacaa aactggggaa ataaatttta aaaaataggt agcatatgtc aatgtcaact     240

atagttttgc aaaatgttaa cattgggaga aactggacta agtccagaag taccttctca     300

gtatttcata caattcggtg tgaatctata atcatctcga taagaaataa acacacacac     360

acacacacac acacacaagg cctgttttta aaaaacgata tatatgtgtg tgtactaata     420

tataaggcag tagaggtaaa tatccaaaca ccagaggtta ggtcataata aaaatgattc     480

ttttcatgtg caggtgacaa tatgcacact ctataacaag caatccctct ggagaaactc 540

atcagccaaa cacactaatg tgacctatat gcaagtaact cctatggctg actgccttct 600

agacacctca ccctgatctt ctctataacg gtctgatctt tccaagaaac ctgctctacc 660

cactgtctgc cccaacacag ttaaggccag accttgagag gagaggccaa caatccttgc 720

acaatgg 727

<210> 153
<211> 730
<212> DNA
<213> Artificial Sequence

<220>
<223> Marker D22S1648

<400> 153
cacataacag gcacaacaat aaaaacttgt tgctgttgaa tggctggaga aagctcatga 60

cagaagcact acattcaaac accttcagta tcactggtgt aacaatcttt ggaaggcagg 120

ctcctgatct actgctgtgg ggactcagat ttcgccaact gcctgcaggt gggaagcaca 180

cccttggctg gttcacagcc atctgctgct gttatctcta ccactttcga ccacaaaaac 240

ttatccccat cttctttagt aagcatgaca caatgacatc tcataggtgt cagatgccta 300

agagattgaa aacatagcac aggaaggaag ctgttgcacc atacaaccat tgtgtgtgtg 360

tgtgtgtgtg tgtgtgtgtg cacgcgccac aaactaaagt ggaaaagtgt cacaggtata 420

tcatttagga gaccttaatc acttctcctg aagcatctgc caacatataa tcaacaaagt 480

tttgagattg ggcgcagtgg ctcactcctg taatcccagc actttgggag gccaaggcag 540

gcagatcact tgctctgagg aattcgagac cactttaggc aacatggtga accccatctc 600

tacaaattaa aaacaaaaaa caaaaattac ccagtagtgg tggtgcaagc ctgtagtctc 660

agctactcag gaggctgagg tgggaggatc tattgagctc gggaggtcga ggctgcagtg 720

agccgtgatc 730

<210> 154
<211> 791
<212> DNA
<213> Artificial Sequence

<220>
<223> Marker D22S941

<400>  154
gtacatgcca ccacacctgg ctaatttttg tatttttagt agagacgcat ttttgccatg      60

ttggccagac tggtcttgaa ctcctaacct caagtgatct gcctgccttg gcctcccaaa      120

gtgctgggat tacaggtgtg agccaccatg cccggcctgt tctatcttat ctcatacccc      180

atatttgacc ctaccttccc aacataccca taacccaaac tttcaggaga gatgcaaatg      240

ttccttaact ttatcaggga acaggttaca aagtacatta acttgtcaaa agtcaacaca      300

ttatacattt aaggtctgga cattttattt atacctgaaa agaatgtgtt tatatataca      360

cacacacaca cacacacaca tacacatata tatatataca tatatatatt acatatacat      420

ataaacacat atatacatgt aatgtattac ataaacacac acacacacag agtctccccc      480

accagctcca accatttctt gccatcacag ccatcaccat cctgatccag accatctctt      540

gcttggatta ctaccagtag ttggcattat aactggtctc ctggctcctc cctccctctg      600

gtcccccaca accaatctag tctccacgta gcagtcagaa agatctctaa aactcacgtc      660

agatcacagt agtcctctgc tccccttgca gtgagaatca aggccttcta gtagcctact      720

aggctctccc aatcagtctg ctattcactc tctggcctcc tctctcacag cccttgcctt      780

gctccaacca c                                                           791

<210>  155
<211>  745
<212>  DNA
<213>  Artificial Sequence

<220>
<223>  Marker D22S944

<400>  155
agcttccagc tggagccttc aaggacctgg agggggcagg accaacccct ccaccctccc      60

tgcccttcac cctcaagttc acctgcaggg ctgtcttctt gcttactaag gcctgggaga      120

gtttaattcc aaagcagagg atgatgactg gagaaacgga tgcctggttg agtcacttct      180

gggggctcag cattggaatc cctctgtaac ctcccagaag cagaagtggt ggcgtccaag      240

gctgcccagg cccctccagt gatgaggagc ccagacccta caacacgcct ttgccccctg      300

cacacccttc ctgctgacag ccccatccca tgctcctccc catctgaccc gtgtgtgtgt      360

gtgtgtgtgt gtgtgtgtgt gtgtgtgtgt gtgtgcgccc cccacccccg cagacctccc      420

gagacagctc tggcagcccc acctgccagg ggtcaagtgc aattcctctg gaagtagcat 480

ctttcacatg gaaacgttcc tttattttca gtagttatgg tcgtcagcga gccctcttaa 540

aggagtcagg tgtcctgctt ttgttaaaat gacgttgtct cctaactctt agagaagagg 600

aaaaatgtct ctgcccagct ggcatgctgg ggcacagacc actgaagcca aggctttgga 660

ctcgggggtt ctggaggctg gaggaaggga gaaggggatg cagcgcagcg tgttgtgtgt 720

gcatagtgct ccagggactc cagag 745

<210> 156
<211> 729
<212> DNA
<213> Artificial Sequence

<220>
<223> Marker D22S1623

<400> 156
ctttaaaaag gtgagtcttt ttcttttttt gagatggatt ctcactctgt cgcccaggct 60

agagggcagt ggcccgatct cggctcactg caacctctgc ctcctgggtt caagcaattc 120

gctgcctcag cctctcgagc agctgggatt ataggcgccc gcagccacac ctggctaagt 180

tttgtgtttt tagtagagac agggtttcac catgttgccc aggctggtct caatctcttg 240

acctcatgat ccacccgcct cggcctccca aaatgctggg attaccagcg tgagccaccg 300

tgcatggcca aaaaggtaaa tctcatagca tgtaaattac atgtcattaa acacacacac 360

acacacacac acacacacat gaggtgggtg tggtggctca ggcctgtaat cccagtactt 420

tcggaggcca aggcgagagg atagcttgag cccaggagtt tgtgacaagc ctgatcaaca 480

gagcaagacc ctatctctac aaaataaaaa ttaaaaaaaa aattagctgg gtatggtggc 540

atgcacctgt agtcctggct gcttggtaag gctgaggcag gaggatcgct tgaacccaaa 600

atctgaggct gtagtgagct attattacac cactacactc cagcttaggt gacagaggaa 660

gactccatct cttaaaaaac aaaaaaacaa aaaaaaaaaa ctaataaaat aaaaacatga 720

aatccaaac 729

<210> 157
<211> 746
<212> DNA
<213> Artificial Sequence

<220>
<223>  Marker D22S264

<400>  157
ctctgcgctc cagccgggtc gacctgccga gactccgcct ctaaaaccag caaataccgg      60

gaggctgagg gacgaggacc gctggagccc gggaggcgga ggttgcagtg agtagctggg     120

attacaggca ggcgccacca cgcccgggta atttttgtgt tattagtaga gacggggttc     180

caccatgttg accaggcagg tcttgaactc ctgacctcaa gtgatccacc ctccttggcc     240

tcccaaagtg ctgggattac aggcgtgagc cattgcgcct ggccaaataa gtaatttaag     300

caattttttt tgtttcagaa aaaaaattaa ctcataaagg agcccccccg acacacacac     360

acacacacac acacacacac acacacacac acacacggga agcagcagtg taattggaat     420

gcccactgct gaggctctcg aatgagtgtc atgtgtgcac attttgcccc tcacaatgca     480

cgtcagacta ttaataggaa gcagccgctg gaatacctct ggtggggtgt gtgcagcagg     540

cagctttgga attacggtat ccccaatgtg acagcaggac aatggcttct tcactggcac     600

tgtccaacca cagtgctgtg gttatgtctc gaggtccatt attgggcgaa ggtcactgca     660

gactcaggga ggcccaggag ctcaccccaa ccagcttctt taccaggtga gtgacctcag     720

ccctgcagct ggactcatgg agacaa                                          746


<210>  158
<211>  748
<212>  DNA
<213>  Artificial Sequence

<220>
<223>  Marker D22S311

<400>  158
gaagctgaag ctctctgcaa ccgggagggc acctggaaga tgtaaaaaca taaatcagat      60

aaccaggaac cagatactgg gtcacacctc tggccatcca attcaatatg ttccctctca     120

ccatgacctg ccaagggacc agtttacagc agctgccctc cttgagacga gtctcaaaag     180

aacagctatg ggcccaatga gtttgctgag aaacaccctt acctttcctt taagtaaatg     240

ttttggttca ataaacttaa atgtttcctg agccaatagc tagtgtgaga taacaaagcc     300

tctcaacatg acagattcaa ttttttattc cctaagtaaa tgtgataaat cacacacaca     360

cacacacaca cacacacaca cacacacaca cacacacaaa acactcggct gggcgaagtg     420

gctcaagtct gtaatcccag cacttttggg gggccaaggc agacggatca cctgaggtcg 480

agttcaagac cagcctggcc aacatggtga aaccccgtct ctactaaaaa tacaaaaatt 540

agctgggtgt ggtggcgcac acctgtaatc ccagccactt gggaggctga gacaggagaa 600

tagctcgaag ccaggaagtg gaggttccag tgagccgaga tcgcaccact gtactccagc 660

ctgggtgaca gaatgagacg ccatctcaaa aaaaaaaaaa aaaatcaatt ttgaatattg 720

aatgttgaat caaggaaaaa agagtatc 748

<210> 159
<211> 745
<212> DNA
<213> Artificial Sequence

<220>
<223> Marker D22S1709

<400> 159
atcccagcac ttcgggaggc tgaggcaggc agatcgcttg aggtcaggag tttgagccca 60

gactgaccaa catggtgaaa ccctgtctct actgaaaaca aaaagatgag ccagatgtgg 120

tgatgcacac ctataatccc agctactctg gaggctgagg caggaggatc ctttgaaccc 180

aggaggtgaa ggttgcagtg agctgagatc tcaccactgc actccagtct ggatgacaga 240

gcaagactct gtctcaaaaa aaaaaaaaaa gtaacatagc atgtcatggg ggatctgggc 300

tctgccagcc cctctcttcc aagttcagtg ctctcacccc aagcttggag cacacacaca 360

cacacacaca cacacacaca cacacacaca cacacggtac tatttttcac ccctgccttt 420

gttcctctgc tgttcttgct gaagtgtcca gctccggcca cctcacctgg gaagctccgc 480

tgagccctgc ccagatacac ctcccctccc agcctttccc tcccagggga gctggtgcac 540

actcactcac tgcgctgatc acaccacatt tcatgatgct tcctttccct cccctctccc 600

cagcaggcaa gttcttaaag gcaagggtgt gtcttgtgtg tctatatcaa tcggaggcct 660

ctcccttcct ttcttccctg acccagcagc ctcaccagag cattctgtct gactatctga 720

tggagacacc ttctgagccg aaaca 745

<210> 160
<211> 735
<212> DNA
<213> Artificial Sequence

<220>
<223>  Marker D22S446

<400>  160
cccaggctgg gatgcagtgg cctaatcttg gctcacggca acctctgcct ccccagttca      60

agctactgtc tcggcctccc aagtagctgg aattacaggt gtgtgccacc acaccaggct     120

aattttttgt atttttagta gagatggggt tttgccatgt tggccaggtt agtcttgaac     180

tcctgacctc aagtgatccg cctgcctcag cctctcaaag tgctgggatt ggccgggcgt     240

ggtggctcac gcctgtaatc ccggaacttt ggaaggccaa ggcaggcgaa tcaggaggtc     300

aggagatcga gaccatcctg gctaacatgg tgaaaccccg tctctactaa acacacacac     360

acacacacac acacacacac acacaaagtg ctgggattac aggcgtgagc caccacgccc     420

atccagacat gttacatttc agatcagtgg ttacccaagt ggagatcagg aacctggagt     480

gctggtgact tagtactggg agtcatggga tacaaatggc atttaaaatt aggagcctag     540

gcaagattac cctgggcctg agtgtagcta ggggcccaag gtactgatct gacccaaggg     600

aagggtctgg cagaagagac ggaagggatc aggtagggct ttggacctct aggcttgatg     660

gccacctgct gctgggcctc cgaggggcct ggaggcggtg gggaccgaat atgcagagag     720

aatgagaagt gagcg                                                      735


<210>  161
<211>  729
<212>  DNA
<213>  Artificial Sequence

<220>
<223>  Marker D22S539

<400>  161
agtgggtttc tcctgaagct ctctcggtcc acagcgatac atacttcggg ttccaggctg      60

cattaagtct ggactgaagg aaacaagttg gggggaactc actaccaggt cagtagcact     120

tagaattcag tattcttcct cactgtgccc atatcatttg ccgtttcaag ttcttagcgg     180

gatgcccttg cagctgtcca ggttttctag ctgcattcaa agggagagac acagagaagt     240

gtgctgactc caccttccct agcatatttt attctgcagg atgtccccaa cccagggatg     300

cattatggct gtaggctgta tgcatttttg cacttttcag tcccttcttt cacacacaca     360

cacacacaca cacacacacg aaaaaaagaa aaaatttcaa tacgtttcat attgcattgg     420

tatgaagata aatataatca aggttggatt cattattgtt atgctttttt ttctgatttc 480

aaaataaatt aaaaccaaag cagccgggcg cagtggctta tgcctgtaat cccagccctt 540

tgggaggccg aggtgggtgg atcacttgag gttaggagtt tgaaaccagc ctggccaata 600

tggtgaaacc ccatctctac taaaaataca aaaattagct gggcatagtg gcacatgcct 660

gtaatcccag ctacttggga ggctgaggca tgagaattac ttgaacccca gaggcggaga 720

ttgcagtaa 729

<210> 162
<211> 724
<212> DNA
<213> Artificial Sequence

<220>
<223> Marker D22S308

<400> 162
cataaaataa agaactcacg gggcagaaac ttaagacaca cctaaatcaa atctttgttt 60

tcagcattat ctttcattac tattctttgt tacaaaaaaa cattgctatc tgtccttttc 120

tatatgttct ctacattaaa acttattatt tgtatacata ctgttcccta catctgcagt 180

gccattccca gctcacatat ctgccctctt aaatttatat caaattcaac tcccttcaaa 240

aagccttctc agttgcttta acccaagcca gtagtctgtc ttctagaaaa cagttgatgc 300

cgatgcctcc ttttccttta gcaactgaaa ataatattat agttatttaa tgtgtgtgtg 360

tgtgtgtgtg tgtgcataac atcatactat ttagtagtat ctatatctat caagtatctg 420

ctacagtgga tgatagatga cttaaagaca gaaatatgtt attcatatct acttctcaca 480

ggtctagtgc tgttgcagga tattttttaa ggaatcagag agactgatgg ggttcaggag 540

gatatttatt atttaggtgc accagcccag tcggattaac atccaaagga ctgagccctg 600

aacaaagagt taagttacct tttaagcatt ttgtagggtg ggaggagatc tgtgcagggg 660

gaagcatgtt agagaagtga gaaacaaaga cagttattca attaattgag aaatgcgtta 720

catc 724

<210> 163
<211> 744
<212> DNA
<213> Artificial Sequence

<220>
<223>  Marker D22S306

<400>  163
tcaaaggaac ttgaactaca atgtttcctg ggcacagaat gagaaacact tggaattggg      60

gaagttccca tcctaacaac ttagactcag gaagaagaac cctggagctt ctccagcatg     120

gccagaatct cagtccactt caaaatcctc agtctttaga tcaggagttc ttttagggat     180

gaggtcaagg tttctgggtg cacattacca ctgcctttca gtcctatcat ctgggcatga     240

ggagcttgtt atctcgttct atccattatt tctaaacctc cttgtagagg gctgggttag     300

agagtgctaa ctgccattta tacagtctgg acctttgtat aaaattataa acacacacac     360

atacacacac acacacaaac acaaacacac atacttattt tgatgttcca gcgaaagagt     420

aaaactaaac tcaacagaaa cagaaacaga tttgaataat acaagacaat ctcattaatg     480

aacatagaag caaaactctc tcagaatatt agcaattaga gtccatttta tatcaatgaa     540

ggattatctt gagcatctgg gaggatgtgg gttgagacat ttgcacagtc attggaagac     600

agtgaacatg atttttgaaa aaacagcatg tttagagata tcaaccagag gtcagatgct     660

tcaatcagag aaagatcctc tgagttctca gggctgcttc cctgaaaagc tacgggagaa     720

ggaaatggag tgactggaca caat                                            744


<210>  164
<211>  767
<212>  DNA
<213>  Artificial Sequence

<220>
<223>  Marker D22S686

<400>  164
gctcaagacc agcctgggca acatgatgaa actccatctc taccaaaagt tataaaactt      60

tgccaggcat ggtggcactg cctgtagtcc catctactca agagactgag atgggaggat     120

tgctggagcc agagaagttg aggctgcaga aggtaaactg tagaggctaa aacatgataa     180

atatttaatt catcacttaa aagaaatgtt ggttacattg aaaagaaatt tgattaatgt     240

aattagatat atttctgttt aaaaaataat tgaagtaagc cctgttagca ccactgcatt     300

ccagcctggg caacagagca aaacctgtta aagaaagaaa gaaagaaaga gaaggaagga     360

tgaggaagga aggaaggaag gaaggaagga aggaaggaag gaaggaagga acgaagggga     420

aagaaaaaaa ggaagaagat atttcaagct ttacagtacc agagccactc tgtaatcaat 480

aagcaacagg accaaaacag aaagagattc tgtagaacag tctaaccagc gatacaatta 540

tacatttctg ttcagatatc aaaaccattt tcaatagtaa atatatggaa tgaactcccc 600

acagagatca agtaggatgt atgatgtgtg aatgaaatta tgagttgaga gtaggaaatt 660

tataaattta atcaaacaaa ttaaagcatt atgaggaagt tgtactgcta atgtattact 720

tggcaagctg ttcagtggaa caccaagata ttcttaagga agttgca 767

<210> 165
<211> 740
<212> DNA
<213> Artificial Sequence

<220>
<223> Marker D22S425

<400> 165
tcatgcttta atttaagacc ctgaatatca aattgaagtg ccttctagct gaacgctcaa 60

ttgtaaaaca ttcccagtca cacactccac acttctttcc tgagaatgta tcctgaaacc 120

ctactgcatg gaatcctccc tctcagagta ggattgcagg gaactcaaaa attaaaaaca 180

aacaaacaaa aaacagtcta gagggaaaag tattgaagag gagagagaga gtgcacaagg 240

agacaactct gaagtctgca aatattcatg cactgaagag cgcttgtgtg tacagaaact 300

ccctctgaaa gagaaagacc cttgagggaa cactccaaag agcgcgcgcg cacgcgcgcg 360

cacacacaca cacacacaca cacacacaca ttccagtcac agaggaaagg cctgagttat 420

ggggcatgag gtggactcat cagaagagct tcggctcagt attgaggaaa atgtcgccct 480

agacataggg ctactctgct ctcaactaag aaagtgtaga agcaaactga gaaaagttaa 540

gacagtttcc aagtaacttt actgaatcca aaaataaaag acgaaaatag agaagaaaat 600

gcaaaagaat tataaactca acaaagaaaa cttcacaatg attggcattc aatcaacatt 660

tcctgcaagg aaaaaagaaa atatatgctc taacacagag aaaaatgaat caatccaaag 720

tggcaataaa attgtatacg 740

<210> 166
<211> 728
<212> DNA
<213> Artificial Sequence

<220>
<223>  Marker D22S257

<400>  166
ctacacaaac aaactcctgg gcttggcacc ctcctgtgcc cacatccggc gccctttctt      60

tgggacctgc cggagcctgg gtcccctggc agggtggggc aggatagggt ggggtgttgc     120

ctgagaaatg gtgcaaacct tcttgcactc cgtaaatgat tcatttcctt caaatgtcct     180

atgctgtttt ccagcagaat ctccattatg tatgaatcaa gatcctggct gacattttct     240

gccttggctg ttgtgagctt tcccgtcaga aatatccttc ccatggggcc tgaagtaatt     300

attttatatt tattggcatt aagtgtatga gagagagaga gagagagaga gtgtgtgtgt     360

gtgtgtgtgt gtgtgtgtca gaagtggtgt atcagctgcc acatagtaag agagcaaagg     420

atcttctttt gtcttatggt gttttacctg tgctgattgg ttcttggcag cctttaaatg     480

cctaggcaga gaggaagaga gaggttccta agcttcatgg tgaatttcta agtctgtgtt     540

gactgcagga aatgtatgag gaaaggatgg tggttgaggc ctctccctca catttaggcc     600

aggggttggc aaattgcagc ttagtcctgt ctgctgcctg tttttgtaaa taaaatttta     660

ttggaacaca gcttgctcat tggcttatgt ataacatttg gctgctttca tgctatgatg     720

acaaggtt                                                              728


<210>  167
<211>  748
<212>  DNA
<213>  Artificial Sequence

<220>
<223>  Marker D22S1174

<400>  167
gaaaagagca gttctttact ccctggattt tctctcatgg tttccagagt gtttctgatg      60

aatgctgtca ctgttgctta tagcagtctt gtgaggcagg ctgggcagcc acgattgtca     120

attttgtagg caggaaaacc aaggacattg gtagagatcc agatgtccat catttggttc     180

agcccaggac ttgaagcttc tgaaggccct gtgcacgttg gctttgccct ttctgaggct     240

atgtgcccag tctctaccag tctcacgtgc ccagcaagcc tcttggagtg tggacagaat     300

ctgctgcttg cttttttctg cctttgtgat tttttttcct gattataaaa gtgtgtgtgt     360

gtgtgtgtgt gtgtgtgtgt gtgtgtgtgt gtgtgtgttt taaagagatt caaacaactc     420

agaagtgtac agtgtgaatg tgaaggcccc tagtgattcc agtatgcccc tccataatcc 480

ctaagagttt gctatgtccc tgtatgtccg aattttttcc tattaacata taaacatata 540

gtaacatttt cttatgattt tgcagcttgc cttttcgctg gacaaaattt aatggaaggt 600

gtggtgagct tccctgcctg ccctagtgcc ctgtagttta gcagggcgtt tgtttatctg 660

cacggaccct tgataggttt ggtttcctct ccacttcttg ctatgacaag gcacaggcag 720

tgcatgtctt tgtacttgaa gcagggag 748

## Description

### FIELD OF INVENTION

The present invention relates to a method for detecting duplications and/or deletions in chromosome 22, and particularly in the chromosome region 22q11.2, which is based on determining the number of alleles present in a set of genetic markers located in regions of the chromosome 22-specific low copy repeats (LCR22s) and to be applied for the diagnosis of the genetic causes of congenital heart diseases, and in prenatal diagnosis as well. The present invention further relates to the primer pairs used for detecting the genetic markers and thus detecting the number of alleles, the kit comprised of such primer pairs and their use for the diagnosis of pathologies related to chromosome modification in region 22q11.2, such as congenital heart diseases, schizophrenia, immunodeficiency, etc.

### STATE OF ART

Congenital heart diseases may be defined as heart anomalies that originate in prenatal life and, consequently, they are caused due to problems during development. Congenital heart diseases are the most frequent birth defects and account for around 1% of births. One of the most known genetic causes of congenital heart diseases, which is responsible for 2% of these cases, is deletion or duplication in chromosome region 22q11.2 (dup/del22q11.2), with an estimated incidence of 1/2000-4000 births.

22q11.2DS is the microdeletion syndrome most frequent in humans, which is caused by the loss of about 40 genes in chromosome region 22q11.2. DiGeorge syndrome and velocardiofacial syndrome (VCF), both included in 22q11.2DS, are associated to a clinical picture that may include hearth birth defects, immunodeficiency caused by the absence or hypoplesia of the thymus, moderate mental retardation, velopharyngeal insufficiency, cleft lip, mental disorders such as schizophrenia, dysmorphic features, hypoparathyroidism, and hypocalcaemia. About 80-90% of patients with DiGeorge/VCF syndrome, independently of their symptoms, carry an identical deletion of 3 Mb at chromosome region 22q11.2, while a 5% carry an identical deletion of 1.5 Mb in that same region. Nevertheless, despite the genetic homogeneity of the people affected, there is a significant variability in the presence of some or all clinical features even in affected individuals of the same family. We can observe people with no clinical features, with just one, or with some or all of the aforementioned clinical features.

There is a characteristic present in practically every patient with deletions or duplications in the 22q11.2 region, independently of their size. This is that they are caused by an abnormal recombination between repeated regions of the chromosome, known as Low Copy Repeats (LCRs), as there are few of them in the genome. There are 8 LCR22s in the 22q11.2 region that could mediate a deletion or a duplication between them and, consequently, they would determine a total of 7 intervals liable of gaining or losing them with certain frequency (Yamagishi, 2002. Keio J Med, 2: 77-88).

It is important to detect a deletion or a duplication in locus 22q11.2, particularly those associated with a congenital heart disease, due to the following reasons:
1) Because it allows for the evaluation of other clinical features associated with the syndrome,
2) Becuase it allows for an early diagnosis and for genetic counseling, and
3) Because congenital heart diseases caused by a deletion at the 22q11.2 locus are associated with a higher mortality during surgery, especially because of immunodeficiency and hypocalcaemia (Mahle et al. Ann Thorac Surg. 2003;76(2):567-71).

Thus, early detecting the presence of a deletion and/or a duplication at the 22q11.2 locus (del/dup22q11.2) will allow for not only the early diagnosis and treatment for congenital heart diseases, but also for the early treatment of related non-heart abnormalities, as well as for genetic counseling for families on the recurrence of the hereditary or de novo mutation.

Nowadays, congenital heart diseases are one of the leading causes of neonatal mortality and morbidity associated with birth defects, which affect around 1% of newborns. They are six times more common than chromosome anomalies and four times more common than neural tube defects. Despite the progress achieved, perinatal mortality in newborns with congenital heart diseases is still considerably high (9.2% at Heart Disease and Stroke Statistics 2016). Moreover, people with congenital heart diseases are ten times more likely than the general population to suffer heart failure (HF). One quarter of people with congenital heart diseases are especially likely to develop HF in their 30s; and those with congenital heart diseases along with Tetralogy of Fallot and transposition of the great arteries have an 80% of probability of developing HF in their 50s. Congenital heart diseases may appear as isolated malformations or in conjunction with other birth defects. It has been estimated that their a priori recurrence risk ranges from 3% to 9%. Also, congenital heart diseases have a high cost for the public healthcare system, as it covers the costs of surgical procedures as well as the required treatment after surgery. For all these reasons, it is of great importance to identify clear genetic markers so as to make an early in utero diagnosis of congenital heart diseases as well as of other pathologies related to deletion or duplication in the 22q11.2 chromosome region on which to act upon.

Nowadays, the techniques employed for prenatal diagnosis (mostly involving ultrasounds) allow us to detect more than 90% of congenital heart diseases before birth (Allan 2007. Am J Med Genet C Semin Med Genet; 145C(1):73-76). These can be detected by an increase in nuchal translucency during the first trimester or by direct visualization of the heart defect during the second trimester. Another technique consists in performing genetic analysis on DNA extracted from any biological sample; being common nowadays to perform this in prenatal samples, such as amniotic fluid or chronionic biopsy, extracted between weeks 10 and 20 of pregnancy. When a deletion or duplication in the 22q11.2 chromosome region is confirmed, this may entail for the newborn not only a congenital heart disease, but also an additional series of clinical features, which can be more or less serious, that may pass down to their descendants.

Historically, the presence of a del22q11.2 was determined using fluorescence in situ hybridization (FISH) with a single probe (TUPLE1 o N25) that hybridizes a shared region between the most frequent 1.5 and 3 Mb deletions. Thus, FISH determination has a sensitivity to detect the most frequent known deletions and an a priori coverage of 95% for patients with del22q11.2. However, this test does not detect deletions outside the region the probe hybridized. Taking into account that most previous studies on del22q11.2 detection were based on FISH, a distortion on the frequency of atypical deletions may be possible, thus these can be actually more frequent than today's estimations. Were this the case, the FISH sensitivity with the TUPLE1 probe might be considerably less than the anticipated 95%. In fact, atypical deletions and duplications are more frequent than what was previously believed, and their phenotypes are not easily distinguishable from the phenotypes that cause the "classic" 1.5 and 3 Mb deletions and duplications. All this seems to indicate that the typical features of del22q11.2 might be caused by deletions or duplications between any LCR22 of the region, many of which cannot be detected by the FISH technique with a TUPLE1 probe.

There are molecular methods that are a substitute or a complement to the FISH technique. These methods are grouped depending on the molecular technique they are based on. The following techniques stand out: RT-PCR (Hwang et al. BMC Med Genet, 2014; 15:106), pyrosequencing (Koontz et al. J Mol Diagn, 2014,16: 5), MALDI-TOF (Kobrynski. Clin. Chem., 2016, 62:1), MLPA (Multiple Ligation-dependent Probe Amplification) (Zhang et al. BMC Genomics, 2015, 16:364), microarrays (Beaujard et al. Eur J Med Genet, 2009, 52: 321-327), STR (short tandem repeats) or microsatellite analysis, and others.

There is a lot of information on prior art in relation to the use of STR markers for detecting deletions in the 222q11.2 chromosome region, but these markers are aimed at identifying regions or intervals from already well-known LCR, thus identifying only typical deletions (Bonnet et al. Am.J.Med. Genet, 1997, 68, 182-184; Driscoll et al. Gen. Testing, 1997, 1, 2; Vittorini et al. Clin Chem Lab Med 2001; 39(12):1249-1258; Yang et al. Electrophoresis, 2009, 30, 465-471). None of the previously described STR markers detect atypical deletions in the 22q11.2 region.

On the other hand, an assay for detecting the presence of chromosome abnormalities is described in international patent application No. WO2008070249; for example, the detection of deletions in the 22q11.2 chromosome region in a prenatal diagnosis by means of different STR markers on different chromosome regions, such as D22S420, D22S446, D22S689, D22S685, AMXY, and DXS8377. Also, a method for the diagnosis of DiGeorge syndrome is described in international patent application No. WO1993007293A1, wherein the DiGeorge syndrome diagnosis is comprised by the identification of functional copies within the loci of the DiGeorge critical region, which corresponds to a region of 5 Mb of chromosome 22 that is flanked by D22S259 and D22S43 polymorphisms and identified by D22S75 and D22S259 markers.

All these techniques and markers also allow for determining the presence of deletions and duplications in the 222q11.2 chromosome region, but these are limited to classical deletions and duplications in the 22q11.2 region as the probes, primers or SNP are designed for already known regions, thus not being useful for identifying atypical deletions and duplications.

Therefore, there is a need of developing a method that allows for a quick, simple, and inexpensive detection, both with the presence or absence of maternal DNA samples, of every possible deletion and/or duplication in the 222q11.2 region, including both typical and atypical, as well as for the detection of maternal contamination in prenatal testing, such as in amniocentesis and in chorion biopsy

### DESCRIPTION OF THE INVENTION

The present invention solves the previously mentioned problems related to the identification of both typical and atypical deletions and/or duplications in the 22q11.2 chromosome region by means of specific markers aimed at identifying the areas in such chromosome region that have not been described yet. Thus, the present invention has identified 38 new STR specifically designed that are strategically distributed on every inter-LCR22 interval so as to detect any atypical deletion and/or duplication in the 22q11.2 region.

The LCR22 regions present in the 22q11.2 chromosome region have different names; **Table 1** (on the first and second columns) shows two of the most common names for each of these regions. Moreover, **Table 1** also shows the delimitations of every LCR region as well as the delimitations of the inter-LCR regions within the 22q11.2 region, which are obtained from the version NCBI37/hg19 of the sequence of the human genome available at UCSC (http://genome.ucsc.edu/).

**Table 1. Delimitation of LCR22s regions within the 22q11.2 chromosome region**

| **LCR22 REGIONS*** | | **LOCALISATION IN CHROMOSOME 22 (pb)** |
|---|---|---|
| LCR22-2 | LCR22A | 18,642,491-19,023,012 |
| interLCR22-2-3A | Inter-LCR-A-B | 19,023,013-20,249,558 |
| LCR22-3A | LCR22B | 20,249,559-20,731,985 |
| interLCR22-3A-3B | Inter-LCR22B-C | 20,731,986-21,021,868 |
| LCR22-3B | LCR22C | 21,021,869-21,074,326 |
| interLCR22-3B-4A | Inter-LCR22C-D | 21,074,327-21,465,673 |
| LCR22-4A | LCR22D | 21,465,674-21,917,116 |
| interLCR22-4A-4B | Inter-LCR22D-E | 21,917,117-22,963,078 |
| LCR22-4B | LCR22E | 22,963,079-22,997,578 |
| interLCR22-4B-5 | Inter-LCR22E-F | 22,997,579-23,649,112 |
| LCR22-5 | LCR22F | 23,649,113-24,021,303 |

| | | |
|---|---|---|
| *Only the two most common names used to identify the different LCR regions within the 22q11.2 chromosome region are shown. | | |

Thus, a first aspect of the present invention relates to an in vitro method to detect duplications and/or deletions in the 22q11.2 chromosome region (del/dup22q11.2) - from now on, first in vitro method of the invention- in an isolated biological sample of a subject, preferably an ADN sample, so as to determine the number of alleles that are present in the genetic markers located in the following regions in chromosome 22:
(i) the previous region to the LCR22A region located between positions 18,642,491-19,023,012 pb of chromosome 22;
(ii) the inter-LCR22A-B region delimited by LCR22A and LCR22B located in positions 18,642,491-19,023,012 pb and 20,249,559-20,731,985 pb of chromosome 22, respectively;
(iii) the inter-LCR22B-C region delimited by LCR22B and LCR22C located in positions 20,249,559-20,731,985 pb and 21,021,869-21,074,326 pb of chromosome 22, respectively;
(iv)the inter-LCR22C-D region delimited by LCR22C and LCR22D located in positions 21,021,869-21,074,326 pb and 21,465,674-21,917,116 pb of chromosome 22, respectively; and
(v) the inter-LCR22D-E region delimited by LCR22D and LCR22E located in positions 21,465,674-21,917,116 pb and 22,963,079-22,997,578 pb of chromosome 22, respectively;
wherein a number of alleles of less than two in at least one of the markers from at least one of the regions (i) to (v) indicates a deletion in the 22q11.2 region, and wherein a number of alleles equal to or greater than three in at least one of the markers from at least one of the regions (i) to (v) indicates a duplication in the 22q11.2 region.

The identification of the previously mentioned regions is made through genetic markers, preferably polymorphic markers, and more preferably STR markers (short tandem repeats). Once these STR markers have been identified, primers are designed for identifying the number of alleles that an individual has for such marker in that specific DNA region where it is located. Thus, by identifying the number of alleles of at least one marker from each inter-LCR22 region and/or from the LCR22 regions previously mentioned, and available in Table 1, it is possible to detect any deletion/duplication, both typical and atypical, that a subject may present in the 22q11.2 chromosome region.

Thus, in a preferred embodiment of the method of the present invention, the markers identifying each region from i) to v) of chromosome 22 that are selected of the list consisting of:
(i) CATCH5 (SEQ ID NO: 111), CATCH53 (SEQ ID NO: 112), and CATCH52 (SEQ ID NO: 113) are markers that identify the previous region to the LCR22A region;
(ii) CATCH4 (SEQ ID NO: 114), CATCH6 (SEQ ID NO: 115), CATCH48 (SEQ ID NO: 116), CATCH22 (SEQ ID NO: 117), CATCH19 (SEQ ID NO: 118), CATCH7 (SEQ ID NO: 119), and CATCH23 (SEQ ID NO: 120) are markers that identify the inter-LCR22A-B region;
(iii) CATCH45 (SEQ ID NO: 121), CATCH10 (SEQ ID NO: 122), and CATCH20 (SEQ ID NO: 123) are markers that identify the inter-LCR22B-C region;
(iv) CATCH42 (SEQ ID NO: 124), CATCH41 (SEQ ID NO: 125), CATCH11 (SEQ ID NO: 126), CATCH12 (SEQ ID NO: 127), CATCH13 (SEQ ID NO: 128), CATCH14 (SEQ ID NO: 129), CATCH39 (SEQ ID NO: 130), CATCH38 (SEQ ID NO: 131), and CATCH37 (SEQ ID NO: 132) are markers that identify the inter-LCR22C-D region; and
(v) CATCH36 (SEQ ID NO: 133), CATCH35 (SEQ ID NO: 134), CATCH31 (SEQ ID NO: 135), CATCH25 (SEQ ID NO: 136), CATCH24 (SEQ ID NO: 137), CATCH26 (SEQ ID NO: 138), CATCH30 (SEQ ID NO: 139), CATCH29 (SEQ ID NO: 140), CATCH15 (SEQ ID NO: 141), CATCH16 (SEQ ID NO: 142), CATCH28 (SEQ ID NO: 143), CATCH17 (SEQ ID NO: 144), CATCH18 (SEQ ID NO: 145), CATCH33 (SEQ ID NO: 146), CATCH27 (SEQ ID NO: 147), and CATCH51 (SEQ ID NO: 148) are markers that identify the inter-LCR22D-E region.

All these markers can be used independently, or in combination thereof, or even in combination with another known markers that have been described on prior art, in order to determine the presence of deletions and/or duplications in the 222q11.2 region.

In another aspect, the invention relates to the use of the in vitro method of the present invention for the diagnosis of pathologies associated with deletions and/or duplications of the 22q11.2 region, such as congenital heart diseases, DiGeorge syndrome / 22q11.2, velocardiofacial syndrome, schizophrenia, mental retardation, immunodeficiency, etc. -from now on, in vitro method for diagnosis of the invention-in which in case of detecting a duplication and/or deletion in the DNA of an individual's biological sample, then that individual has a high probability of suffering this kind of pathologies.

In a particular embodiment of the in vitro method for diagnosis of the invention, such diagnosis is a prenatal diagnosis, consisting in the performance of the in vitro diagnosis object of the present invention from an isolated biological sample containing fetal cells, which may also contain maternal DNA, in which in case of detecting a duplication and/or deletion in the DNA from the fetus, then the fetus has a high probability of suffering this kind of pathologies.

In another aspect, the invention relates to a primer pair able to independently identify the number of alleles present in the following markers: CATCH5 (SEQ ID NO: 111), CATCH53 (SEQ ID NO: 112), CATCH52 (SEQ ID NO: 113), CATCH4 (SEQ ID NO: 114), CATCH6 (SEQ ID NO: 115), CATCH48 (SEQ ID NO: 116), CATCH22 (SEQ ID NO: 117), CATCH19 (SEQ ID NO: 118), CATCH7 (SEQ ID NO: 119), CATCH23 (SEQ ID NO: 120), CATCH45 (SEQ ID NO: 121), CATCH10 (SEQ ID NO: 122), CATCH20 (SEQ ID NO: 123), CATCH42 (SEQ ID NO: 124), CATCH41 (SEQ ID NO: 125), CATCH11 (SEQ ID NO: 126), CATCH12 (SEQ ID NO: 127), CATCH13 (SEQ ID NO: 128), CATCH14 (SEQ ID NO: 129), CATCH39 (SEQ ID NO: 130), CATCH38 (SEQ ID NO: 131), CATCH37 (SEQ ID NO: 132), CATCH36 (SEQ ID NO: 133), CATCH35 (SEQ ID NO: 134), CATCH31 (SEQ ID NO: 135), CATCH25 (SEQ ID NO: 136), CATCH24 (SEQ ID NO: 137), CATCH26 (SEQ ID NO: 138), CATCH30 (SEQ ID NO: 139), CATCH29 (SEQ ID NO: 140), CATCH15 (SEQ ID NO: 141), CATCH16 (SEQ ID NO: 142), CATCH28 (SEQ ID NO: 143), CATCH17 (SEQ ID NO: 144), CATCH18 (SEQ ID NO: 145), CATCH33 (SEQ ID NO: 146), CATCH27 (SEQ ID NO: 147), and CATCH51 (SEQ ID NO: 148) wherein these primers are described in Table 4.

In another aspect, the present invention relates to the use of in vitro markers, as well as of the primers that identify those markers, for the detection of duplications and/or deletions in the 22q11.2 chromosome region, preferably for the in vitro diagnosis of congenital heart diseases, DiGeorge/velocardiofacial syndrome and/or for the in vitro diagnosis of schizophrenia, moderate mental retardation and/or immunodeficiency as previously described.

In another aspect, the invention relates to a kit -from now on, the kit of the invention-, which is comprised of, at least, a pair of primers, which identify the number of alleles that are present in at least one of the markers of the invention, and/or any combination of the markers of the invention, as previously described (Table 4), and, optionally, the reagents for performing a PCR and/or the reagents for isolating DNA from a biological sample.

In another aspect, the invention relates to the use of the kit of the invention for the detection of duplications and/or deletions in the 22q11.2 chromosome region, and/or for the diagnosis of congenital heart diseases, DiGeorge syndrome, velocardiofacial syndrome, schizophrenia, mental retardation, and/or immunodeficiency in an isolated biologal sample from a subject.

### BRIEF DESCRIPTION OF THE DRAWINGS

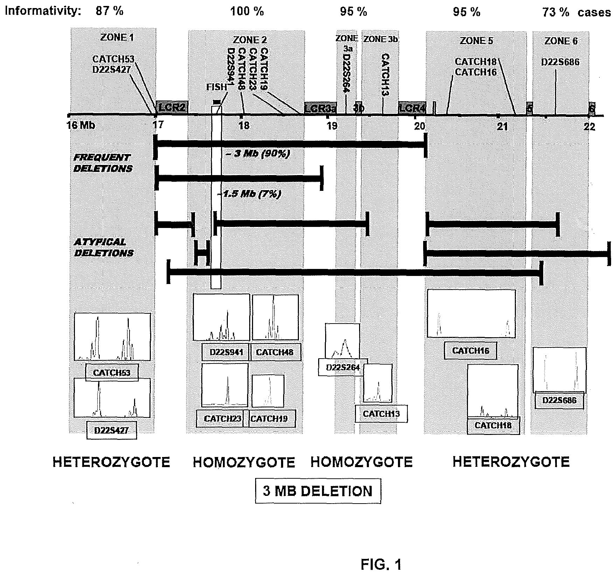

**FIG. 1****.** Graphical representation showing a map for the 22q11.2 chromosome region, where the markers used for the two multiplex PCR described in Example 2 are located. Letters from A to F represent the different LCR22s that delimit the intervals used for detecting typical and atypical deletions and/or duplications in the 22q11.2 chromosome region.
   This figure also shows the regions where the most frequent typical deletions and the most atypical deletions are produced. The top of this figure shows the percentage of patients (n=1448) in whom the presence of deletions (Informativity) in the 22q11.2 region has been ruled out after finishing the studio with the in vitro method of the invention using the multiplex PCR described in Example 2; pointing out the markers used in such multiplex PCR, by means of automatic sequencing, if there is a deletion in the analyzed sample.

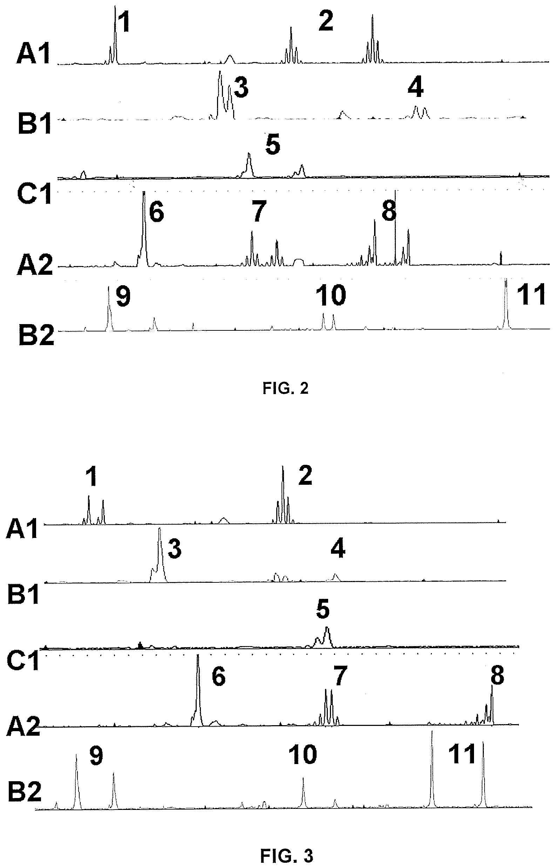

**FIG. 2****.** Capillary electrophoresis results of the two multiplex PCR with the eleven proposed primer pairs in a healthy control subject (see Example 2). The peaks marked with letter A refer to the identification with markers marked with fluorescein fluorochrome. The peaks marked with letter B refer to the identification with markers marked with HEX fluorochrome. The peaks marked with letter C refer to the identification with markers marked with TAMRA fluorochrome. Letters A1, B1 and C1 correspond to the multiplex PCR 1 while A2 and B2 correspond to the multiplex PCR 2. The 11 primer pairs identified the following markers: 1: D22S427; 2: CATCH48; 3: CATCH13; 4: CATCH19; 5: D22S264; 6: CATCH53; 7: CATCH18; 8: D22S941; 9: CATCH16; 10: CATCH23 and 11: D22S686. Primers identifying such markers are described in **Table 1.** The results show that the individual is heterozygous for markers 2: CATCH48; 3: CATCH13; 4: CATCH19; 5: D22S264, 7: CATCH18; 8: D22S941 and 10: CATCH23, thus indicating this individual has no deletions.
**FIG. 3****.** Capillary electrophoresis results of the two multiplex PCR with the eleven proposed primer pairs in a subject with a 3 Mb deletion (see Example 2). The peaks marked with letter A refer to the identification with markers marked with fluorescein fluorochrome. The peaks marked with letter B refer to the identification with markers marked with HEX fluorochrome. The peaks marked with letter C refer to the identification with markers marked with TAMRA fluorochrome. Letters A1, B1 and C1 correspond to the multiplex PCR 1 while A2 and B2 correspond to the multiplex PCR 2. The 11 primer pairs identified the following markers: 1 (D22S427), 2 (CATCH48), 3 (CATCH13), 4 (CATCH19), 5 (D22S264), 6 (CATCH53), 7 (CATCH18), 8 (D22S941), 9 (CATCH16), 10 (CATCH23), and 11 (DS22S686). Primers identifying such markers are described in **Table 1.** The results show that the individual is homozygous for markers 2 (CATCH48), 3 (CATCH13), 4 (CATCH19), 5 (D22S264), 8 (D22S941), and 10 (CATCH23). Therefore, taking into account the high improbability of 3 consecutive markers in an interval flanked by LCR22s being homozygous (p=0.075), we can conclude that the individual has the most common deletion of 3 Mb.

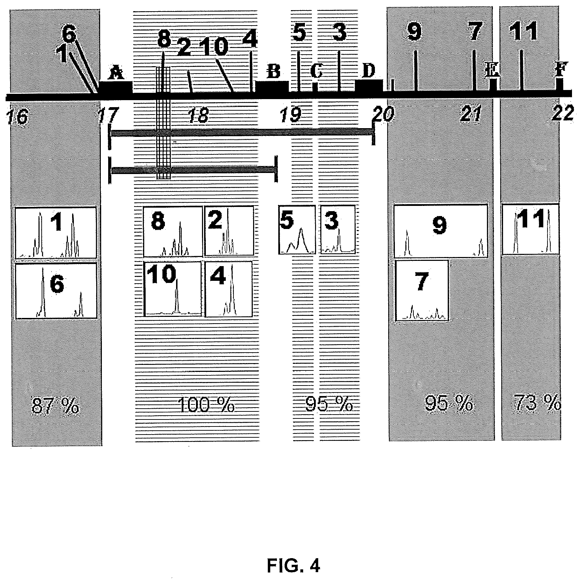

**FIG. 4****.** Capillary electrophoresis results of the two multiplex PCR with the eleven proposed primer pairs in a subject with a 3 Mb deletion, and a region map. From top to bottom, this figure shows: Position in the genetic map of the following markers: 1 (D22S427), 2 (CATCH48), 3 (CATCH13), 4 (CATCH19), 5 (D22S264), 6 (CATCH53), 7 (CATCH18), 8 (D22S941), 9 (CATCH16), 10 (CATCH23), and 11 (D22S686); the map for the region with the LCR22s (A, B, C, D, E, F) that cause the deletions and/or duplications are marked with black squares; the most frequent deletions are shown as grey bars; the position for the FISH TUPLE 1 probe (squared rectangle); the bottom part shows the results of the analysis of the eleven markers The bottom of the figure shows the informativity percentages (% for interval from at least one heterozygous marker in normal individuals). All markers located in the interval between the LCR22s A and D that limits the most frequent 3 Mb deletion are homozygous, thus indicating the presence of a deletion as explained in Figure 3. That deleted interval is shown by means of horizontal bars with grey shadowing.

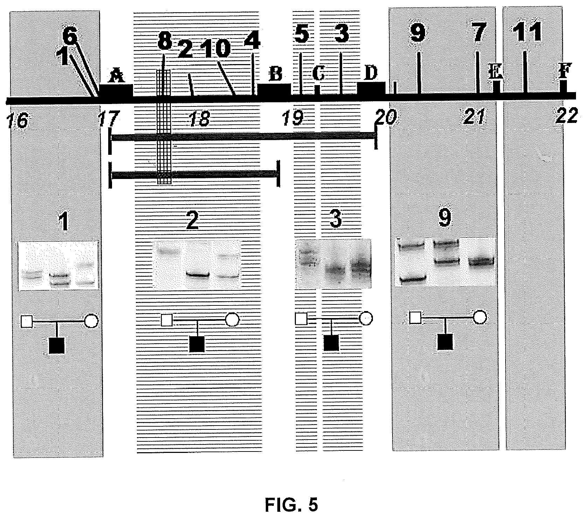

**FIG. 5****.** Visualization of an individual that presents a 3 Mb deletion in parallel with the visualization of their progenitors analyzed by means of electrophoresis. At the top, this figure also shows the map of the 222q11.2 chromosome region. From top to bottom, it shows: Map position of the following markers: 1 (D22S427), 2 (CATCH48), 3 (CATCH13), 4 (CATCH 19), 5 (D22S264), 6 (CATCH53), 7 (CATCH18), 8 (D22S941), (CATCH 16), 10 (CATCH239, and 11 (D22S686); the map of the region containing the LCR22s (A, B, C, D, E, F) that cause the deletions and/or duplications are marked with black squares; the most frequent deletions are shown as grey bars; the position for the FISH TUPLE1 probe (squared rectangle); and the bottom part shows the results of the analysis of some of the proposed microsatellites for the kit marked with silver (D22S427, CATCH48, CATCH13, CATCH16). The bottom part shows the results from and individual and from their parents, which are represented by their genealogy. This is an individual with a 3 Mb deletion. That deleted interval is shown by means of horizontal bars with shadowing. The typing from the deleted individual and from their parents are included. This test is conclusive when carried out in presence of the parents; in this case, the presence of a deletion due to not inheriting the paternal allele. Therefore, this is a de novo deletion with paternal origin.

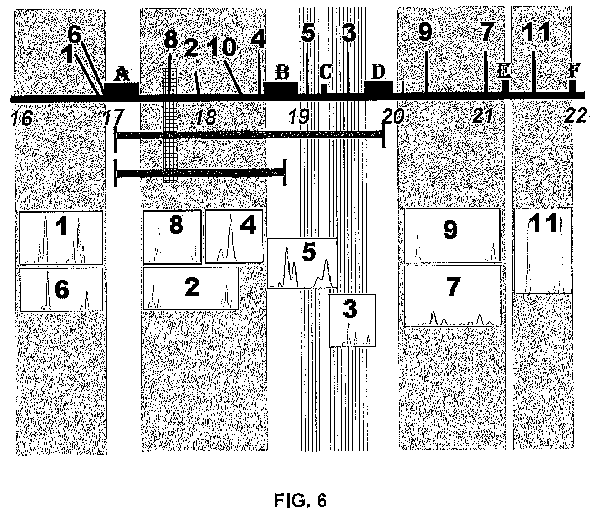

**FIG. 6****.** Capillary electrophoresis results of the two multiplex PCR with the eleven proposed primer pairs in a subject with a 1 Mb duplication. At the top, this figure also shows the map of the 222q11.2 chromosome region. From top to bottom, it shows: Map position of the microsatellite markers: 1 (D22S427), 2 (CATCH48), 3 (CATCH13), 4 (CATCH19), 5 (D22S264), 6 (CATCH53), 7 (CATCH18), 8 (D22S941), 9 (CATCH16), 10 (CATCH23), 11 (D22S686); the map of the region containing the LCR2222s (A, B, C, D, E, F) that cause the deletions and/or duplications are marked with black squares; the most frequent deletions are shown as grey bars; the position for the FISH TUPLE1 probe (squared rectangle); and the bottom part shows the results of the analysis of the eleven microsatellites. The bottom part shows the percentages of informativity (% for the interval of at least one heterozygous marker in normal individuals). Markers CATCH13 (3) and D22S264 (5) have three alleles; this being conclusive evidence of an atypical duplication in this region. Such duplicated interval is shown via the shadowed vertical bars.

### DETAILED DESCRIPTION OF THE INVENTION

The authors of the present invention have developed and validated an in vitro method for the detection of duplications and/or deletions in the 22q11.2 chromosome region, which is based on detecting the number of alleles that are present in a combination of genetic markers located in every interval delimited by low copy repeats regions of the 22q11.2 chromosome region (LCR22). Table 1 shows the limitation of both the LCR22 regions and the inter-LCR regions, obtained from the NCBI37/hg19 version of the human genome sequence, available at UCSC (http://genome.ucsc.edu/), along with the two most common days nowadays (first and second column of Table 1).

For obtaining those markers, the inventors precisely located the exact position of the LCR22 regions in the 22q11.2 chromosome region, as the Example 1 indicates. Afterwards, they identified the STR-type genetic markers in every interval delimited by those LCR22 regions, and more specifically in those inter-LCR intervals. Once the new polymorphic markers were identified in the inter-LCR22 intervals, the inventors designed primer pairs able to amplify such markers so they could finally determine their number of alleles in an isolated biological sample of a subject. This way, the number of alleles is determined by identifying at least one STR-type marker for every identified inter-LCR22 and/or LCR22 region **(Table 1),** thus enabling the detection of any typical or atypical deletion/duplication that may take place in the 22q11.2 chromosome region. This can be useful for the diagnosis of pathologies associated with the presence of deletions and/or deletions in the 22q11.2 region, and more preferably, for the prenatal diagnosis of such pathologies.

As those skilled in the art know, the determination of chromosome regions with deletions/duplications is carried out by studying the number of amplicons produced by the PCR process from a patient's isolated biological sample, and more specifically, in the present invention this isolated biological sample contains DNA.

For the purpose of the present invention, in order to determine if an individual carries a deletion, the STR markers analyzed in the present invention will be analyzed to determine if they are homozygous and/or if that that allele only corresponds to one of the parents with which the isolated biological sample of the individual is compared. If there is no parental DNA available for comparison, the presence of a deletion may be assigned according to the homozygozity of many markers within an interval.

For the purpose of the present invention, in order to determine if the individual carries a duplication, the identification of the presence of at least three alleles is diagnosis of duplication if when compared with the parental DNA two alleles are inherited from one of the parents and the third is inherited from the other parent. The detection of at least three alleles in prenatal samples would indicate maternal contamination, which may need to be ruled out by comparing them with the parental alleles or by using another well-known technique of the prior art for that same objective.

In a preferred embodiment, the diagnosis of a deletion and/or duplication will be carried out, with absolute certainty, on samples previously compared with that of their parents, particularly with that of their mother. However, if a parental biological sample is not available, the deletion and/or duplication will preferably be confirmed by means of another techniques, such as FISH, MLPA, real time PCR, array CGH, or any other technique known by those skilled in the art.

Although in principle the fetal DNA or a patient's is enough to confirm the suspicion of deletions and/or deletions in the 22q11.2 chromosome region with no need of a parental sample, the use of the STR-type markers described in the present invention combined with genetic markers with the same purpose described on prior art may be useful for, especially, avoiding possible mistakes in diagnosis due to potential contaminations in the the analyzed sample; for which the sample should always be a fetal one, along with a maternal sample. Therefore, the diagnosis of pathologies associated with genetic gain and/or loss in the 22q11.2 chromosome region carried out by the in vitro method described in the present invention may be made with presence of parental DNA or just with the propositus' (fetus and its surroundings, such as amniotic fluid). A deletion will be detected 100% certainly, preferably in those cases in which the analysis was made together with the parental DNA, when the propositus' sample has a single allele in a marker, and when this allele corresponds to just one of the parents and there is no other allele inherited from the other parent. A duplication will be detected 100% certainly when there are at least three alleles in a marker, two from one of the parents and the third one from the other parent.

The detection of deletions and/or duplications in the absence of paternal samples is possible by means of two characteristics from the in vitro method of this invention. The first of these characteristics is the use of new STR-type markers, preferably along with their primer pairs, for each interval described for the 22q11.2 chromosome region, wherein those STR-type markers also have a high heterozygosity (0.7-0.8) and, therefore, associated information. It is highly improbable for three consecutive markers in a interval flanked by LCR22s to be homozygous with that not being a deletion. The second of these characteristics is the identification of a deletion and/or duplication in the 22q11.2 being determined by the semiquantitative nature of the technique to be used, being the PCR technique in this case.

Therefore, the in vitro method for detecting duplications and/or deletions in the 22q11.2 chromosome region, from an isolated biological sample, has been applied to samples from different subjects that could be suffering such genetic modifications, with a 100% success rate. This has been carried out primarily in prenatal biological samples. Thus, the in vitro method of the present invention is preferably useful for the detection of duplications and/or deletions in the 22q11.2 chromosome region in prenatal samples. The in vitro method of the invention has the following advantages:
- High speed: results are obtained in 4-5 hours,
- Detection of contaminated maternal sample, which avoids false negatives,
- Information on which parent produced the deletion and/or duplication even in de novo cases, being this the only known method at present that shows such characteristic.
- Inexpensive technology, as only the primer pairs here described are needed in order to identify the presence of STR markers described in the invention, and a PCR technique as well.

On this basis, the inventors have developed a series of inventive aspects that will be described next.

### A METHOD FOR DETECTING DUPLICATIONS AND/OR DELETIONS IN THE

### CHROMOSOME REGION 22q11.2

A first aspect of the present invention relates to an in vitro method to detect duplications and/or deletions in the 22q11.2 chromosome region in an isolated biological sample of a subject -from now on, first in vitro method of the invention-wherein this in vitro method covers the determination, in that subject's isolated biological sample, of the number of alleles that are present in the genetic markers located in at least one of the following regions in chromosome 22:
(i) the previous region to the LCR22A region that covers the polymorphic markers that are selected of the list consisting of: CATCH5 (SEQ ID NO: 111), CATCH53 (SEQ ID NO: 112), CATCH52 (SEQ ID NO: 113), and/or any combination thereof;
(ii) the inter-LCR22A-B region that covers the polymorphic markers that are selected of the list consisting of: CATCH4 (SEQ ID NO: 114), CATCH6 (SEQ ID NO: 115), CATCH48 (SEQ ID NO: 116), CATCH22 (SEQ ID NO: 117), CATCH19 (SEQ ID NO: 118), CATCH7 (SEQ ID NO: 119), CATCH23 (SEQ ID NO: 120) and/or any combination thereof;
(iii) the inter-LCR22B_C region delimited by the LCR22B regions that covers the polymorphic markers that are selected of the list consisting of: CATCH45 (SEQ ID NO: 121), CATCH10 (SEQ ID NO: 122), CATCH20 (SEQ ID NO: 123), and/or any combination thereof;
(iv) the inter-LCR22C-D region that covers the polymorphic markers that are selected of the list consisting of: CATCH42 (SEQ ID NO: 124), CATCH41 (SEQ ID NO: 125), CATCH11 (SEQ ID NO: 126), CATCH12 (SEQ ID NO: 127), CATCH13 (SEQ ID NO: 128), CATCH14 (SEQ ID NO: 129), CATCH39 (SEQ ID NO: 130), CATCH38 (SEQ ID NO: 131), CATCH37 (SEQ ID NO: 132), and/or any combination thereof;
(v) the inter-LCR22D-E region that covers the polymorphic markers that are selected of the list consisting of: CATCH36 (SEQ ID NO: 133), CATCH35 (SEQ ID NO: 134), CATCH31 (SEQ ID NO: 135), CATCH25 (SEQ ID NO: 136), CATCH24 (SEQ ID NO: 137), CATCH26 (SEQ ID NO: 138), CATCH30 (SEQ ID NO: 139), CATCH29 (SEQ ID NO: 140), CATCH15 (SEQ ID NO: 141), CATCH16 (SEQ ID NO: 142), CATCH28 (SEQ ID NO: 143), CATCH17 (SEQ ID NO: 144), CATCH18 (SEQ ID NO: 145), CATCH33 (SEQ ID NO: 146), CATCH27 (SEQ ID NO: 147), CATCH51 (SEQ ID NO: 148), and/or any combination thereof;
wherein a number of alleles fewer than two in at least one of the markers, preferably of every marker, from at least one of the regions (i) to (v), preferably in at least two of the (i) to (v) regions, and more preferably in at least three of the (i) to (v), and more preferably in at least four of the (i) to (v) regions, and even more preferably in all regions (i) to (v), indicates a deletion in the 22q11.2 region; and wherein a number of alleles equal to or superior than three in at least one of the markers, preferably of every marker, from at least one of the regions (i) to (v), preferably in at least two of the (i) to (v) regions, more preferably in at least three of the (i) to (v) regions, and more preferably in at least four of the (i) to (v) regions, and even more preferably in all regions (i) to (v), indicates a duplication in the 222q11.2 region.

Throughout the present invention the term "duplications in the 22q11.2 chromosome region" refers to the rearrangement of genetic material" comprising an increase in the number of copies of a DNA fragment in the 11.2 region of the long arm of chromosome 22.

Throughout the present invention the term "deletions in the 22q11.2 chromosome regions" refers to the rearrangement of genetic material comprising the loss of a DNA fragment in the 11.2 region of the long arm of chromosome 22.

"LCR22" or "low copy repeat" refers throughout the present invention to a region of a eukaryotic genome with a high level of sequence homology (94-99%) with other LCR22s with a range in size from 200-400 kb. LCR22s appear as a consequence of segmental duplication. They are frequently located at the pericentromeric or subtelomeric chromosome region and may contain genes and pseudogenes. Chromosome regions rich in LCR22s tend to yield deletions and/or duplications in such region due to a bad alignment during the non-allelic homologous recombination, which usually results in the loss or duplication of one or many chromosome regions located between two consecutive LCR22s (defined in the present invention as interLCR22 regions). Particularly in the present invention, the deletions and/or duplications under study are those that appear in the interLCR22 regions delimited by the LCR22s that appear in the 222q11.2 region that could mediate a deletion and/or duplication between them and would determine a total of 7 intervals liable to a loss or win with certain frequency (Yamagishi, 2002. Keio J Med, 2: 77-88). These LCR22s in the 22q11.2 chromosome region are commonly referred to as LCR22-A, LCR22-B, LCR22-C, LCR22-D, LCR22-E, and LCR22F, and the location in the 22q11.2 chromosome region is described from the NCBI37/hg19 version of the human genome sequence available at UCSC (http://genome.ucsc.edu/), and they are also shown in **Table1.** Table 1 also shows the inter-LCR22 regions described in the present invention.

Once the position of every LCR22 and inter-LCR22 region in the 222q11.2 chromosome region is known, the in vitro method of the invention comprises the identification, in an isolated biological sample of a subject, of the number of alleles present in each genetic marker located in every inter-LCR region and/or, alternatively, in the LCR22 regions of the 22q11.2 segment as well.

The nucleotide sequences defining every STR markers named CATCH in the present invention, which are located in the different inter-LCR regions previously mentioned, are described in **Table 2.**

**Table 2. STR markers in the 222q11.2 region between the 18,270,416 pb and 26,013,117 pb positions of chromosome 22 that are described in the present invention. (1) Position of the first base of the marker in the nucleotide's number in the human chromosome 22 (NCBI37/hg19).**

| **MARKER** | **SEQ ID NO:** | **POSITION ON THE GENOME⁽¹⁾** | **SIZE (pb)** | **lnter-LCRs22 region** |
|---|---|---|---|---|
| CATCH5 | 111 | 18,450,189 | 116 | Previous to the LCR22A region |
| CATCH53 | 112 | 18,527,297 | 159 | |
| CATCH52 | 113 | 18,572,418 | 125 | |
| CATCH4 | 114 | 19,009,774 | 80 | Inter-LCR22A-B |
| CATCH6 | 115 | 19,050,230 | 159 | |
| CATCH48 | 116 | 19,577,125 | 200 | |
| CATCH22 | 117 | 19,648,997 | 178 | |
| CATCH19 | 118 | 20,309,780 | 194 | |
| CATCH7 | 119 | 19,748,438 | 227 | |
| CATCH23 | 120 | 19,940,450 | 221 | |
| CATCH45 | 121 | 20,775,419 | 167 | Inter LCR22B-C |
| CATCH10 | 122 | 20,791,919 | 129 | |
| CATCH20 | 123 | 20,920,715 | 161 | |
| CATCH42 | 124 | 21,124,842 | 98 | InterLCR22C-D |
| CATCH41 | 125 | 21,125,735 | 179 | |
| CATCH 11 | 126 | 21,144,894 | 194 | |
| CATCH12 | 127 | 21,241,693 | 116 | |
| CATCH13 | 128 | 21,242,550 | 139 | |
| CATCH14 | 129 | 21,270,836 | 213 | |
| CATCH39 | 130 | 21,315,718 | 110 | |
| CATCH38 | 131 | 21,420,596 | 150 | |
| CATCH37 | 132 | 21,435,309 | 232 | |
| CATCH36 | 133 | 21,802,025 | 110 | InterLCR22D-E |
| CATCH35 | 134 | 22,011,833 | 150 | |
| CATCH51 | 148 | 22,094,302 | 158 | |
| CATCH31 | 135 | 22,352,685 | 250 | |

| **MARKER** | **SEQ ID NO:** | **POSITION ON THE GENOME⁽¹⁾** | **SIZE (pb)** | **Inter-LCRs22 region** |
|---|---|---|---|---|
| CATCH25 | 136 | 22,378,875 | 177 | |
| CATCH24 | 137 | 22,378,719 | 150 | |
| CATCH26 | 138 | 22,381,844 | 174 | |
| CATCH30 | 139 | 22,416,063 | 125 | |
| CATCH29 | 140 | 22,494,246 | 140 | |
| CATCH15 | 141 | 22,560,523 | 216 | |
| CATCH16 | 142 | 22,571,539 | 109 | |
| CATCH28 | 143 | 22,605,429 | 185 | |
| CATCH17 | 144 | 22,695,613 | 161 | |
| CATCH18 | 145 | 22,931,665 | 184 | |
| CATCH33 | 146 | 22,186,134 | 209 | |
| CATCH27 | 147 | 22,914,286 | 247 | |

The identification of the number of alleles present in each region described in **Table 1** in the 22q11.2 chromosome region is carried out by detecting, in each of those regions, at least one polymorphic marker, preferably an STR marker, as they are described in **Table 2,** or the specific combinations of every one of those STR markers for each identified LCR22 region.

For the purpose of the present invention, the term "biological sample" relates to any sample to which is possible to isolate nucleic acids, preferably DNA, and it includes, under no limitation, biological fluids or tissues from an individual, obtained by any method that serves the same purpose known by those skilled in the art. The biological sample could be, for example, and under no limitation, a fluid sample, such as blood, plasma, serum, amniotic fluid or tissue. The biological sample in the present invention can be fresh, frozen, fixed or fixed and embedded in paraffin. The following are examples, and under no limitation, of the biological samples from which DNA can be extracted: biological fluids, saliva, urine, sperm, tissues, etc. The in vitro method of the invention can be used for the diagnosis of pathologies in prenatal stage. In this sense, the isolated biological sample is obtained from cells originated from the developing embryo or fetus directly with biopsy, with amniocentesis, or it is obtained from chorionic villi, with no damage to the embryo. Any of the existing procedures on the prior art can be used for extracting the biological sample from an individual, which, so as to simplify its conservation and is management, can be fixed in formalin and be embedded in parafin, or it can be frozen first, followed by embedment in an cryogenic medium, such as OCT compound, by immersion in a high cryogenic medium that allows for a quick freeze.

Once the biological sample from the subject to be analyzed is obtained, this must be processed so as to obtain the DNA sample. The biological sample can be treated in order to physically or mechanically disaggregate the structure from its tissue or cell, releasing the intracellular components in an aqueous or organic solution for the preparation of the DNA. The appropriate DNA fragment for the carrying out of the invention is nuclear DNA or genomic DNA. DNA extraction can be performed with any method known by those skilled in the art (Sambrock et al. , 2001. "Molecular cloning: a Laboratory Manual", 3rd ed., Cold Spring Harbor Laboratory Press, N.Y., Vol. 1-3); this includes but is not limited to: density-gradient centrifugation, two-phase extraction using aqueous phenol or chloroform with ethanol, column chromatography, methods based on the DNA's ability to attach on glass surface and/or on silicates, such as diatomaceous earth or glass beads,use of comercial kits, as the "Q-Biogene fast DNA kit" or the "QIAmp(R) DNA Blood Mini Kit" (Qiagen, Hilden, Alemania), the "G-Spin IIp" (Intron Biotechnology, Corea) or (Fast Prep System Bio 101" (Qbiogene, Madrid, Spain).

Throughout the present invention, the terms "subject" and "individual" are used interchangeably and they refer to every animal classified as a mammal, which includes but is not limited to, farm animals, household animals, primates, and humans, like human beings, non-human primates, cows, hogs, sheep, goats, dogs, cat and rodents. Preferably, the subject is a male or female human being, not matter the age or race. This term also covers nasciturus, preferably embryos or fetus, to which no damage is involved during the obtention of the sample.

Throughout the present invention, "allele" refers to the nucleotides sequence that may present a gene or a genetic marker. The terms "genetic marker" and just "marker" are anologous and may be used interchangeably throughout the present description. There are two alleles per gen or marker (a paternal allele and a maternal allele) in an individual with no genetic abnormalities. An individual is "homozygous" for a genetic marker when both alleles are identical in both homologous chromosomes; it is "heterozygous" when both alleles are different. "Genetic marker" refers to the ADN segment or sequence, coding or not, with a physical location that can be identified in a chromosome. Genetic markers work as a signal for the different regions in a genome. Also, genetic markers can be polymorphic or non-polymorphic.

For the purpose of the present invention, a "microsatellite" is a DNA sequence where a fragment with 2-8 nucleotides in length is repeated in a consecutive way and it is also flanked by unique sequences. The variation in the number of repetitions creates different alleles. Short tandem repeats (STR) or short sequence repeats (SSR) are examples of microsatellites sequences. An "indel" is a common polymorphism form that comprises a small insertion or elimination that is typical for only a few nucleotides in length.

For the purpose of the present invention, a marker is "polymorphic" when there are different variations or alleles of the marker within the population under study. The marker can comprise any allele of any type of variation found in the genome, including nucleotide polymorphisms or SNP (single nucleotide polymorphism) and microsatellites (STR).

For the purposes of the present invention, a "single-nucleotide polymorphism" or "SNP" is a variation of the DNA sequence that occurs when a single nucleotide in a specific location in the genome is different from those of the same species or between paired chromosomes in an individual. Most SNP polymorphisms have two alleles. Each individual is in this case, those homozygous for an allele of polymorphism (that is, both chromosome SNP of the individual have the same nucleotide in the (rs) position, or those heterozygous (that is, the two chromatid of the individual contain different nucleotides). SNPs are commonly determined with an ID tag (rs) with an official reference assigned for each SNP by the National Center for Biotechnology Information (NCBI).

For the purpose of the present invention, a marker is "non-polymorphic" when the marker has no variations within the population under study. Between the non-polymorphic markers we find the sequence tagged sites (STS), which are unique DNA sequences of 200-500 base pairs found in the genome; and also the expressed sequence tags (EST), which are STS derived from cDNA.

In a preferred embodiment of the in vitro method of the invention, the markers that define each inter-LCR22s region, and the LCR22s regions as well, described in the present document, are preferably polymorphic markers, and more preferably, STR markers.

In another preferred embodiment of the in vitro method of the invention, the method is characterized by the determination of the number of alleles present in the following markers:
(i) CATCH53 (SEQ ID NO: 112), located in the previous region to the LCR22A region;
(ii) CATCH48 (SEQ ID NO: 116), CATCH19 (SEQ ID NO: 118), CATCH23 (SEQ ID NO: 120), and/or any combination thereof, located in the inter-LCR22A-B region;
(iii) CATCH45 (SEQ ID NO: 121), CATCH10 (SEQ ID NO: 122), CATCH20 (SEQ ID NO: 123), and/or any combination thereof, located in the inter-LCR22B-C region;
(iv) CATCH13 (SEQ ID NO: 128), located in the inter-LCR22C-D region; and
(v) CATCH16 (SEQ ID NO: 142), CATCH18 (SEQ ID NO: 145), and/or any combination thereof, located in the inter-LCR22D-E region.

In another preferred embodiment, the in vitro method of the invention is characterized by the identification of the number of alleles present in the following markers:
(i) CATCH5 (SEQ ID NO: 111), CATCH53 (SEQ ID NO: 112), and CATCH52 (SEQ ID NO: 113), located in the previous region to the LCR22A region;
(ii) CATCH4 (SEQ ID NO: 114), CATCH6 (SEQ ID NO: 115), CATCH48 (SEQ ID NO: 116), CATCH22 (SEQ ID NO: 117), CATCH19 (SEQ ID NO: 118), CATCH7 (SEQ ID NO: 119), and CATCH23 (SEQ ID NO: 120), located in the inter-LCR22A-B region;
(iii) CATCH45 (SEQ ID NO: 121), CATCH10 (SEQ ID NO: 122), and CATCH20 (SEQ ID NO: 123), located in the inter-LCR22B-C region;
(iv) CATCH42 (SEQ ID NO: 124), CATCH41 (SEQ ID NO: 125), CATCH11 (SEQ ID NO: 126), CATCH12 (SEQ ID NO: 127), CATCH13 (SEQ ID NO: 128), CATCH14 (SEQ ID NO: 129), CATCH39 (SEQ ID NO: 130), CATCH38 (SEQ ID NO: 131), and CATCH37 (SEQ ID NO: 132), located in the inter-LCR22C-D region, and
(v) CATCH36 (SEQ ID NO: 133), CATCH35 (SEQ ID NO: 134), CATCH31 (SEQ ID NO: 135), CATCH25 (SEQ ID NO: 136), CATCH24 (SEQ ID NO: 137), CATCH26 (SEQ ID NO: 138), CATCH30 (SEQ ID NO: 139), CATCH29 (SEQ ID NO: 140), CATCH15 (SEQ ID NO: 141), CATCH16 (SEQ ID NO: 142), CATCH28 (SEQ ID NO: 143), CATCH17 (SEQ ID NO: 144), CATCH18 (SEQ ID NO: 145), CATCH33 (SEQ ID NO: 146), CATCH27 (SEQ ID NO: 147), and CATCH51 (SEQ ID NO: 148), located in the inter-LCR22D-E region,

Additionally, the detection of the described STR markers of the present invention **(Table 2)** can be combined with the detection of other known markers in prior art for every described region for 22q11.2 **(Table 1).**

**Table 3** shows, as a way of a non-limiting illustration, a list of polymorphic STR-type markers known in the prior art, which recognize different regions of the 22q11.2 chromosome region, and that can me used in combination with the polymorphic markers (CATCH) described in the present invention for the determination of deletions and/or duplications in such chromosome region.

**Table 3. STR markers in the 22q11.2 region known in the prior art. (1) Position in the number of nucleotides in the human chromosome 22 (NCBI37/hg19).**

| **MARKER** | **SEQ ID NO:** | **POSITION ON THE GENOME⁽¹⁾** | **SIZE (pb)** | **LCRs22 region** |
|---|---|---|---|---|
| D22S420 | 149 | 17,859,476 | 154 | Previous to LCR22 A region |
| D22S427 | 150 | 18,591,376 | 96 | |
| D22S1638 | 151 | 18,994,949 | 93 | |
| 22K48-2 | 152 | 19,406,393 | 142 | Inter-LCR22A-B |
| D22S1648 | 153 | 19,407,498 | 152 | |
| D22S941 | 154 | 19,409,818 | 224 | |
| D22S944 | 155 | 19,610,551 | 158 | |
| D22S1623 | 156 | 19,642,894 | 140 | |
| D22S264 | 157 | 20,773,416 | 190 | Inter-LCR22B-C |
| D22S311 | 158 | 21,173,575 | 262 | lnter-LCR22C-D |
| D22S1709 | 159 | 21,410,849 | 110 | |
| D22S446 | 160 | 22,019,174 | 153 | Inter-LCR22D-E |
| D22S539 | 161 | 22,257,872 | 124 | |
| D22S308 | 162 | 22,506,170 | 196 | |
| D22S306 | 163 | 22,562,969 | 103 | |
| D22S686 | 164 | 23,068,519 | 180 | Subsequent to LCR22E |
| D22S425 | 165 | 23,082,549 | 218 | |
| D22S257 | 166 | 23,568,429 | 153 | |
| D22S1174 | 167 | 24,488,486 | 214 | |

In a preferred embodiment, the method of the invention comprises the combination of the markers of the invention described in **Table 2** with at least one of the already known markers, described in **Table 3** and/or any of their combinations.

Therefore, in another preferred embodiment, the first in vitro method of the present invention is characterized in that, apart from identifying the number of alleles of the markers of the invention (CATCH) as previously described, it comprises the determination of the number of alleles in at least one of the markers to be selected among:
(i) D22S420 (SEQ ID NO: 149), D22S427 (SEQ ID NO: 150), and/or any combination thereof, located in the previous region to the LCR22A region;
(ii) D22S1638 (SEQ ID NO: 151), 22K48-2 (SEQ ID NO: 152), D22S1648 (SEQ ID NO: 153), D22S941 (SEQ ID NO: 154), D22S944 (SEQ ID NO: 155), D22S1623 (SEQ ID NO: 156), and/or any combination thereof, located in the inter-LCR22A-B region;
(iii) D22S264 (SEQ ID NO: 157), located in the inter-LCR22B-C region;
(iv) D22S311 (SEQ ID NO: 158), D22S1709 (SEQ ID NO: 159), and/or any combination thereof, located in the inter-LCR22C-D region;
(v) D22S446 (SEQ ID NO: 160), D22S539 (SEQ ID NO: 161), D22S308 (SEQ ID NO: 162), D22S306 (SEQ ID NO: 163), and/or any combination thereof, located in the inter-LCR22D-E region; and
(vi) D22S686 (SEQ ID NO: 164), D22S425 (SEQ ID NO: 165), D22S1174 (SEQ ID NO: 167), y D22S257 (SEQ ID NO: 166), and/or any combination thereof, located in the region subsequent to the LCR22E region (positions 22,963,079-22,997,578pb) of chromosome 22;
wherein a number of alleles of less than two in at least one of the markers from at least one of the regions (i) to (vi) indicates a deletion in the 22q11.2 region, and wherein a number of alleles equal to or greater than three in at least one of the markers of at least one of the regions (i) to (vi) indicates a duplication in the 22q11.2 region.

In another more preferred embodiment of the first in vitro method of the invention, the method is characterized by the determination of the number of alleles present in the following markers:
(i) CATCH53 (SEQ ID NO: 112) y D22S427 (SEQ ID NO: 150), located in the previous region to the LCR22A region;
(ii) CATCH48 (SEQ ID NO: 116), CATCH19 (SEQ ID NO: 118), CATCH23 (SEQ ID NO: 120), and D22S941 (SEQ ID NO: 154), located in the inter-LCR22A-B region;
(iii) D22S264 (SEQ ID NO: 157) located in the inter-LCR22B-C region;
(iv) CATCH13 (SEQ ID NO: 128) located in the inter-LCR22C-D region;
(v) CATCH16 (SEQ ID NO: 142), and CATCH18 (SEQ ID NO: 145), located in the inter-LCR22D-E region; and
(vi) D22S686 (SEQ ID NO: 194), located in the region subsequent to theLCR22E region, between the positions 22,963,079-22,997,578 of chromosome 22.

In another more preferred embodiment, the first in vitro method is characterized in that for determining the number of alleles in the 22q11.2 chromosome region as it is described in the present document, a combination of polymorphic STR markers (CATCH) of the invention are used along with polymorphic STR markers already known, such as:
(i) CATCH5 (SEQ ID NO: 111), CATCH53 (SEQ ID NO: 112), CATCH52 (SEQ ID NO: 113), D22S420 (SEQ ID NO: 149), D22S427 (SEQ ID NO: 150) and D22S1638 (SEQ ID NO: 151), located in the previous region to theLCR22A region;
(ii) CATCH4 (SEQ ID NO: 114), CATCH6 (SEQ ID NO: 115), CATCH48 (SEQ ID NO: 116), CATCH22 (SEQ ID NO: 117), CATCH19 (SEQ ID NO: 118), CATCH7 (SEQ ID NO: 119), CATCH23 (SEQ ID NO: 120), 22K48-2 (SEQ ID NO: 152), D22S1648 (SEQ ID NO: 153), D22S941 (SEQ ID NO: 154), D22S944 (SEQ ID NO: 155), D22S1623 (SEQ ID NO: 156), located in the inter-LCR22A-B region;
(iii) CATCH45 (SEQ ID NO: 121), CATCH10 (SEQ ID NO: 122), CATCH20 (SEQ ID NO: 123), D22S264 (SEQ ID NO: 157), located in the inter-LCR22B-C region;
(iv) CATCH42 (SEQ ID NO: 124), CATCH41 (SEQ ID NO: 125), CATCH11 (SEQ ID NO: 126), CATCH12 (SEQ ID NO: 127), CATCH13 (SEQ ID NO: 128), CATCH14 (SEQ ID NO: 129), CATCH39 (SEQ ID NO: 130), CATCH38 (SEQ ID NO: 131), CATCH37 (SEQ ID NO: 132), D22S311 (SEQ ID NO: 158), y D22S1709 (SEQ ID NO: 159), located in the inter-LCR22C-D region;
(v) CATCH36 (SEQ ID NO: 133), CATCH35 (SEQ ID NO: 134), CATCH31 (SEQ ID NO: 135), CATCH25 (SEQ ID NO: 136), CATCH24 (SEQ ID NO: 137), CATCH26 (SEQ ID NO: 138), CATCH30 (SEQ ID NO: 139), CATCH29 (SEQ ID NO: 140), CATCH15 (SEQ ID NO: 141), CATCH16 (SEQ ID NO: 142), CATCH28 (SEQ ID NO: 143), CATCH17 (SEQ ID NO: 144), CATCH18 (SEQ ID NO: 145), CATCH33 (SEQ ID NO: 146), CATCH27 (SEQ ID NO: 147) and CATCH51 (SEQ ID NO: 148), D22S446 (SEQ ID NO: 160), D22S539 (SEQ ID NO: 161), D22S308 (SEQ ID NO: 162), D22S306 (SEQ ID NO: 163), and located in the inter-LCR22D-E region; and
(vi) D22S686 (SEQ ID NO: 164), D22S425 (SEQ ID NO: 165), D22S257 (SEQ ID NO: 166), D22S1174 (SEQ ID NO: 167), located in the region subsequent to the LCR22E region.

Additionally, the inventors have observed that by means of the in vitro method of the present invention, more particularly when the markers of the invention (CATCH) are combined with the markers with high heterozygosity known in the prior art for each inter-LCR and LCR region previously described, the presence of deletions and/or duplications in the 22q11.2 chromosome region is identified with high precision.

Throughout the present invention, "heterozygosity" refers to the concept of population that measures the genetic variability of a specific locus. The heterozigosity of a specific locus results from dividing the number of heterozygous found in a specifc locus by the total of analysed individuals. Markers with a heterozygosity equal to or superior than 0.6 may be considered highly informative, that is that they give reliable information on the presence or abscence of a specific locus. For the purpose of the present invention, it is preferred for markers to have a heterozygosity of at least 60%, more preferably of at least 70%, more preferably of between 70%-80%, more preferably of at least 80%, and even more preferably of at least 90%. The selection of the markers with such heterozygosity levels for the method of the invention allows for the detection of deletions and/or duplications in the 22q11.2 chromosome region when a parental sample is missing. This technical advantage of the in vitro method of the invention is so because it would be practically impossible to detect three consecutive markers in an interval delimited by LCR22s with them being homozygous/heterozygous but with that not being a deletion and/or duplication.

Therefore, in another preferred embodiment of the in vitro method of the invention, the polymorphic markers of the invention with a higher heterozygosity are: CATCH53, CATCH18, CATCH13, CATCH23, CATCH48, CATCH16, and CATCH19. In another preferred embodiment, the polymorphic markers in the prior art that have high heterozygosity are: D22S941, D22S686, D22S427, and D22S264. In another more preferred embodiment, the set of polymorphic markers described in the present invention with high heterozygosity are: CATCH53, CATCH18 CATCH13, CATCH23, CATCH48, CATCH16, CATCH19, D22S941, D22S686, D22S427, and D22S264 **(Table 5).**

In general, every STR marker used in the present document, both the new markers named CATCH in the present document as the other markers already known in prior art, are amplified by means of the polymerase chain reaction (PCR), a standard technique in molecular biology laboratories. The primers for the PCR amplification can be easily synthesized through standard techniques, such as through solid-phase synthesis of phosphoramidite chemistry.

Another aspect of the present invention relates to the primers complementary to the sequences of the polymorphic markers, named CATCH in the present invention **(Table 2)** used to amplify those regions.

**Table 4** shows the set of primers designed for the amplification of every polymorphic CATCH marker described in the present invention.

**TABLE 4. Markers from the 22q11.2 region described in the present invention along with the sequences of the primers used for their identification. Direct primers are shown in bold. The term "repetition" refers to the STR nucleotide sequence amplified in each marker.**

| **MARKER** | **SEQUENCING PRIMER (5'→3')** | **SEQ ID NO** | **REPEAT** |
|---|---|---|---|
| CATCH5 | **TATGGATTCACAGTCTTGGCCAAC** | 3 | (CA)n |
| | TGTCAGTGGGATCCCCATGC | 4 | |
| CATCH53 | **ACACCCGTGGCATGGGGCATTC** | 5 | (GT)n |
| | CGTACTTTCTATCAAACCCGTGG | 6 | |
| CATCH52 | **TGGTACATTTTCGGGGGTTG** | 7 | (TTTG)n |
| | TGAGCTGAGATCACGCCACCCTG | 8 | |
| CATCH4 | **AAAAAATTCAGCAAGGGGTG** | 13 | (GT)n |
| | AGTATTCTGTCTATGGCTTGC | 14 | |
| CATCH6 | **CGTCTTGGTATTTCCAAGCAGC** | 15 | (CT)n |
| | GGAGGACAAGCTTGCCAGGAC | 16 | |
| CATCH48 | **GATCCTTACTGAGAGGAATGTTGTG** | 23 | (TA)n |
| | AGGGTGATGAAAAGGTTCTGG | 24 | |
| CATCH22 | **AGTAGGCAGGGGCCATAAGG** | 29 | (CTTC)n |
| | CACTGCACTCCAGCTTGGGTG | 30 | |
| CATCH7 | **CAGCAGCCTGAGCAGCCTGG** | 31 | (CCG)n |
| | CCCCGGGGCCCTCGGGCTCG | 32 | |
| CATCH23 | **TGTGACAGAGTGACAGCCCGTCTC** | 33 | (AAAT)n |
| | AGGCCCACCGTGGCAGCAGC | 34 | |
| CATCH 19 | **GCACGTTCTGCACATGTACCCC** | 35 | (TA)n(CA)n |
| | TGGCCCAGGAGTCCCCATGC | 36 | |
| CATCH45 | **TGTTTCTCTGTGTCTGGTGGAAG** | 39 | (GT)n |
| | ATGTAGACACAGATATGCACCCTGC | 40 | |
| CATCH10 | **CACGGTGTCCGGCAGCATCC** | 41 | (CAG)n |
| | CGCCTCATGGAGGGCGCAGG | 42 | |
| CATCH20 | **AGATGAGTGATAACCGAGTGC** | 43 | (CAG)n |
| | TGAATTGGTGGCTGGGCCTG | 44 | |
| CATCH42 | **TGCTATGTTGCCCAAGCTGG** | 45 | (CA)n |
| | ACTGTGTGTGTCTCTGTGTCCAGC | 46 | |
| CATCH41 | **CTGTTACCACTAAATGGGAAAATG** | 47 | (TTTA)n |
| | TGGGTGACAGAGTGAGACTCTG | 48 | |
| CATCH11 | **AGCCAAAATCACGCCACTGC** | 49 | (CA)n |
| | CTTTATGGTTACCCCTTGGACC | 50 | |
| CATCH12 | **AAAGGCAGAAAGCAGCCCTG** | 53 | (CA)n |
| | GGGTGAAGAGTAAAGCCTTTTCC | 54 | |
| CATCH13 | **CGTTTGGTGGAGGAGGGTCC** | 55 | (CA)n |
| | T AACACTGA TCCTTTCTGGGAGG | 56 | |
| CATCH 14 | **GAAGGAAAGGAAAGGGAAAGG** | 57 | (AGAA)n |
| | GGCCCCGAGGCTTCCCCTGC | 58 | |
| CATCH39 | **CACTGCACTCCAGCCTGGGAG** | 59 | (TAAA)n |
| | CAGGCTCTGGGATAGCCAGTC | 60 | |
| CATCH38 | **GTACCTGTAGAATTACCAGTGCTGG** | 63 | (ATTT) n |
| | CTCCAGCCTGGGCAACAGAGC | 64 | |
| CATCH37 | **TGGCCGCTGTGGGCTGCATG** | 65 | (CA)n |
| | GTGCATGCCATCATGCCCAG | 66 | |
| CATCH36 | **AATGCCGGGGCCGTCGGTAC** | 67 | (GT)n |
| | AACATCTGCCCATGCATACGG | 68 | |
| CATCH35 | **CTGTACTCCAGCCTGGGCAAC** | 69 | (TAA)n |
| | GTAAATGATGTTCCACCCGTTTG | 70 | |
| CATCH51 | **ACTTTCGGAGGCTGAGGCAGGTGG** | 73 | (GT)n |
| | CCTGGGACTACAGGCGTGTGG | 74 | |
| CATCH33 | **CCTGGTCCCAGATACAGTGC** | 75 | (GT)n |
| | GGGTAATGGGTGGAGATTGATG | 76 | |
| CATCH31 | **GAACACACAGGCCCGCTGTTCC** | 79 | (GT)n(GA)n |
| | CAACAAAAATACCCATGATACAGCTC | 80 | |
| CATCH25 | **TTGTCAAGGTCACAGGCATC** | 81 | (GT)n |
| | GTGGCATTGTATGCTGAGATAG | 82 | |
| CATCH24 | **TTTACCCAGGGTTGGGATAGGTGG** | 83 | (GT)n |
| | TTCTCCATGGTAACGCTAAAACC | 84 | |
| CATCH26 | **GCATATCAAAAGCTAAGTTATAGC** | 85 | (CA)n(TA)n |
| | GTTCCTTCTTTTGGTTGAAG | 86 | |
| CATCH30 | **GTCTGCTGACCTGCATACTC** | 87 | (GT)n |
| | CCAAGGGAACTGGAAGCAGG | 88 | |
| CATCH29 | **TTAGATACTCTGATGAGCCAGC** | 89 | (GT)n |
| | CCCTCAAATCTTTCAGAATCAG | 90 | |
| CATCH15 | **TAGGCAGGAATGGGTCCTG** | 93 | (CT)n(CA)n |
| | TTCAACCTGAGGGTGGCTGG | 94 | |
| CATCH16 | **GCAGTAAGATGATGACTCCGGACC** | 97 | (TG)n(CG)n |
| | ATATGGTAACACACATGCGTGTGC | 98 | |
| CATCH28 | **ACCTGCACCATGCTTCCTGC** | 99 | (TA)n |
| | GTAATATAGCCTAAACATAAAGTAC | 100 | |
| CATCH 17 | **CCTTACTTGTATCCTAGGCCAGC** | 101 | (TC)n(TG)n |
| | TGATGCTGCAGGGCTTGGAC | 102 | |
| CATCH27 | **TGTTCCAGCCTGGGCGACAG** | 103 | (GAAA)n |
| | CCTGGTATTGCCTTTCCAG | 104 | |
| CATCH 18 | **GAAGATAGCCCACCTTGCTGG** | 105 | (TG)n |
| | CCTGAGATATTAGGGCAAACAGG | 106 | |
| D22S420 | **TGTTCTACACTGAAAATTCTGACGG** | 1 | (GT)n |
| | GAGGGCGTTATCCATGACC | 2 | |
| D22S427 | **TGCTGTTTTGTAGAGTGTTTAGAC** | 9 | (CA)n |
| | AAAT ACGGCTGGGCAC | 10 | |
| D22S1638 | **GACAACAGCAAATTGCACATT** | 11 | (GT)n |
| | TCACGCCACTACCCTCCAG | 12 | |
| 22K48-2 | **CGGTGTGAATCTATAATCATCTCG** | 17 | (CA)n |
| | ACCTCTGGTGTTTGGATATTTACC | 18 | |
| D22S1648 | **AGTTGTCAGATGCCTAAGAGA** | 19 | (GT)n |
| | CAGATGCTTCAGGAGAAGTG | 20 | |
| D22S941 | **CAGGTTACAAAGTACATTAACTT** | 21 | (CA)n |
| | ACCAGCTCCAACCATTTCTTG | 22 | |
| D22S944 | **CATGTGAAAGATGCTACTTCC** | 25 | (GT)n |
| | ATGGGGAGGAGCATGGGAT | 26 | |
| D22S1623 | **CACAACTCCTGGGCTCAAGCT** | 27 | (CA)n |
| | ACGTAAATCTCATACCATGTAAA | 28 | |
| D22S264 | **ATTAACTCATAAAGGAGCCC** | 37 | (CA)n |
| | GGAATACCTCTGGTGGGGTG | 38 | |
| D22S311 | **TTTTTGTATTTTTAGTAGAGACGG** | 51 | (CA)n |
| | GCTAGTGTGAGATAACGAAGCC | 52 | |
| D22S1709 | **CACTTCAGCAAGAACAGCAGA** | 61 | (CA)n |
| | CTCTTCCAAGTTCAGTGCTCT | 62 | |
| D22S446bis | **CCAAGGCAGGCGAATCAGG** | 71 | (CA)n |
| | TGTCTGGATGGGCGTGGTGG | 72 | |
| D22S539 | **CATTATGGCTGTAGGCTGTA** - | 77 | (CA)n |
| | CATACCAATGCAATATGAA | 78 | |
| D22S308 | **TCCTGCAACAGCACTAGACC** | 91 | (GT)n |
| | GCTAAAGGAAAAGGAGGCATC | 92 | |
| D22S306 | **CTCTTTCGCTGGAACATCAAA** | 95 | (CA)n |
| | GGTCCAGACTGTATAAATGGC | 96 | |
| D22S686 | **TTGATTACAGAGTGGCTCTGG** | 107 | (GGAA)n |
| | TAAGCCCTGTTAGCACCACT | 108 | |
| D22S1174 | **GAATCACTAGGGGCCTTCA** | 109 | (TG)n |
| | TGAGGCTATGTGGCCCAG | 110 | |

The determination of the number of alleles of the genetic markers present in each of the indicated regions can be carried out by any technique previously described in prior art. For example, fluorescence based techniques, PCR, nested PCR, and other techniques used for nucleic acid amplification. The specific methodologies available for SNP genotyping include, but not limited to, TaqMan genotyping assays and SNPlex systems (Applied Biosystems), mass spectrometry (like, the MassARRAY system from Sequenom), minisequencing methods, real-time PCR, the Bio-Plex system (BioRad), CEQ and SNPstrem system (Beckman), technologies for the molecular inversion probes (such as Affymetrix GeneChip), and BeadArray technologies (such as the Illumina GoldenGate and Infinium assays).

The detection of the number of alleles can also be carried out by hybridisation methods, including, but not limited to, Southern hybridisation analysis, Northern hybridisation analysis, and/or in situ hybridisations (cf. Current Protocols in Molecular Biology, Ausubel, F. y otros, eds., John Wiley & Sons, including every supplement).

The presence of more than one allele-specific marker can be indicated by the use of many sequence-specific nucleic acid probes, and being each one specific for a particular allele.

A sequence-specific probe can be directed to hybridize genomic DNA, RNA, or cDNA. An "acid nucleic probe", as it is referred to in the present invention, can be a DNA probe or a RNA probe hybridizing with a complementary sequency. Those skilled in the art will know how to design a probe in such a way that the sequence-specific hybridization will only take place if the particular allele is present in a genomic sequence from an individual's biological sample. The nucleic acid probe can be of any appropriate size, provided that it is enough for the hybridization of the appropriate RNA or genomic DNA to take place specifically under severe conditions. The probe can be of 5-100 nucleotides, preferably, of 10-50 nucleotides in length, and more preferably, of 12-30 nucleotides in length. The probe can contain a segment from the 222q11.2 genomic region comprising, at least, one allele of a marker present in such genomic region, or the complentary sequence to that segment. As those skilled in the art know, the probe can comprise a fluorescent fraction or a group in its 3' end and a extinguisher in its 5' end. Also, the probe may include modified bases, such as modified adenine or modified guanine, which may be useful for adjusting the melting temperature in regions with a low percentage of G and C bases (like a modified adenine for the formation of three hydrogen bonds to its complementary thymine). Alternatively, a peptide nucleic acid (PNA) probe can be used along with, or in substitution of, the nucleic acid probe previously described. A PNA is an emulated DNA with a peptide-type inorganic structure, like N-(2-aminoethyl)glycine units, with an organic base (A, G, C, T or U) bonded with glycine nitrogen by a bonding metalcarbonyl.

With these and other available methods for those skilled in the art, the number of alleles in polymorphic and/or non-polymorphic markers can be identified.

Nevertheless, in a particular embodiment of the method of the invention, the determination of the number of alleles of the markers is carried out by at least one polymerase chain reaction amplification or PCR, preferably, by at least one multiplex PCR reaction, and more preferably, two multiplex PCR reactions.

It is by means of PCR, which stands for polymerase chain reaction, that we can obtain millions of copies of the DNA regions we look for. PCR is characterized by the use of primer pairs that delimit the region from where the million copies will be made, which is also known as "amplifying DNA" during the PCR. It has a determinate number of cycles, which further comprise three different phases in which the DNA strands are separated, then the primers are bounded, and new DNA strands are elongated. In every cycle, if the reaction efficiency is 100% the DNA fragments under amplification result exponentially amplified. In the present invention, "multiplex amplification reaction" refers to the PCR reaction in which more than one marker in the same reaction are amplified, by means of two or more primer pairs in a single tube containing the other reagents of the reaction with the purpose of simultaneously amplify multiple DNA sequences or markers.

Therefore, after obtaining the DNA from the biological sample, it goes through at least one multiplex amplification reaction; although, as those skilled in the art know, it can also be carried out by 2, 3, or more multiplex PCR on the DNA sample.

In the present invention, the "multiplex PCR" refers to a PCR reaction in which more than one sequence in the same reaction are amplified. To carry it out, two or more primer pairs specific for the segments to be amplified must be combined in a single tube with the other reagents of the reaction in enough quantity. This way, we obtain information from many loci in a single reaction; fewer moulds for the analysis, fewer reagents, and a quick database construction. For the purpose of distinguishing the products from each amplification reaction occuring in a multiplex PCR, it is possible to mark each reaction with different fluorochrome so the amplification products can be detected in an adequate wavelength. The fluorochromes appropriate for the present invention include, but are not limited to, fluorescein, hexachloro-fluorescein (HEX), N,N,N';N'-tetramethyl-6-carboxyrhodamine (TAMRA), 5-carboxyfluorescein (5-FAM), 2',7'-dimethoxy-4',5'-dichloro-6-carboxyrhofluorescein (JOE), 6-carboxy-x-rhodamine (ROX), CY3, NED, VIC, PET, tetrachlorofluorescein (TET), and similars.

Thus, in another particular embodiment of the in vitro method of the invention, the number of alleles of the markers described in the present invention is determined by multiplex PCR, more preferably by at least two multiplex PCR reactions.

In a more particular embodiment of the first in vitro method of the invention, the first multiplex PCR is performed to determine the number of alleles present in the following markers: CATCH53, CATCH18, D22S941, CATCH13, CATCH23, and D22S686, by amplifying them using the primers described in the present document for that purpose. The second multiplex PCR is performed to determine the number of alleles present in the following markers: D22S427, CATCH48, CATCH16, CATCH19, and D22S264, by the set of primers specifically described in the present invention for that purpose. For the purpose of the present invention, it is possible to use other primers able to amplify the described markers. Those skilled in the art will be able to obtain alternatives primers to those described in the present invention and also useful for the amplification of the described markers by using other known methods.

In another particular embodiment of the in vitro method of the invention, the primers used for determining the presence of the markers previously mentioned are described in **Table 4.**

In the present invention, "primer", "oligonucleotide" or "olige" refer to the nucleotide sequence from which the DNA polymerase starts to synthesize the new DNA molecule. Primers are short nucleotide sequences, of about 15-24 nucleotides in length, that can be aligned with a target DNA strand thanks to the bases being complementary for the hybrid formation between a primer and the target DNA strand. Afterwards, the enzyme DNA polymerase can extend the primer throughout the target DNA strand. The methods for the preparation and use of the primers are described, for example, in Sambrook et al. (2001) and Ausubel et al. (1999).

In the context of the present invention, the term "primer pairs" refers to the set of two primers that, used in the same amplification reaction or PCR, allow for the obtention of multiple copies of a target DNA sequence. Each one of the primers hybridizes with the target sequence thus amplifying the nucleotides sequence which is delimited by each primer pair. The extension of the primers during the PCR cycles determines the exponential multiplication 2^{N} of the nucleotides sequence delimited by the primers, in which N is the number of cycles of the PCR reaction.

The amplification of the different markers described in the present invention requires specific reaction conditions and procedures for each one of the primer pairs used, which can be obtained by systematic variation of each parameter. The number of cycles and the annealing temperature used for the PCR reaction must be adequate in order to obtain accurate results.

### USE OF THE IN VITRO METHOD FOR DETECTING DUPLICATIONS AND/OR DELETIONS IN THE CHROMOSOME REGION 22q11.2

As explained on the prior art of the invention, one of the most known genetic causes of congenital heart diseases, DiGeorge syndrome, and velocardiofacial syndrome are deletions and/or duplications in the 222q11.2 region. For this reason, it would be useful to figure out the presence of a deletion and/or duplication in that region so as to diagnose if an individual will suffer or not a congenital heart disease or the DiGeorge syndrome, which can be carried out by means of the in vitro method of the invention. On the other hand, the del22q11.2 syndrome is not only the known genetic cause most frequent of congenital heart diseases (a minimum of 2% for all of them), but it can also cause schizophrenia (1%), as having a 22q11.2 increases 30 times the risk of suffering such disorder; and it is also responsible for moderate mental retardation and immunodeficiency.

Therefore, in another aspect, the present invention is related to the in vitro method for the diagnosis and/or prognosis of congenital heart diseases, DiGeorge syndrome, or velocardiofacial syndrome in an isolated biological sample of a subject, by using the in vitro method previously described in the present document, where the detection of a del/dup22q11.2, as mentioned in the in vitro method of the invention previously described, indicates that the subject has a high probability of suffering a congenital heart disease, DiGeorge syndrome, or VCF syndrome.

In another aspect, the present invention refers to the in vitro method for the diagnosis of schizophrenia, mental retardation and/or immunodeficiency in an isolated biological sample of a subject, by using the in vitro method previously described in the present document, where the detection of a del/dup22q11.2, as mentioned in the in vitro method of the invention previously described, indicates that the subject has a high probability of suffering these pathologies, which are all related with a del/dup22q11.2.

The terms "duplication", "deletion", "individual", and "biological sample" have been previously described and they are to be applied equally throughout the present aspect of the invention.

"Diagnosis" refers to the procedure in which a disease, nosological entity, pathology, or syndrome is identified from certain indications, parameters, or syntoms. In the context of the present invention, the disease to be identified is a set of diseases classified in "congenital heart diseases", in which the parameter used is the presence or absence of del/dup22q11.2.

In the present invention, "congenital heart diseases" refer to the set of diseases or pathologies characterized in that the structure and functioning of the heart is abnormal compared to a healthy heart due to an abnormality in its development prior to birth. Generally, these are anatomycal injuries in one or more of the four chambers, in the dividing septums, or in the outflow tracts or valves (ventricular space from where the blood leaves the heart) with a congenital origin (present from birth). There are multiple congenital heart diseases; some with a mild nature and evolution and/or mild treatment with a good prognosis, and others are more severe and with a reserved prognosis. It is frequent for congenital heart injuries to combine between them so a patient may suffer multiple congenital heart injuries.

In a particular embodiment, the congenital heart diseases is to be selected from the following group: Fallot tetralogy, transposition of the great arteries, tricuspid atresia, total anomalous pulmonary venous return, truncus arteriosus, hypoplastic left heart syndrome, pulmonary atresia, some forms of total anomalous pulmonary venous return, Ebstein anomaly, ventricular septal defect, atrial septal defect, patent ductus arteriosus, aortic stenosis, pulmonary stenosis, coarctation of the aorta, atrioventricular canal (endocardial cushion defect), congenital heart malformation in chambers and canals (transposition of the great vessels, persistent truncus arteriosus), malformations in heart septum (interauricural communication, interventricural communication, malformations in the pulmonary and tricuspid valves (pulmonary atresia, Ebstein anomaly), malformations in the aortic and mitral valves, dextrocardia, congenital malformations of the great arteries (coarctation of the aorta, patent ductus arteriosus), ventricular septal defect, total transposition of the great vessels, aortic valve stenosis or aortic stenosis, pulmonary atresia, double outlet right ventricle, interrupted aortic arch (types A, B, or C), patent ductus arteriosus, hypoplastic left heart syndrome, persistent truncus arteriosus or truncus arteriosus, single ventricle, among others.

On the other hand, as previously explained, the in vitro method of the invention is carried out from an isolated biological sample of an individual that, in the context of the present invention, refers to both nasciturus (embryos and fetus) and already born individuals in any development stage. Therefore, if the isolated biological sample comes from the nasciturus, the in vitro method of the invention can be used for the prenatal diagnosis of congenital heart diseases, DiGeorge syndrome, VCF syndrome, schizophrenia, mental retardation, and/or immunodeficiency.

So, in another aspect, the invention relates to a in vitro method for the prenatal diagnosis of pathologies associated with deletions and/or duplications in the 22q11.2 chromosome region, in an isolated biological sample containing fetal cells, wherein the detection of dup/del22q11.2 in that isolated biological sample, wherein the detection of a duplication and/or deletion in the 22q11.2 chromosome region, indicates that the subject presents a pathology that is selected of the list consisting of: congenital heart disease, DiGeorge syndrome, velocardiofacial syndrome, schizophrenia, mental retardation, and immunodeficiency.

In a more particular embodiment, the isolated biological sample containing fetal cells is selected from the following group: blood, chorial biopsy or amniotic fluid.

In order to determine if the individual carries or not a dup/del22q11.1, the diagnosis strategy carried out over the obtained results through the method of the invention in an isolated biological sample of an individual must be in accordance with the following instructions:
- To begin with, a biological sample from a subject must be obtained in accordance with its description in the present invention.
- Then, the DNA is extracted from that biological sample.
- Over the extracted DNA, the 11 markers described in **Table 6** must be analysed or typed by the two multiplex PCR techniques previously described, and then proceed with a capillary electrophoresis by means of an automated sequencer.
- The number of alleles present in each of the markers that underwent the multiple PCRs is analysed so as to reach a diagnosis for the analysed individual.

If the obtained results express that the 6 intervals inter-LCR22 (previous to LCR22A, interLCR22A-B, inter-LCR22B-C, inter-LCR22CD, inter-LCR22D-E, subsequent to LCR22E) that were analyzed with the method of the invention have at least one heterozygous markers of two alleles, then in that analyzed sample there are no deletions nor duplications in those intervals, so the subject has no deletion and/or duplication in the 22q11.2 chromosome region.

If, on the contrary, the obtained results express that at least one of the analyzed marker presents more than two alleles, then the isolated biological sample presents a duplication in the LCR22 interval where the marker or markers with more than two alleles are located, and the subject presents a duplication in that LCR22 interval in the 22q11.2 chromosome region. Optionally, in order to remove any doubts regarding the presence of a duplication in the region where more than two alleles for at least one of the analyzed markers was detected, any other technique known in prior art can be carried out so as to corroborate those results. Among the techniques that can be used for corroborating the results obtained via the method of the invention, we find MLPA, FISH, CGH-array, etc. Alternatively, in order to confirm the presence of a duplication in the analyzed region, the segregation results obtained after the analysis of the parental sample related to the subject in question can also be used.

On the other hand, if the obtained results express that in one of the interLCR22 intervals all markers are homozygous (a single allele), then the isolated biological sample indicates the presence of a deletion in the LCR22 interval where the marker or markers with a single allele are located, so the subject will have a high probability of having a deletion in the LCR22 interval in the 22q11.2 chromosome region. In order to corroborate the presence of a deletion in the region detected via the in vitro method of the invention, this deleted interval will be analyzed with additional markers that will recognize or map the specific chromosome region, and, also, the obtained results will be compared with the segregation results that were obtained after analyzing the parental sample related to the subject in question. If the obtained results with those additional markers along with the parental data express that none of them is heterozygous for that region, then there would be no doubt regarding the subject having a deletion in the interLCR22 interval where none of the markers are heterozygous with two alleles. This deletion can also be corroborated by using any other technique known in prior art, such as MLPA, FISH, CGH-array, etc.

### PRIMERS OF THE INVENTION

In order to carry out the in vitro method of the invention, as well as the in vitro methods for diagnosis previously described, the inventors not only needed to determinate the limits of the LCR22 regions of the 22q11.2 chromosome region, but also they needed to identify new genetic markers between the identified LCR22 regions (inter-LCR22 regions) and also design primer pairs specific for the genetic markers identified in such inter-LCR22 regions.

Therefore, in another aspect, the invention is related to the primers described in **Table 4.** In a particular embodiment, the invention relates to at least a primer pair selected from the group consisting in the oligonucleotide pairs SEQ ID NO: 3 and SEQ ID NO: 4, able to amplify the marker CATCH5; SEQ ID NO: 5 and SEQ ID NO: 6 able to amplify the marker CATCH53; SEQ ID NO: 7 and SEQ ID NO: 8 able to amplify the marker CATCH52; SEQ ID NO: 13 and SEQ ID NO: 14 cable to amplify the marker CATCH4; SEQ ID NO: 15 and SEQ ID NO: 16 able to amplify the marker CATCH6; SEQ ID NO: 23 and SEQ ID NO: 24 able to amplify the marker CATCH48; SEQ ID NO: 29 and SEQ ID NO: 30 able to amplify the marker CATCH22; SEQ ID NO: 31 and SEQ ID NO: 32 able to amplify the marker CATCH7; SEQ ID NO: 33 and SEQ ID NO: 34 able to amplify the marker CATCH23; SEQ ID NO: 35 and SEQ ID NO: 36 able to amplify the marker CATCH19; SEQ ID NO: 39 and SEQ ID NO: 40 able to amplify the marker CATCH45; SEQ ID NO: 41 and SEQ ID NO: 42 able to amplify the marker CATCH10; SEQ ID NO: 43 and SEQ ID NO: 44 able to amplify the marker CATCH20; SEQ ID NO: 45 and SEQ ID NO: 46 able to amplify the marker CATCH42; SEQ ID NO: 47 and SEQ ID NO: 48 able to amplify the marker CATCH41; SEQ ID NO: 49 and SEQ ID NO: 50 able to amplify the marker CATCH11; SEQ ID NO: 53 and SEQ ID NO: 54 able to amplify the marker CATCH12; SEQ ID NO: 55 and SEQ ID NO: 56 able to amplify the marker CATCH13; SEQ ID NO: 57 and SEQ ID NO: 58 able to amplify the marker CATCH14; SEQ ID NO: 59 and SEQ ID NO: 60 able to amplify the marker CATCH39; SEQ ID NO: 63 and SEQ ID NO: 64 able to amplify the marker CATCH38; SEQ ID NO: 65 and SEQ ID NO: 66 able to amplify the marker CATCH37; SEQ ID NO: 67 and SEQ ID NO: 68 able to amplify the marker CATCH36; SEQ ID NO: 69 and SEQ ID NO: 70 able to amplify the marker CATCH35; SEQ ID NO: 73 and SEQ ID NO: 74 able to amplify the marker CATCH51; SEQ ID NO: 75 and SEQ ID NO: 76 able to amplify the marker CATCH33; SEQ ID NO:79 and SEQ ID NO: 80 able to amplify the marker CATCH31; SEQ ID NO: 81 and SEQ ID NO: 82 able to amplify the marker CATCH25; SEQ ID NO: 83 and SEQ ID NO: 84 able to amplify the marker CATCH24; SEQ ID NO: 85 and SEQ ID NO: 86 able to amplify the marker CATCH26; SEQ ID NO: 87 and SEQ ID NO: 88 able to amplify the marker CATCH30; SEQ ID NO: 89 and SEQ ID NO: 90 able to amplify the marker CATCH29; SEQ ID NO: 93 and SEQ ID NO: 94 able to amplify the marker CATCH15; SEQ ID NO: 97 and SEQ ID NO: 98 able to amplify the marker CATCH16; SEQ ID NO: 99 and SEQ ID NO: 100 able to amplify the marker CATCH28; SEQ ID NO: 101 and SEQ ID NO: 102 able to amplify the marker CATCH17; SEQ ID NO: 103 and SEQ ID NO: 104 able to amplify the marker CATCH27; SEQ ID NO: 105 and SEQ ID NO: 106 able to amplify the marker CATCH18; SEQ ID NO: 1 and SEQ ID NO: 2 able to amplify the marker D22S420; SEQ ID NO: 9 and SEQ ID NO: 10 able to amplify the marker D22S427; SEQ ID NO: 11 and SEQ ID NO: 12 able to amplify the marker D22S1638; SEQ ID NO: 17 and SEQ ID NO: 18 able to amplify the marker 22K48-2; SEQ ID NO: 19 and SEQ ID NO: 20 able to amplify the marker D22S1648; SEQ ID NO: 21 and SEQ ID NO: 22 able to amplify the marker D22S941; SEQ ID NO: 25 and SEQ ID NO: 26 able to amplify the marker D22S944; SEQ ID NO: 27 and SEQ ID NO: 28 able to amplify the marker D22S1623; SEQ ID NO: 37 and SEQ ID NO: 38 able to amplify the marker D22S264; SEQ ID NO: 51 and SEQ ID NO: 52 able to amplify the marker D22S311; SEQ ID NO: 61 and SEQ ID NO: 62 able to amplify the marker D22S1709; SEQ ID NO: 71 and SEQ ID NO: 72 able to amplify the marker D22S446; SEQ ID NO: 77 and SEQ ID NO: 78 able to amplify the marker D22S539; SEQ ID NO: 91 and SEQ ID NO: 92 able to amplify the marker D22S308; SEQ ID NO: 95 and SEQ ID NO: 96 able to amplify the marker D22S306; SEQ ID NO: 107 and SEQ ID NO: 108 able to amplify the marker D22S686; SEQ ID NO: 109 and SEQ ID NO: 110 able to amplify the marker D22S1174.

In a more preferred embodiment, the primers that are selected of the list consisting of: SEQ ID NO: 3 and SEQ ID NO: 4, able to amplify the marker CATCH5; SEQ ID NO: 5 and SEQ ID NO: 6 able to amplify the marker CATCH53; SEQ ID NO: 7 and SEQ ID NO: 8 able to amplify the marker CATCH52; SEQ ID NO: 13 and SEQ ID NO: 14 able to amplify the marker CATCH4; SEQ ID NO: 15 and SEQ ID NO: 16 able to amplify the marker CATCH6; SEQ ID NO: 23 and SEQ ID NO: 24 able to amplify the marker CATCH48; SEQ ID NO: 29 and SEQ ID NO: 30 able to amplify the marker CATCH22; SEQ ID NO: 31 and SEQ ID NO: 32 able to amplify the marker CATCH7; SEQ ID NO: 33 and SEQ ID NO: 34 able to amplify the marker CATCH23; SEQ ID NO: 35 and SEQ ID NO: 36 able to amplify the marker CATCH19; SEQ ID NO: 39 and SEQ ID NO: 40 able to amplify the marker CATCH45; SEQ ID NO: 41 and SEQ ID NO: 42 able to amplify the marker CATCH10; SEQ ID NO: 43 and SEQ ID NO: 44 able to amplify the marker CATCH20; SEQ ID NO: 45 and SEQ ID NO: 46 able to amplify the marker CATCH42; SEQ ID NO: 47 and SEQ ID NO: 48 able to amplify the marker CATCH41; SEQ ID NO: 49 and SEQ ID NO: 50 able to amplify the marker CATCH11; SEQ ID NO: 53 and SEQ ID NO: 54 able to amplify the marker CATCH12; SEQ ID NO: 55 and SEQ ID NO: 56 able to amplify the marker CATCH13; SEQ ID NO: 57 and SEQ ID NO: 58 able to amplify the marker CATCH14; SEQ ID NO: 59 and SEQ ID NO: 60 able to amplify the marker CATCH39; SEQ ID NO: 63 and SEQ ID NO: 64 able to amplify the marker CATCH38; SEQ ID NO: 65 and SEQ ID NO: 66 able to amplify the marker CATCH37; SEQ ID NO: 67 and SEQ ID NO: 68 able to amplify the marker CATCH36; SEQ ID NO: 69 and SEQ ID NO: 70 able to amplify the marker CATCH35; SEQ ID NO: 73 and SEQ ID NO: 74 able to amplify the marker CATCH51; SEQ ID NO: 75 and SEQ ID NO: 76 able to amplify the marker CATCH33; SEQ ID NO:79 and SEQ ID NO: 80 able to amplify the marker CATCH31; SEQ ID NO: 81 and SEQ ID NO: 82 able to amplify the marker CATCH25; SEQ ID NO: 83 and SEQ ID NO: 84 able to amplify the marker CATCH24; SEQ ID NO: 85 and SEQ ID NO: 86 able to amplify the marker CATCH26; SEQ ID NO: 87 and SEQ ID NO: 88 able to amplify the marker CATCH30; SEQ ID NO: 89 and SEQ ID NO: 90 able to amplify the marker CATCH29; SEQ ID NO: 93 and SEQ ID NO: 94 able to amplify the marker CATCH15; SEQ ID NO: 97 and SEQ ID NO: 98 able to amplify the marker CATCH16; SEQ ID NO: 99 and SEQ ID NO: 100 able to amplify the marker CATCH28; SEQ ID NO: 101 and SEQ ID NO: 102 able to amplify the marker CATCH17; SEQ ID NO: 103 and SEQ ID NO: 104 able to amplify the marker CATCH27; SEQ ID NO: 105 and SEQ ID NO: 106 able to amplify the marker CATCH 18; and/or any combination thereof.

In another preferred embodiment, the primers that are selected of the list consisting of: SEQ ID NO: 5 and SEQ ID NO: 6 able to amplify the marker CATCH53; SEQ ID NO: 105 and SEQ ID NO: 106 able to amplify the marker CATCH18; SEQ ID NO: 55 and SEQ ID NO: 56 able to amplify the marker CATCH13; SEQ ID NO: 33 and SEQ ID NO: 34 able to amplify the marker CATCH23; SEQ ID NO: 23 and SEQ ID NO: 24 able to amplify the marker CATCH48; SEQ ID NO: 97 and SEQ ID O: 98 able to amplify the marker CATCH16; SEQ ID NO: 35 and SEQ ID NO: 36 able to amplify the marker CATCH 19.

In another preferred embodiment, the primers that are selected of the list consisting of: SEQ ID NO: 5 and SEQ ID NO: 6 able to amplify the marker CATCH53; SEQ ID NO: 105 and SEQ ID NO: 106 able to amplify the marker CATCH18, SEQ ID NO: 21 and SEQ ID NO: 22 able to amplify the marker D22S941, SEQ ID NO: 55 and SEQ ID NO: 56 able to amplify the marker CATCH13, SEQ ID NO: 33 and SEQ ID NO: 34 able to amplify the marker CATCH23, SEQ ID NO: 107 and SEQ ID NO: 108 able to amplify the marker D22S686, SEQ ID NO: 9 and SEQ ID NO: 10 able to amplify the marker D22S427, SEQ ID NO: 23 and SEQ ID NO: 24 able to amplify the marker CATCH48, SEQ ID NO: 97 and SEQ ID NO: 98 able to amplify the marker CATCH16, SEQ ID NO: 35 and SEQ ID NO: 36 able to amplify the marker CATCH19, and SEQ ID NO: 37 and SEQ ID NO: 38 able to amplify the marker D22S264.

In another aspect, the present invention relates to the in vitro use of the primers of the invention for the detection of dup/del22q11.1 in an isolated biological sample of a subject.

In another aspect, the present invention relates to the in vitro use of the primers of the invention for the diagnosis and/or prognosis of the pathologies associated with deletions and/or duplications in the 22q11.2 chromosome region, preferably wherein the pathologies are selected of the list consisting of: congenital heart diseases, Digeorge syndrome, velocardiofacial syndrome, schizophrenia, mental retardation, and/or immunodeficiency.

### KIT OF THE INVENTION

The primer pairs designed for the application of the in vitro methods described in the present invention can be found comprising a "kit".

In the context of the present invention, "kit" refers to a product containing the different reagents for applying the method of the invention, adequately packed to allow its transportation and storage. The adequate materials for the packing of the kit components include, and are not limited to, glass, plastic (polyethylene, polypropylene, polycarbonate, and similars), bottles, vials, envelopes, and similars.

Thus, in another aspect, the invention relates to a kit, from now on "the kit of the invention", comprising at least one set of primer pairs able to identify the markers that are selected of the list consisting of: CATCH53, CATCH48, CATCH23, CATCH19, CATCH13, CATCH16, CATCH18, CATCH5, CATCH52, CATCH4, CATCH6, CATCH22, CATCH7, CATCH45, CATCH10, CATCH20, CATCH42, CATCH41, CATCH11, CATCH12, CATCH14, CATCH39, CATCH38, CATCH37, CATCH36, CATCH35, CATCH51, CATCH33, CATCH31, CATCH25, CATCH24, CATCH26, CATCH30, CATCH29, CATCH15, CATCH28, CATCH17, CATCH27, and/or any combination thereof and, optionally, the reagents to carry out the PCR and/or the reagents to isolate DNA from an isolated biological sample, as it is described in the present document.

In another preferred embodiment, the kit of the invention comprises at least one primer pair able to identify the markers that are selected of the list consisting of: D22S420, D22S1638, D22S420, 22K48-2, D22S1648, D22S944, D22S1623, D22S311, D22S1709, D22S446bis, D22S539, D22S308, D22S306, D22S1174, and/or any combination thereof.

In another preferred embodiment, the kit of the invention comprises the set of primer pairs able to identify the markers that are selected of the list consisting of: CATCH53, CATCH48, CATCH23, CATCH19, CATCH13, CATCH16, CATCH18, CATCH5, CATCH52, CATCH4, CATCH6, CATCH22, CATCH7, CATCH45, CATCH10, CATCH20, CATCH42, CATCH41, CATCH11, CATCH12, CATCH14, CATCH39, CATCH38, CATCH37, CATCH36, CATCH35, CATCH51, CATCH33, CATCH31, CATCH25, CATCH24, CATCH26, CATCH30, CATCH29, CATCH15, CATCH28, CATCH17, CATCH27.

In another preferred embodiment, the kit of the invention comprises the set of primer pairs able to identify the markers that are selected of the list consisting of: CATCH53, CATCH48, CATCH23, CATCH19, CATCH13, CATCH16, CATCH18, CATCH5, CATCH52, CATCH4, CATCH6, CATCH22, CATCH7, CATCH45, CATCH10, CATCH20, CATCH42, CATCH41, CATCH11, CATCH12, CATCH14, CATCH39, CATCH38, CATCH37, CATCH36, CATCH35, CATCH51, CATCH33, CATCH31, CATCH25, CATCH24, CATCH26, CATCH30, CATCH29, CATCH15, CATCH28, CATCH17, CATCH27, D22S420, D22S1638, D22S420, 22K48-2, D22S1648, D22S944, D22S1623, D22S311, D22S1709, D22S446bis, D22S539, D22S308, D22S306, D22S1174.

In another preferred embodiment, the kit of the invention comprises a set of primer pairs able to identify the markers that are selected of the list consisting of: CATCH53, CATCH48, CATCH23, CATCH19, CATCH13, CATCH16, and CATCH18. In another more preferred embodiment, the kit of the invention further comprises the set of primer pairs able to identify the markers that are selected of the list consisting of: CATCH53, CATCH48, CATCH23, CATCH19, CATCH13, CATCH16, CATCH18, D22S427, D22S941, D22S264, and D22S686.

As previously mentioned in the present document, the nucleotide sequences from the pairs that identify each of the markers of the invention are described in **Table 4.**

As those skilled in the art know, the kit of the invention, apart from being comprended of at least one of the primer pairs previously indicated, it can optionally include the reagents necessary for the application of the multiplex amplification reaction, including, but not limited to, deoxyribonucleotide triphosphate (dNTPs), divalent and/or monovalent ions, a buffer solution maintaining the adequate pH for the functioning of the polymerase DNA, polymerase DNA o a mixture of different polymerases, etc. Nevertheless, if the kit of the invention is not comprised of the reagents necessary for applying the method of the invention, these are commercially available and can also be found comprising another kit. Any kit from those commercially available containing the reagents necessary for applying an amplification reaction, such as the Qiagen Multiplex PCR Kit (Qiagen, Valencia, CA), can be successfully used for the application of the method of the invention. Also, other reagents that are used in the application of the method of the invention and that can also be part of the kit include the material necessary for obtaining the biological sample from the individual, such as clamps, gauze, swabs, cotton swabs, syringes, etc., and the reagents used for isolating the DNA from the biological sample obtained, such as alcohol, detergents, etc.

Additionally, the kits may contain the instructions for the use of the different components found in the kit. Those instructions may be found as print or digital material, in such a way that these can be read by an individual, such as in electronic storage media (magneto-optical drives, cassete, and similars), optical drives (CD-ROM, DVD) and similars. Additionally or alternatively, the media may contain web addresses, which contain those instructions.

Throughout the description and claims, the term "comprise" and its variants do not pretend to exclude other technical characteristics, additives, components, or steps. Additional objects, advantages and features of the invention will become apparent to those skilled in the art upon examination of the description or application of the invention. The following examples and figures are given as a way of illustration and they do not pretend to limit the present invention.

### EXAMPLES.

### EXAMPLE 1. Development of the in vitro method of the invention for the detection of deletions and/or duplications in the 22q11.2 chromosome region.

### Identification of the LCR22s of the 222q11.2 region

The precise identification of the limits of the LCR22 of the 22q11.2 region has been carried out in the context of the last version of the sequence of the human geneome (UCSC; version of February 2009 Human Reference Sequence (GRCh37)). To this end, the consecutive frame sequences of 25 Kb (between the positions 16 and 23 Mb of chromosome 22) were compared with the complete sequence of the human genome by means of the BLAST software from the NCBI (http://www.ncbi.nlm.nih.gov/BLAST/) and the BLAT software from the UCSC Genome Browse (http://genome.ucsc.edu/). This strategy allowed for the detection of identical or almost identical sequences located in different positions to those known in the chromosome 22 or even in other chromosomes forming LCR22s.

The precise position and size obtained for the LCR22s were the following (See **Table 1):** LCR22-A 18,642,491-19,023,012 (380,521 Kb); LCR22-B 20,249,559-20,731,985 (482,426 Kb); LCR22-C 21,021,869-21,074,326 (52,457 Kb); LCR22-D 21,465,674-21,917,116 (451,422 Kb); LCR22-E 22,963,079-23,136,903 (173,824 Kb), and LCR22-F 23,649,113- 24,021,303 (372,219 Kb).

### Identification of new STR markers for the 22q11.2 chromosome region

The repetitions that form the new polymorphic STR markers were identified by using the "Tandem Repeats Finder" from the sequence of the human genome of reference of the "UCSC Genome Browser" (http://genome.ucsc.edu/). By means of this software tandem repeats or microsatellites are identified throughout the genome.

The STR polymorphic markers identified in the present invention are shown in **Table 2,** and they have been given the generic name "CATCH" followed by a numbering scheme. As it can be observed in **Table 2,** a total of 38 new polymorphic STR markers that identify the 222q11.2 genome region have been identified.

On the other hand, in order to obtain an even more precise and accurate identification from the analyzed individual through the in vitro method described in the invention, the CATCH markers of the invention have been combined with other polymorphic STR markers already known in prior art, which also identify the 222q11.2 genome region. Those known polymorphic markers are described in **Table 3.**

All these polymorphic STR markers, both the new previously described (CATCH) as those already known, are localized between the positions 17,859,476 (D22S420) pb and 24,488,486 (D22S1174) pb of chromosome 22, which corresponds approximately to the zone between LCR22-A and LCR22-F **(Table 1);** and they are described in **Table 2.**

Once the polymorphic CATCH markers of the invention have been identified, the specific primers for their amplification by PCR techniques were designed.

### Design of the primers used in the PCR technique that recognize the markers of the invention

The design of the primers for the amplification of the STR markers of the invention by PCR techniques was carried out using the "Oligos 9.6" software available at the Institute of Biotechnology of the University of Helsinki's website (www.biocenter.helsinki.fi/bi/DNA/links.htm). Once their sequences were obtained, the sequences surrounding the STRs were compared with the rest of the geneome by means of the BLAST software from the NCBI (http://www.ncbi.nlm.nih.gov/BLAST/) and the BLAT from the UCSC Genome Browser so as to insure the existence of a single copy of the markers.

The primers de novo designed for the identification of the new STR markers of the present invention, named CATCH, are shown in **Table 4,** along with the primers identifying the STR markers already known in prior art and the primers identifying the non-polymorphic STS markers, also known in prior art.

Determination of the heterozygosity of the markers analyzed in the present invention.

The heterozygosity of the markers was determined typing each one of them in 100 DNA samples of the analyzed population under study. The heterozygosity was calculated by dividing the number of heterozygous DNA samples with no kindred by the total number of analyzed samples. The heterozygosity results for each of the analyzed markers are shown in **Table 5.**

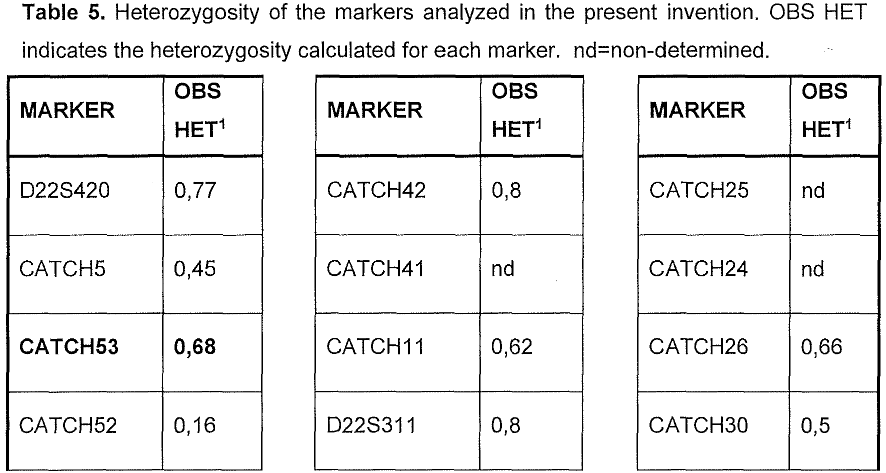

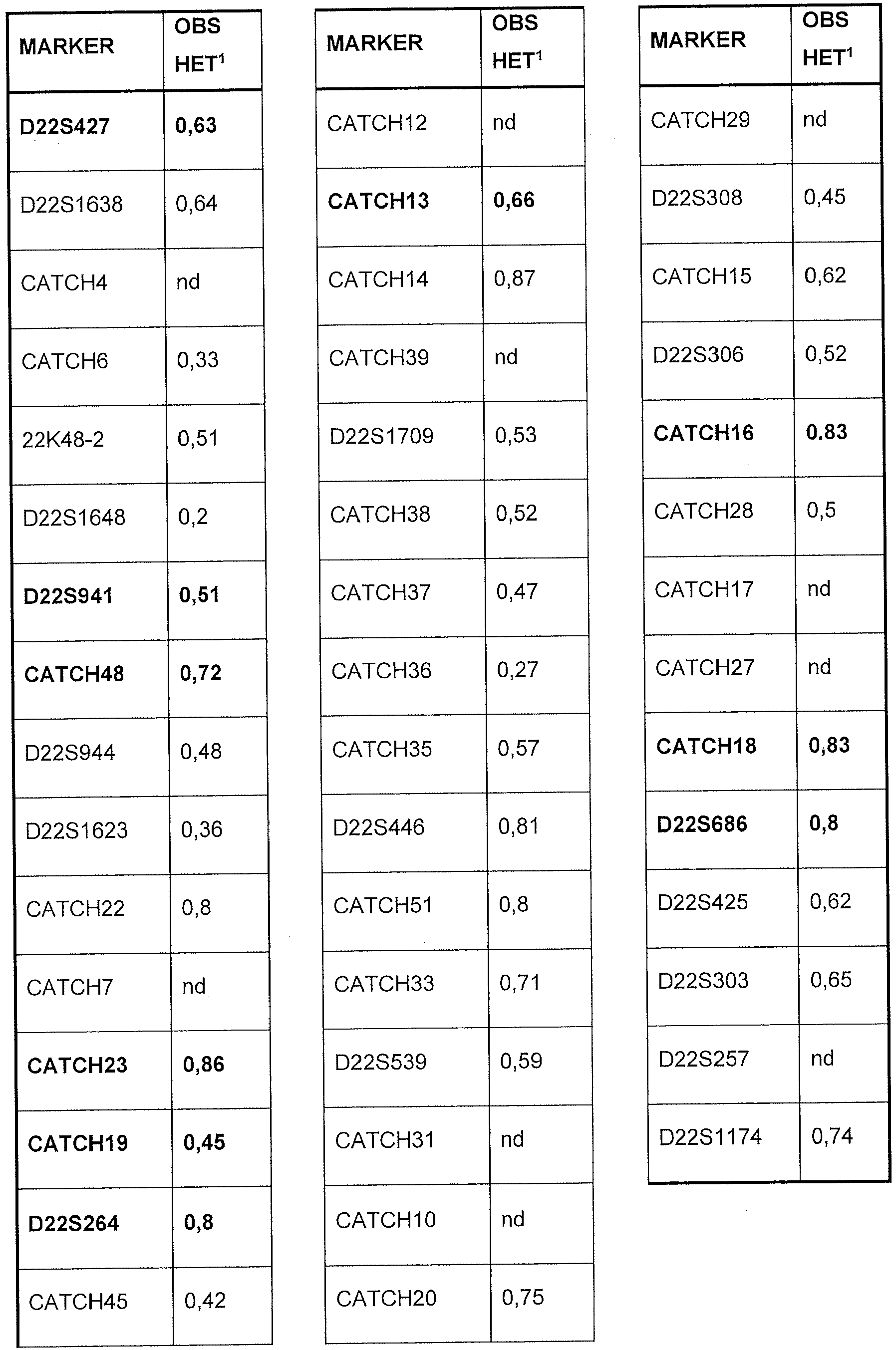

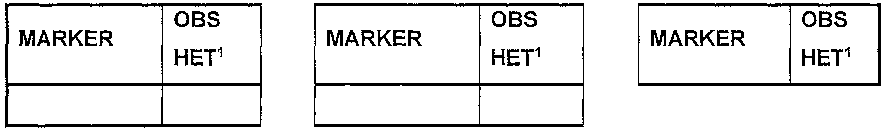

### Selection of the CATCH markers of the invention used for its identification in a subject's sample by means of multiplex PCR techniques

For the selection of the markers intended for the multiplex PCR, the most polymorphic markers (those with a higher heterozygosity) and those with best results were separated.

Next, eleven markers were selected, which comprised polymorphic STR markers of the present invention (CATCH) and polymorphic markers already known as well, so as there were at least one of the markers in every interval delimited by LCR22. The eleven selected markers were:
- marker localized in the previous region to the LCR22A region: CATCH53 and D22S427,
- marker localized in the inter-LCR22A-B region: CATCH48, CATCH19, CATCH23, and D22S941,
- marker localized in the inter-LCR22B-C region: D22S264,
- marker localized in the inter-LCR22C-D region: CATCH13,
- marker localized in the inter-LCR22D-E region: CATCH16 and CATCH18, and
- marker localized in the region subsequent to LCR22E: D22S686.

Two multiplex PCR reactions were designed with the eleven selected markers. The size of the markers that were to be amplified was taken into account in order to mark with different fluorochromes those with a similar size. This way, two multiplex PCR were designed (M1 and M2), as **Table 6** shows.

Once the marked primers were obtained, the preparations began for determining the optimal conditions for amplification. First, two conditions (condition A and condition B) of different PCR were tested:
**Condition A.-** In a final volume of 25 µl with 100-150 ng of DNA, buffer 1,4x, 0.3 mM dNTP, 80 nM from each primer, 2 mM of magnesium chloride, and 2U of Taq DNA polymerase.
**Condition B.-** In a final volume of 25 µl with 100-150 ng of DNA, buffer 1x, 0.2 mM dNTP, 80 nM from each primer, 1.5 mM of magnesium chloride, and 2U of Taq DNA polymerase.

The amplification reactions were carried out with a "MJ research" thermocycler. As for the parameters for the amplification, these were: 30 cycles of denaturing for 10 seconds at 94 °C (2 minutes for the first cycle); annealing at 54 °C for 45 seconds; and extension at 65 °C for 2 minutes. Finally, the reactions were analyzed with an automated sequencer.

After analyzing the obtained results with both A or B conditions, we concluded that Condition A was the better option.

**TABLE 6. Multiplex PCRs. Genetic markers composing the two multiplex PCR (M1 and M2) and size of the most frequent alleles.**

| | **MARKER** | **FLUOROCHROME** | **MOST FREQUENT ALLELES (pb of the product from the PCR obtained)** |
|---|---|---|---|
| M1 | CATCH53 | FI | 140,142,144,154,156 |
| | CATCH 18 | FI | 174,176,178,180,182,186,188,192 |
| | D22S941 | FI | 223,227,237,247,249,250,252,254,256 |
| | CATCH13 | HEX | 126,137,141,143,145,147 |
| | CATCH23 | HEX | 201,206,210,214,219 |
| | D22S686 | HEX | 269,288,292 |
| | | | |
| M2 | D22S427 | FI | 90,92,94,96,98,100 |
| | CATCH48 | FI | 168,170,172,181,186,189,200,202,204 |
| | CATCH16 | HEX | 80,82,85,99,100,102 |
| | CATCH19 | HEX | 185,187 |
| | D22S264 | TAMRA | 178,189,197,199,203,206,210 |

### EXAMPLE 2. Validation of the in vitro method of the invention for the detection of deletions and/or duplications in the 22q11.2 chromosome region.

In order to express the utility of the in vitro method of the invention for the identification of deletions and/or duplications in the 22q11.2, a total of 104 patients with suspected presence of deletions and/or duplications in the 22q11.2 region had samples of their DNA analyzed.

As it has been established throughout the present document, the 22q11.2 genome region was divided in six intervals delimited by different LCR22 (Table 1). In both of the multiplex PCR carried out, M1 and M2, at least three polymorphic STR markers of the invention (CATCH) were included in both combinations, wherein those CATCH markers identified the identified intervals of the 22q11.2 region so as to detect any any deletion and/or duplication in the 22q11.2 region with no need of a parental DNA sample. Only in cases of prenatal diagnosis the maternal DNA was used, due to the DNA being extracted from the amniotic fluid and it is necessary to have the maternal DNA in order to rule out maternal contamination. Thus, the presence of two alleles in one marker of more of those used for each inter-LCR22 interval allows for ruling out the presence of any deletion between LCRs.

The results obtained through the two multiplex PCR (M1 and M2) in the samples from the 104 patients showed that 5 patients presented deletions (which were later confirmed through FISH).

As it can be observed in **FIG. 1****,** the rest of the patients presented at least one informative marker with 2 alleles (heterozygous) in the interval between LCR22A in (18,642,491-19,023,012 pb) and LCR22B (20,249,559-20,731,985 pb), thus ruling out the most frequent deletions of 1.5 and 1.3 Mb in the 100% of those cases because both deletions include the LCR22A-LCR22B interval.

**FIG. 2** shows the capillary electrophoresis of the resulting products from the two multiplex PCR of the invention that were carried out on a biological sample of DNA from a healthy individual. As it can be observed in the figure, this individual is heterozygous (has 2 alleles) for the markers CATCH48, CATCH13, CATCH19, D22S264, CATCH18, D22S941, and CATCH23; thus indicating that the individual does not carry any deletion in the 22q11.2 chromosome region.

**FIG. 3** shows the capillary electrophoresis of the resulting products from the two multiplex PCR of the invention that were carried out on a biological sample of DNA from an individual presenting a 3 Mb deletion. As it can be observed in the figure, the electrophoresis shows that the individual is homozygous (has a single allele) for the markers CATCH48, CATCH13, CATCH19, D22S264, CATCH53, D22S941, and CATCH23; thus confirming, by means of the described method of the present invention, that the individual carries a 3 Mb deletion.

**FIG. 4** shows the results of the capillary electrophoresis of the resulting products from the two multiplex PCR of the invention that were carried out on a biological sample of DNA from an individual with a 3 Mb deletion along with a map for the analysed 22q11.2 region. All markers located within the interval between the LCR22 A and D (D22S427), CATCH48, CATCH13, CATCH19, D22S264, CATCH53, D22S941, CATCH23) included in the 3Mb deletion are homozygous (a single allele), thus indicating the presence of such 3 Mb deletion.

**FIG.5** shows the results obtained through gel electrophoresis of the resulting products from the two multiplex PCR of the invention in a biological sample of DNA from an individual with a 3 Mb deletion in the 22q11.2 region, as well as in a biological sample of DNA from their progenitors. The deleted interval is shown in the figure by means of horizontal bars with shading. This test is conclusive when carried out in presence of the parents; in this case, the presence of a deletion due to not inheriting the paternal allele. Therefore, this is a de novo deletion with paternal origin.

**FIG. 6** shows the capillary electrophoresis of the resulting products from the two multiplex PCR of the invention that were carried out on a biological sample of DNA from an individual presenting a 1 Mb duplication. The electrophoresis shows how the CATCH13 and the D22S264 markers present three alleles, which is conclusive evidence of an atypical duplication in such region.

## Claims

1. *In vitro* method for detecting duplications and/or deletions of 22q11.2 chromosome region in a subject comprising the identification of the number of alleles in an isolated biological sample from that subject that are present in the markers localized in the following regions of chromosome 22:
(i) the previous region to the LCR22A region located between the positions 18,642,491-19,023,012 pb of chromosome 22, by identifying at least one marker selected from the list consisting of: CATCH5 (SEQ ID NO: 111), CATCH53 (SEQ ID NO: 112), CATCH52 (SEQ ID NO: 113) and/or any combination thereof;
(ii) the inter-LCR22A-B region, delimited by the LCR22A (located between positions 18,642,491-19,023,012 pb) and the LCR22B (located between positions 20,249,559-20,731,985 pb) regions of chromosome 22, by identifying at least one marker selected from the list consisting of: CATCH4 (SEQ ID NO: 114), CATCH6 (SEQ ID NO: 115), CATCH48 (SEQ ID NO: 116), CATCH22 (SEQ ID NO: 117), CATCH19 (SEQ ID NO: 118), CATCH7 (SEQ ID NO: 119), CATCH23 (SEQ ID NO: 120) and/or any combination thereof;
(iii) the inter-LCR22B-C region, delimited by the LCR22B (located between positions 20,249,559-20,731,985 pb) and the LCR22C (located between positions 21,021,869-21,074,326 pb) regions of chromosome 22, by identifying at least one marker selected from the list consisting of: CATCH45 (SEQ ID NO: 121), CATCH10 (SEQ ID NO: 122), CATCH20 (SEQ ID NO: 123), and/or any combination thereof;
(iv) the inter-LCR22C-D region, delimited by the LCR22C (located between positions 21,021,869-21,074,326 pb) and the LCR22D (located between positions 21,465,674-21,917,116 pb) regions of chromosome 22, by identifying at least one marker selected from the list consisting of: CATCH42 (SEQ ID NO: 124), CATCH41 (SEQ ID NO: 125), CATCH11 (SEQ ID NO: 126), CATCH12 (SEQ ID NO: 127), CATCH13 (SEQ ID NO: 128), CATCH14 (SEQ ID NO: 129), CATCH39 (SEQ ID NO: 130), CATCH38 (SEQ ID NO: 131), CATCH37 (SEQ ID NO: 132), and/or any combination thereof; and
(v) the inter-LCR22D-E region, delimited by the LCR22D (located between positions 21,465,674-21,917,116 pb) and the LCR22E (located between positions 22,963,079-22,997,578pb pb) regions of chromosome 22, by identifying at least one marker selected from the list consisting of: CATCH36 (SEQ ID NO: 133), CATCH35 (SEQ ID NO: 134), CATCH31 (SEQ ID NO: 135), CATCH25 (SEQ ID NO: 136), CATCH24 (SEQ ID NO: 137), CATCH26 (SEQ ID NO: 138), CATCH30 (SEQ ID NO: 139), CATCH29 (SEQ ID NO: 140), CATCH15 (SEQ ID NO: 141), CATCH16 (SEQ ID NO: 142), CATCH28 (SEQ ID NO: 143), CATCH17 (SEQ ID NO: 144), CATCH18 (SEQ ID NO: 145), CATCH33 (SEQ ID NO: 146), CATCH27 (SEQ ID NO: 147), CATCH51 (SEQ ID NO: 148), and/or any combination thereof;
wherein a number of alleles less than two in at least one of the markers from at least one of the regions (i) to (v) indicates a deletion in said 22q11.2 region, and wherein a number of alleles equal to or greater than three in at least one of the markers from at least one of the regions (i) to (v) indicates a duplication in said 22q11.2 region.

2. The *in vitro* method according to claim 1, wherein the method comprises:
(i) Identifying the number of alleles present in the group of markers selected of the list consisting of: CATCH5 (SEQ ID NO: 111), CATCH53 (SEQ ID NO: 112) y CATCH52 (SEQ ID NO: 113), located in the previous region to the LCR22A region;
(ii) Identifying the number of alleles present in the group of markers selected of the list consisting of: CATCH4 (SEQ ID NO: 114), CATCH6 (SEQ ID NO: 115), CATCH48 (SEQ ID NO: 116), CATCH22 (SEQ ID NO: 117), CATCH19 (SEQ ID NO: 118), CATCH7 (SEQ ID NO: 119), and CATCH23 (SEQ ID NO: 120), located in the inter-LCR22A-B region;
(iii) Identifying the number of alleles present in the group of markers selected of the list consisting of: CATCH45 (SEQ ID NO: 121), CATCH10 (SEQ ID NO: 122) y CATCH20 (SEQ ID NO: 123), located in the inter-LCR22B-C region;
(iv) Identifying the number of alleles present in the group of markers selected of the list consisting of: CATCH42 (SEQ ID NO: 124), CATCH41 (SEQ ID NO: 125), CATCH11 (SEQ ID NO: 126), CATCH12 (SEQ ID NO: 127), CATCH13 (SEQ ID NO: 128), CATCH14 (SEQ ID NO: 129), CATCH39 (SEQ ID NO: 130), CATCH38 (SEQ ID NO: 131) and CATCH37 (SEQ ID NO: 132), located in the inter-LCR22C-D region;
(v) Identifying the number of alleles present in the group of markers selected of the list consisting of: CATCH36 (SEQ ID NO: 133), CATCH35 (SEQ ID NO: 134), CATCH31 (SEQ ID NO: 135), CATCH25 (SEQ ID NO: 136), CATCH24 (SEQ ID NO: 137), CATCH26 (SEQ ID NO: 138), CATCH30 (SEQ ID NO: 139), CATCH29 (SEQ ID NO: 140), CATCH15 (SEQ ID NO: 141), CATCH16 (SEQ ID NO: 142), CATCH28 (SEQ ID NO: 143), CATCH17 (SEQ ID NO: 144), CATCH18 (SEQ ID NO: 145), CATCH33 (SEQ ID NO: 146), CATCH27 (SEQ ID NO: 147), and CATCH51 (SEQ ID NO: 148), located in the inter-LCR22D-E region.

3. The *in vitro* method according to any of claims 1 or 2, wherein the method further comprises:
(i)Identifying the number of alleles present in at least one marker selected of the list consisting of: D22S420 (SEQ ID NO: 149), D22S427 (SEQ ID NO: 150), and/or any combination thereof, located in the LCR22A region;
(ii) Identifying the number of alleles present in at least one marker selected of the list consisting of: D22S1638 (SEQ ID NO: 151), 22K48-2 (SEQ ID NO: 152), D22S1648 (SEQ ID NO: 153), D22S941 (SEQ ID NO: 154), D22S944 (SEQ ID NO: 155), D22S1623 (SEQ ID NO: 156), and/or any combination thereof, located in the inter-LCR22A-B region;
(iii) Identifying the number of alleles present in the marker: D22S264 (SEQ ID NO: 157), located in the inter-LCR22B-C region;
(iv) Identifying the number of alleles present in at least one marker selected of the list consisting of: D22S311 (SEQ ID NO: 158), D22S1709 (SEQ ID NO: 159), and/or any combination thereof, located in the inter-LCR22C-D region;
(v) Identifying the number of alleles present in at least one marker selected of the list consisting of: D22S446 (SEQ ID NO: 160), D22S539 (SEQ ID NO: 161), D22S308 (SEQ ID NO: 162), D22S306 (SEQ ID NO: 163), and/or any combination thereof, located in the inter-LCR22D-E region, and
(vi) Identifying the number of alleles present in at least one marker selected of the list consisting of: D22S686 (SEQ ID NO: 164), D22S425 (SEQ ID NO: 165), D22S1174 (SEQ ID NO: 167), and D22S257 (SEQ ID NO: 166), and/or any combination thereof, located in the region subsequent to LCR22E.
wherein a number of alleles less than two in at least one of the markers from at least one of the regions (i) to (vi) indicates a deletion in the 22q11.2 region, and wherein a number of alleles equal to or greater than three in at least one of the markers fromat least one of the regions (i) to (vi) indicates a duplication in the 22q11.2 region.

4. The *in vitro* method according to any of claims 1 or 3, wherein the method comprises:
(i) Identifying the number of alleles present in the markers selected of the list consisting of: CATCH53 (SEQ ID NO: 112) and D22S427 (SEQ ID NO: 150), located in the previous region to the LCR22A region of chromosome 22;
(ii) Identifying the number of alleles present in the markers selected of the list consisting of: CATCH48 (SEQ ID NO: 116), CATCH19 (SEQ ID NO: 118), CATCH23 (SEQ ID NO: 120) and D22S941 (SEQ ID NO: 154), located in the inter-LCR22A-B region of chromosome 22;
(iii) Identifying the number of alleles present in the polymorphic markers selected of the list consisting of: D22S264 (SEQ ID NO: 157), located in the inter-LCR22B-C region of chromosome 22;
(iv) Identifying the number of alleles present in the polymorphic markers selected of the list consisting of: CATCH13 (SEQ ID NO: 128) located in the inter-LCR22C-D region of chromosome 22;
(v) Identifying the number of alleles present in the polymorphic markers selected of the list consisting of: CATCH16 (SEQ ID NO: 142), and CATCH18 (SEQ ID NO: 145), located in the inter-LCR22D-E region of chromosome 22; and
(vi) Identifying the number of alleles present in the marker D22S686 (SEQ ID NO: 194), located in the region subsequent to the LCR22E region of chromosome 22.

5. The *in vitro* method according to any of claims 1 to 4, wherein the method comprises:
(i) Identifying the number of alleles present in the markers selected of the list consisting of: CATCH5 (SEQ ID NO: 111), CATCH53 (SEQ ID NO: 112), CATCH52 (SEQ ID NO: 113), D22S420 (SEQ ID NO: 149), D22S427 (SEQ ID NO: 150) and D22S1638 (SEQ ID NO: 151), located in the previous region to theLCR22A region of chromosome 22;
(ii)Identifying the number of alleles present in the markers selected of the list consisting of: CATCH4 (SEQ ID NO: 114), CATCH6 (SEQ ID NO: 115), CATCH48 (SEQ ID NO: 116), CATCH22 (SEQ ID NO: 117), CATCH19 (SEQ ID NO: 118), CATCH7 (SEQ ID NO: 119), CATCH23 (SEQ ID NO: 120), 22K48-2 (SEQ ID NO: 152), D22S1648 (SEQ ID NO: 153), D22S941 (SEQ ID NO: 154), D22S944 (SEQ ID NO: 155), D22S1623 (SEQ ID NO: 156), located in the inter-LCR22A-B region of chromosome 22;
(iii) Identifying the number of alleles present in the markers selected of the list consisting of: CATCH45 (SEQ ID NO: 121), CATCH10 (SEQ ID NO: 122), CATCH20 (SEQ ID NO: 123), D22S264 (SEQ ID NO: 157), located in the inter-LCR22B-C region of chromosome 22;
(iv) Identifying the number of alleles present in the markers selected of the list consisting of: CATCH42 (SEQ ID NO: 124), CATCH41 (SEQ ID NO: 125), CATCH11 (SEQ ID NO: 126), CATCH12 (SEQ ID NO: 127), CATCH13 (SEQ ID NO: 128), CATCH14 (SEQ ID NO: 129), CATCH39 (SEQ ID NO: 130), CATCH38 (SEQ ID NO: 131), CATCH37 (SEQ ID NO: 132), D22S311 (SEQ ID NO: 158), y D22S1709 (SEQ ID NO: 159), located in the inter-LCR22C-D region of chromosome 22;
(v)ldentifying the number of alleles present in the markers selected of the list consisting of: CATCH36 (SEQ ID NO: 133), CATCH35 (SEQ ID NO: 134), CATCH31 (SEQ ID NO: 135), CATCH25 (SEQ ID NO: 136), CATCH24 (SEQ ID NO: 137), CATCH26 (SEQ ID NO: 138), CATCH30 (SEQ ID NO: 139), CATCH29 (SEQ ID NO: 140), CATCH15 (SEQ ID NO: 141), CATCH16 (SEQ ID NO: 142), CATCH28 (SEQ ID NO: 143), CATCH17 (SEQ ID NO: 144), CATCH18 (SEQ ID NO: 145), CATCH33 (SEQ ID NO: 146), CATCH27 (SEQ ID NO: 147) and CATCH51 (SEQ ID NO: 148), D22S446 (SEQ ID NO: 160), D22S539 (SEQ ID NO: 161), D22S308 (SEQ ID NO: 162), D22S306 (SEQ ID NO: 163), located in the inter-LCR22D-E region of chromosome 22; and
(vi) Identifying the number of alleles present in the markers selected of the list consisting of: D22S686 (SEQ ID NO: 164), D22S425 (SEQ ID NO: 165), D22S257 (SEQ ID NO: 166), D22S1174 (SEQ ID NO: 167), located in the region subsequent to the LCR22E region.

6. The *in vitro* method according to any of claims 1 to 5, wherein the identification of the number of alleles from each marker is carried out by PCR techniques.

7. The *in vitro* method according to claim 6, wherein the PCR technique is a multiplex PCR.

8. The *in vitro* method according to any of claims 1 to 7, wherein the identification of the number of alleles, depending on the selected marker, is carried out by the primer pairs selected of the list consisting of: SEQ ID NO: 3 and SEQ ID NO: 4, SEQ ID NO: 5 and SEQ ID NO: 6, SEQ ID NO: 7 and SEQ ID NO: 8, SEQ ID NO: 13 and SEQ ID NO: 14, SEQ ID NO: 15 and SEQ ID NO: 16, SEQ ID NO: 23 and SEQ ID NO: 24, SEQ ID NO: 29 and SEQ ID NO: 30, SEQ ID NO: 31 and SEQ ID NO: 32, SEQ ID NO: 33 and SEQ ID NO: 34, SEQ ID NO: 35 and SEQ ID NO: 36, SEQ ID NO: 39 and SEQ ID NO: 40, SEQ ID NO: 41 and SEQ ID NO: 42, SEQ ID NO: 43 and SEQ ID NO: 44, SEQ ID NO: 45 and SEQ ID NO: 46, SEQ ID NO: 47 and SEQ ID NO: 48, SEQ ID NO: 49 and SEQ ID NO: 50, SEQ ID NO: 53 and SEQ ID NO: 54, SEQ ID NO: 55 and SEQ ID NO: 56, SEQ ID NO: 57 and SEQ ID NO: 58, SEQ ID NO: 59 and SEQ ID NO: 60, SEQ ID NO: 63 and SEQ ID NO: 64, SEQ ID NO: 65 and SEQ ID NO: 66, SEQ ID NO: 67 and SEQ ID NO: 68, SEQ ID NO: 69 and SEQ ID NO: 70, SEQ ID NO: 73 and SEQ ID NO: 74, SEQ ID NO: 75 and SEQ ID NO: 76, SEQ ID NO: 79 and SEQ ID NO: 80, SEQ ID NO: 81 and SEQ ID NO: 82, SEQ ID NO: 83 and SEQ ID NO: 84, SEQ ID NO: 85 and SEQ ID NO: 86, SEQ ID NO: 87 and SEQ ID NO: 88, SEQ ID NO: 89 and SEQ ID NO: 90, SEQ ID NO: 93 and SEQ ID NO: 94, SEQ ID NO: 97 and SEQ ID NO: 98, SEQ ID NO: 99 and SEQ ID NO: 100, SEQ ID NO: 101 and SEQ ID NO: 102, SEQ ID NO: 103 and SEQ ID NO: 104, SEQ ID NO: 105 and SEQ ID NO: 106, SEQ ID NO: 1 and SEQ ID NO: 2, SEQ ID NO: 9 and SEQ ID NO: 10, SEQ ID NO: 11 and SEQ ID NO: 12, SEQ ID NO: 17 and SEQ ID NO: 18, SEQ ID NO: 19 and SEQ ID NO: 20, SEQ ID NO: 21 and SEQ ID NO: 22, SEQ ID NO: 25 and SEQ ID NO: 26, SEQ ID NO: 27 and SEQ ID NO: 28, SEQ ID NO: 37 and SEQ ID NO: 38, SEQ ID NO: 51 and SEQ ID NO: 52, SEQ ID NO: 61 and SEQ ID NO: 62, SEQ ID NO: 71 and SEQ ID NO: 72, SEQ ID NO: 77 and SEQ ID NO: 78, SEQ ID NO: 91 and SEQ ID NO: 92, SEQ ID NO: 95 and SEQ ID NO: 96, SEQ ID NO: 107 and SEQ ID NO: 108 and SEQ ID NO: 109 and SEQ ID NO: 110.

9. *In vitro* method for diagnosing pathologies associated with deletions and/or duplications in the 22q11.2 chromosome region, in an isolated biological sample from a subject, comprises the method according to any of claims 1 to 8, wherein the detection of a duplication and/or deletion in the 22q11.2 chromosome region indicates that the subject presents a pathology that is selected from the list consisting of: congenital heart disease, DiGeorge syndrome, velocardiofacial syndrome, schizophrenia, mental retardation, and immunodeficiency.

10. The *in vitro* method according to claim 9, wherein the congenital heart diseases is selected from the list consisting of: atrial septal defect, ventricular septal defect, Fallot tetralogy, transposition of the great vessels, aortic valve stenosis, pulmonary atresia, double outlet right ventricle, interrupted aortic arch, patent ductus arteriosus, hypoplastic left heart syndrome, persistent truncus arteriosus or truncus arteriosus, atrial septal defect, ventricular septal defect, tricuspid atresia, Ebstein anomaly, dextrocardia, coarctation of the aorta, patent ductus arteriosus, scimitar syndrome and single ventricle.

11. The *in vitro* method according to any of claims 9 to 10, wherein the diagnosis is a prenatal diagnosis.

12. The *in vitro* method according to any of claims 1 to 11, wherein the isolated biological sample is selected from the list consisting of: biological fluids and/or tissues.

13. The *in vitro* method according to claim 12, wherein the biological fluid sample is selected from the list consisting of: blood, plasma, serum, amniotic fluid.

14. Kit or array for detecting duplications and/or deletions in the 22q11.2 chromosome region, that comprises at least a primer pair that are selected from the list consisting of: SEQ ID NO: 5 and SEQ ID NO: 6, SEQ ID NO: 23 and SEQ ID NO: 24, SEQ ID NO: 35 and SEQ ID NO: 36, SEQ ID NO: 33 and SEQ ID NO: 34, SEQ ID NO: 55 and SEQ ID NO: 56, SEQ ID NO: 97 and SEQ ID NO: 98, SEQ ID NO: 105 and SEQ ID NO: 106 and/or any combination thereof.

15. The kit or array according to claim 14 that further comprises at least one of primer pairs that are selected from the list consisting of: SEQ ID NO: 3 and SEQ ID NO: 4, SEQ ID NO: 7 and SEQ ID NO: 8, SEQ ID NO: 13 and SEQ ID NO: 14, SEQ ID NO: 15 and SEQ ID NO: 16, SEQ ID NO: 29 and SEQ ID NO: 30, SEQ ID NO: 31 and SEQ ID NO: 32, SEQ ID NO: 39 and SEQ ID NO: 40, SEQ ID NO: 41 and SEQ ID NO: 42, SEQ ID NO: 43 and SEQ ID NO: 44, SEQ ID NO: 45 and SEQ ID NO: 46, SEQ ID NO: 47 and SEQ ID NO: 48, SEQ ID NO: 49 and SEQ ID NO: 50, SEQ ID NO: 53 and SEQ ID NO: 54, SEQ ID NO: 57 and SEQ ID NO: 58; SEQ ID NO: 59 and SEQ ID NO: 60, SEQ ID NO: 63 and SEQ ID NO: 64, SEQ ID NO: 65 and SEQ ID NO: 66, SEQ ID NO: 67 and SEQ ID NO: 68, SEQ ID NO: 69 and SEQ ID NO: 70, SEQ ID NO: 73 and SEQ ID NO: 74, SEQ ID NO: 75 and SEQ ID NO: 76, SEQ ID NO: 79 and SEQ ID NO: 80, SEQ ID NO: 81 and SEQ ID NO: 82, SEQ ID NO: 83 and SEQ ID NO: 84, SEQ ID NO: 85 and SEQ ID NO: 86, SEQ ID NO: 87 and SEQ ID NO: 88, SEQ ID NO: 89 and SEQ ID NO: 90, SEQ ID NO: 93 and SEQ ID NO: 94, SEQ ID NO: 99 and SEQ ID NO: 100, SEQ ID NO: 101 and SEQ ID NO: 102, SEQ ID NO: 103 and SEQ ID NO: 104, and/or any combination thereof.

16. The kit or array according to any of claims 14 to 15, that comprises the primer pairs that are selected from the list consisting of: SEQ ID NO: 5 and SEQ ID NO: 6, SEQ ID NO: 23 and SEQ ID NO: 24, SEQ ID NO: 35 and SEQ ID NO: 36, SEQ ID NO: 33 and SEQ ID NO: 34, SEQ ID NO: 55 and SEQ ID NO: 56, SEQ ID NO: 97 and SEQ ID NO: 98, SEQ ID NO: 105 and SEQ ID NO: 106, SEQ ID NO: 3 and SEQ ID NO: 4, SEQ ID NO: 7 and SEQ ID NO: 8, SEQ ID NO: 13 and SEQ ID NO: 14, SEQ ID NO: 15 and SEQ ID NO: 16, SEQ ID NO: 29 and SEQ ID NO: 30, SEQ ID NO: 31 and SEQ ID NO: 32, SEQ ID NO: 39 and SEQ ID NO: 40, SEQ ID NO: 41 and SEQ ID NO: 42, SEQ ID NO: 43 and SEQ ID NO: 44, SEQ ID NO: 45 and SEQ ID NO: 46, SEQ ID NO: 47 and SEQ ID NO: 48, SEQ ID NO: 49 and SEQ ID NO: 50, SEQ ID NO: 53 and SEQ ID NO: 54, SEQ ID NO: 57 and SEQ ID NO: 58; SEQ ID NO: 59 and SEQ ID NO: 60, SEQ ID NO: 63 and SEQ ID NO: 64, SEQ ID NO: 65 and SEQ ID NO: 66, SEQ ID NO: 67 and SEQ ID NO: 68, SEQ ID NO: 69 and SEQ ID NO: 70, SEQ ID NO: 73 and SEQ ID NO: 74, SEQ ID NO: 75 and SEQ ID NO: 76, SEQ ID NO: 79 and SEQ ID NO: 80, SEQ ID NO: 81 and SEQ ID NO: 82, SEQ ID NO: 83 and SEQ ID NO: 84, SEQ ID NO: 85 and SEQ ID NO: 86, SEQ ID NO: 87 and SEQ ID NO: 88, SEQ ID NO: 89 and SEQ ID NO: 90, SEQ ID NO: 93 and SEQ ID NO: 94, SEQ ID NO: 99 and SEQ ID NO: 100, SEQ ID NO: 101 and SEQ ID NO: 102, SEQ ID NO: 103 and SEQ ID NO: 104.

17. The kit or array according to any of claims 14 to 16, that further comprises at least one of primer pairs that are selected from the list consisting of: SEQ ID NO: 1 and SEQ ID NO: 2, SEQ ID NO: 9 and SEQ ID NO: 10, SEQ ID NO: 11 and SEQ ID NO: 12, SEQ ID NO: 17 and SEQ ID NO: 18, SEQ ID NO: 19 and SEQ ID NO: 20, SEQ ID NO: 21 and SEQ ID NO: 22, SEQ ID NO: 25 and SEQ ID NO: 26, SEQ ID NO: 27 and SEQ ID NO: 28, SEQ ID NO: 37 and SEQ ID NO: 38, SEQ ID NO: 51 and SEQ ID NO: 52, SEQ ID NO: 61 and SEQ ID NO: 62, SEQ ID NO: 71 and SEQ ID NO: 72, SEQ ID NO: 77 and SEQ ID NO: 78, SEQ ID NO: 91 and SEQ ID NO: 92, SEQ ID NO: 95 and SEQ ID NO: 96, SEQ ID NO: 107 and SEQ ID NO: 108, SEQ ID NO: 109 and SEQ ID NO: 110, and/or any combination thereof.

18. The kit or array according to any of claims 14 to 17, that comprises the primer pairs that are selected from the list consisting of: SEQ ID NO: 5 and SEQ ID NO: 6, SEQ ID NO: 23 and SEQ ID NO: 24, SEQ ID NO: 35 and SEQ ID NO: 36, SEQ ID NO: 33 and SEQ ID NO: 34, SEQ ID NO: 55 and SEQ ID NO: 56, SEQ ID NO: 97 and SEQ ID NO: 98, SEQ ID NO: 105 and SEQ ID NO: 106, SEQ ID NO: 3 and SEQ ID NO: 4, SEQ ID NO: 7 and SEQ ID NO: 8, SEQ ID NO: 13 and SEQ ID NO: 14, SEQ ID NO: 15 and SEQ ID NO: 16, SEQ ID NO: 29 and SEQ ID NO: 30, SEQ ID NO: 31 and SEQ ID NO: 32, SEQ ID NO: 39 and SEQ ID NO: 40, SEQ ID NO: 41 and SEQ ID NO: 42, SEQ ID NO: 43 and SEQ ID NO: 44, SEQ ID NO: 45 and SEQ ID NO: 46, SEQ ID NO: 47 and SEQ ID NO: 48, SEQ ID NO: 49 and SEQ ID NO: 50, SEQ ID NO: 53 and SEQ ID NO: 54, SEQ ID NO: 57 and SEQ ID NO: 58; SEQ ID NO: 59 and SEQ ID NO: 60, SEQ ID NO: 63 and SEQ ID NO: 64, SEQ ID NO: 65 and SEQ ID NO: 66, SEQ ID NO: 67 and SEQ ID NO: 68, SEQ ID NO: 69 and SEQ ID NO: 70, SEQ ID NO: 73 and SEQ ID NO: 74, SEQ ID NO: 75 and SEQ ID NO: 76, SEQ ID NO: 79 and SEQ ID NO: 80, SEQ ID NO: 81 and SEQ ID NO: 82, SEQ ID NO: 83 and SEQ ID NO: 84, SEQ ID NO: 85 and SEQ ID NO: 86, SEQ ID NO: 87 and SEQ ID NO: 88, SEQ ID NO: 89 and SEQ ID NO: 90, SEQ ID NO: 93 and SEQ ID NO: 94, SEQ ID NO: 99 and SEQ ID NO: 100, SEQ ID NO: 101 and SEQ ID NO: 102, SEQ ID NO: 103 and SEQ ID NO: 104, SEQ ID NO: 1 and SEQ ID NO: 2, SEQ ID NO: 9 and SEQ ID NO: 10, SEQ ID NO: 11 and SEQ ID NO: 12, SEQ ID NO: 17 and SEQ ID NO: 18, SEQ ID NO: 19 and SEQ ID NO: 20, SEQ ID NO: 21 and SEQ ID NO: 22, SEQ ID NO: 25 and SEQ ID NO: 26, SEQ ID NO: 27 and SEQ ID NO: 28, SEQ ID NO: 37 and SEQ ID NO: 38, SEQ ID NO: 51 and SEQ ID NO: 52, SEQ ID NO: 61 and SEQ ID NO: 62, SEQ ID NO: 71 and SEQ ID NO: 72, SEQ ID NO: 77 and SEQ ID NO: 78, SEQ ID NO: 91 and SEQ ID NO: 92, SEQ ID NO: 95 and SEQ ID NO: 96, SEQ ID NO: 107 and SEQ ID NO: 108, SEQ ID NO: 109 and SEQ ID NO: 110.

19. Use of the kit or array according to any of claims 14 to 18 for detecting duplications and/or deletions in the 22q11.2 chromosome region.
